# EUROPÄISCHE PATENTSCHRIFT

(11) **EP 1 222 163 B1**
(45) Veröffentlichungstag und Bekanntmachung des Hinweises auf die Patenterteilung: **12.07.2006**
(21) Anmeldenummer: 00967703.0
(22) Anmeldetag: 19.09.2000
(51) Int. Cl.: C07C 271/22, C07D 213/40, C07C 311/19, C07C 311/06, C07C 311/13, C07C 233/11, C07C 233/87, C07C 235/38, C07C 275/28, C07C 275/24, C07C 335/16, C07C 335/12, C07D 233/54, A61K 31/165, A61P 9/06

(54) **2'-SUBSTITUIERTE 1,1'-BIPHENYL-2-CARBOXAMIDE, VERFAHREN ZU IHRER HERSTELLUNG, IHRE VERWENDUNG ALS MEDIKAMENT SOWIE SIE ENTHALTENDE PHARMAZEUTISCHE ZUBEREITUNGEN**
2'-SUBSTITUTED 1,1'-BIPHENYL-2-CARBONAMIDES, METHOD FOR THE PRODUCTION THEREOF, USE THEREOF AS A MEDICAMENT AND PHARMACEUTICAL PREPARATIONS CONTAINING SAID COMPOUNDS
1,1'-BIPHENYL-2-CARBONAMIDES 2'-SUBSTITUES, PROCEDE DE FABRICATION, UTILISATION EN TANT QUE MEDICAMENT, AINSI QUE PREPARATIONS PHARMACEUTIQUES CONTENANT CES COMPOSES

(30) Priorität: 02.10.1999 DE 19947457
(43) Veröffentlichungstag der Anmeldung: 17.07.2002
(73) Patentinhaber: Sanofi-Aventis Deutschland GmbH, 65929 Frankfurt am Main (DE)
(72) Erfinder: BRENDEL, Joachim, 61118 Bad Vilbel (DE); SCHMIDT, Wolfgang, D-65929 Frankfurt (DE); BELOW, Peter, 60529 Frankfurt (DE)
(86) Internationale Anmeldenummer: PCT/EP2000/009151
(87) Internationale Veröffentlichungsnummer: WO 2001/025189

(56) Entgegenhaltungen:
- EP-A- 0 620 216
- BRANDMEIER, VOLKER ET AL: "Antiparallel.beta.-sheet conformation in cyclopeptides containing a pseudo-amino acid with a biphenyl moiety" HELV. CHIM. ACTA, Bd. 77, Nr. 1, 1994, Seiten 70-85, XP002159083 in der Anmeldung erwähnt
- LÜLLMAN H., MOHR K.: "Pharmakologie und Toxikologie" 1999 , GEORG THIEME VERLAG , STUTTGART XP002159084 Seite 151 -Seite 153

## Beschreibung

Die Erfindung betrifft Verbindungen der Formel I, 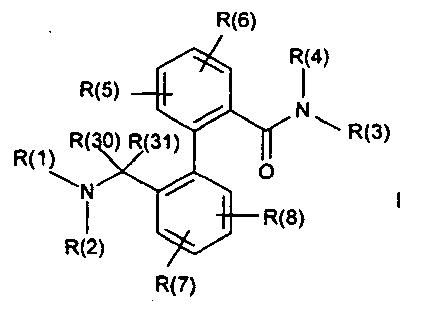 worin R(1), R(2), R(3), R(4), R(5), R(6), R(7), R(8), R(30) und R(31) die im folgenden angegebenen Bedeutungen haben, ihre Herstellung und ihre Verwendung, insbesondere in Arzneimitteln.

Die erfindungsgemäßen Verbindungen der Formel I sind bisher nicht bekannt. Sie wirken auf den sogenannten Kv1.5-Kalium-Kanal und inhibieren einen als "ultra-rapidly activating delayed rectifier" bezeichneten Kaliumstrom im humanen Herzvorhof. Die Verbindungen sind deshalb ganz besonders geeignet als neuartige antiarrhythmische Wirkstoffe, insbesondere zur Behandlung und Prophylaxe von Vorhof-Arrhythmien, z.B. Vorhof-Flimmern (atriale Fibrillation, AF) oder Vorhof-Flattem (atriales Flattern).

Vorhof-Flimmern (AF) und Vorhof-Flattem sind die häufigsten anhaltenden Herzarrhythmien. Das Auftreten erhöht sich mit zunehmenden Alter und führt häufig zu fatalen Folgeerscheinungen, wie zum Beispiel Gehimschlag. AF betrifft ca. 1 Millionen Amerikaner jährlich und führt zu mehr als 80.000 Schlaganfällen jedes Jahr in den USA. Die zur Zeit gebräuchlichen Antiarrhythmika der Klasse I und III reduzieren die Wiederauftrittsrate von AF, finden aber wegen ihrer potentiellen proarrhythmischen Nebenwirkungen nur eingeschränkte Anwendung. Deshalb besteht eine hohe medizinische Notwendigkeit für die Entwicklung besserer Medikamente zur Behandlung atrialer Arrhythmien (S. Nattel, Am. Heart J. 130, 1995, 1094 - 1106; "Newer developments in the management of atrial fibrillation").

Es wurde gezeigt, daß den meisten supraventrikulären Arrhythmien sogenannte "Reentry" Erregungswellen unterliegen. Solche Reentries treten dann auf, wenn das Herzgewebe eine langsame Leitfähigkeit und gleichzeitig sehr kurze Refraktärperioden besitzt. Das Erhöhen der myokardialen Refraktärzeit durch Verlängerung des Aktionspotentials ist ein anerkannter Mechanismus, um Arrhythmien zu beenden bzw. deren Entstehen zu verhindem (T.J. Colatsky et al, Drug Dev. Res. 19, 1990, 129 - 140; "Potassium channels as targets for antiarrhythmic drug action"). Die Länge des Aktionspotentials wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, der aus 3 verschiedenen Komponenten besteht: IKᵣ, IKₛ und IKᵤᵣ.

Die meisten bekannten Klasse III- Antiarrhythmika (z.B. Dofetilide, E4031 und d-Sotalol) blockieren überwiegend oder ausschließlich den schnell aktivierenden Kaliumkanal IKr, der sich sowohl in Zellen des menschlichen Ventrikel als auch im Vorhof nachweisen läßt. Es hat sich jedoch gezeigt, daß diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D. M. Roden, Am. J. Cardiol. 72, 1993, 44B-49B; "Current status of class III antiarrhythmic drug therapy"). Neben diesem hohen, zum Teil tödlichen Risiko bei niedriger Frequenz, wurde für die I_{Kr}-Blocker ein Nachlassen der Wirksamkeit unter den Bedingungen von Tachykardie, in der die Wirkung gerade benötigt wird, festgestellt ("negative use-dependence").

Während einige dieser Nachteile durch Blocker der langsam aktivierenden Komponente (IKₛ) möglicherweise überwunden werden können, wurde deren Wirksamkeit bisher nicht bewiesen, da keine klinischen Untersuchungen mit IKₛ₋Kanalblockern bekannt sind.

Die "besonders schnell" aktivierende und sehr langsam inaktivierende Komponente des delayed Rectifier IKᵤᵣ (=ultra-rapidly activating delayed rectifier), die dem Kv1.5-Kanal entspricht, spielt eine besonders große Rolle für die Repolarisationsdauer im menschlichen Vorhof. Eine Inhibierung des IKᵤᵣ-Kaliumauswärtsstroms stellt somit im Vergleich zur Inhibierung von IK, bzw. IKₛ eine besonders effektive Methode zur Verlängerung des atrialen Aktionspotentials und damit zur Beendigung bzw. Verhinderung von atrialen Arrhythmien dar. Mathematische Modelle des menschlichen Aktionspotentials legen nahe, daß der positive Effekt einer Blockade des IKᵤᵣ gerade unter den pathologischen Bedingungen einer chronischen atrialen Fibrillation besonders ausgeprägt sein sollte (M. Courtemanche, R. J. Ramirez, S. Nattel, Cardiovascular Research 1999, 42, 477-489: "Ionic targets for drug therapy and atrial fibrillation-induced electrical remodeling: insights from a mathematical model").

Im Gegensatz zu IKᵣ und IKₛ, die auch im menschlichen Ventrikel vorkommen, spielt der IKᵤᵣ zwar eine bedeutende Rolle im menschlichen Vorhof, jedoch nicht im Ventrikel. Aus diesem Grunde ist bei Inhibierung des IKᵤᵣ-Stroms im Gegensatz zur Blockade von IKᵣ oder IKₛ das Risiko einer proarrhythmischen Wirkung auf den Ventrikel von vornherein ausgeschlossen. (Z. Wang et al, Circ. Res. 73, 1993, 1061 -1076: "Sustained Depolarisation-Induced Outward Current in Human Atrial Myocytes"; G.-R. Li et al, Circ. Res. 78, 1996, 689 - 696: "Evidence for Two Components of Delayed Rectifier K⁺-Current in Human Ventricular Myocytes"; G. J. Amos et al, J. Physiol. 491, 1996, 31 - 50: "Differences between outward currents of human atrial and subepicardial ventricular myocytes").

Antiarrhythmika, die über eine selektive Blockade des IKᵤᵣ-Stroms bzw. Kv1.5-Kanals wirken, sind auf dem Markt bisher jedoch nicht verfügbar. Für zahlreiche pharmazeutische Wirkstoffe (z.B. Tedisamil, Bupivacaine oder Sertindole) wurde zwar eine blockierende Wirkung auf den Kv1.5-Kanal beschrieben, doch stellt die Kv1.5-Blockade hier jeweils nur eine Nebenwirkung neben anderen Hauptwirkungen der Substanzen dar.

In WO 98 04 521 werden Aminoindane als Kaliumkanalblocker beansprucht, die den Kv1.5-Kanal blockieren. In den Anmeldungen WO 98 18 475 und WO 98 18 476 wird die Verwendung verschiedener Pyridazinone und Phosphinoxide als Antiarrhythmika beansprucht, die über eine Blockade des IKᵤᵣ wirken sollen. Die gleichen Verbindungen wurden ursprünglich jedoch auch als Immunsuppressiva beschrieben (WO 96 25 936). Die in diesen genannten Anmeldungen beschriebenen Verbindungen sind strukturell völlig andersartig als die erfindungsgemäßen Verbindungen dieser Anmeldung.

Es wurde nun überraschenderweise gefunden, daß die hier beschriebenen 2'-substituierten 1,1'-Biphenyl-2-carbonamide potente Blocker des humanen Kv1.5-Kanals sind. Sie können deshalb verwendet werden als neuartige Antiarrhythmika mit besonders vorteilhaftem Sicherheitsprofil. Insbesondere eignen sich die Verbindungen zur Behandlung supraventrikulärer Arrhythmien, z. B. Vorhof-Flimmem oder Vorhof-Flattern.

Die Verbindungen können eingesetzt werden zur Terminierung von bestehendem Vorhof-Flimmem oder -Flattern zur Wiedererlangung des Sinus-Rhythmus (Kardioversion). Darüber hinaus reduzieren die Substanzen die Anfälligkeit zur Entstehung neuer Flimmer-Ereignisse (Erhalt des Sinus-Rhythmus, Prophylaxe).

Die erfindungsgemäßen Verbindungen sind bisher nicht bekannt. Einige strukturell verwandte Verbindungen sind beschrieben in Helv. Chim. Acta 1994 (70) 70 und dort zitierter Literatur. Für die dort beschriebenen peptidischen Verbindungen (z.B. Verbindung A) ist jedoch keine Kaliumkanal blockierende Aktivität bekannt. Darüber hinaus sollten derartige Verbindungen aufgrund der zahlreichen peptidischen Bindungen eine zu geringe metabolische Stabilität für eine Anwendung als Antiarrhythmikum aufweisen.

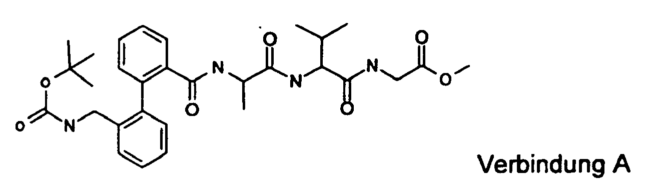

Eine weitere ähnliche Verbindung (Verbindung B) ist in der Europäischen Patentanmeldung EP 0620216 aufgeführt. Die Verbindung B und alle übrigen Verbindungen dieser Anmeldung sind dadurch gekennzeichnet, daß sie in der Position von R(3) einen speziellen Substituenten tragen (z. B. Benzoyl-1,2,3,4-tetrahydroisochinolin), der bei den erfindungsgemäßen Verbindungen dieser Anmeldung nicht umfaßt ist. Die in EP 0620 216 genannten Verbindungen wirken als Vasopressin- Antagonisten und haben damit eine völlig andere biologische Aktivität als die hier beschriebenen Blocker des Kv1.5-Kanals.

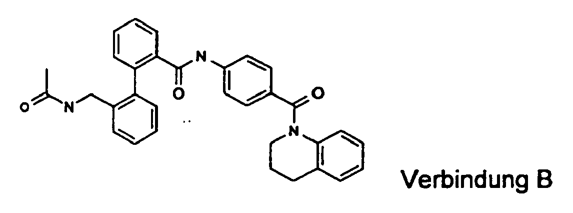

Die vorliegende Erfindung betrifft Verbindungen der Formel I, 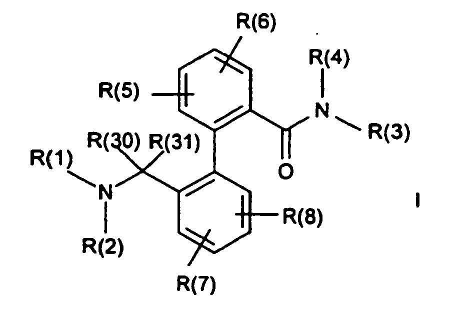 worin bedeuten:
- R(1): C(O)OR(9), SO₂R(10), COR(11), C(O)NR(12)R(13) oder C(S)NR(12)R(13);
R(9) CₓH₂ₓ-R(14);
x 0, 1, 2, 3 oder 4,
wobei x nicht 0 sein kann, wenn R(14) OR(15) oder SO₂Me bedeutet;
R(14) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(15), SO₂Me Phenyl, Naphthyl, Biphenylyl, Furyl, Thienyl oder ein N-haltiger Heteroaromat mit 1, 2, 3, 4, 5, 6, 7, 8 oder 9 C-Atomen,
wobei Phenyl, Naphthyl, Biphenylyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Alkyl mit 1, 2, 3, 4 oder 5 C-Atomen, Cycloalkyl mit 3, 4, 5 oder 6 C-Atomen, CF₃ oder Phenyl, das unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(10), R(11) und R(12) unabhängig von einander wie R(9) definiert;
R(13) Wasserstoff, Alkyl mit 1, 2, 3 oder 4 C-Atomen oder CF₃;
- R(2): Wasserstoff, Alkyl mit 1, 2, 3 oder 4 C-Atomen oder CF₃;
- R(3): C_{y}H_{2y}-R(16);
y 0, 1, 2, 3 oder 4,
wobei y nicht 0 sein kann, wenn R(16) OR(17) oder SO₂Me bedeutet;
R(16) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(17), SO₂Me, Phenyl, Naphthyl, Furyl, Thienyl oder ein N-haltiger Heteroaromat mit 1, 2, 3, 4, 5, 6, 7, 8 oder 9 C-Atomen,
wobei Phenyl, Naphthyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(17) Wasserstoff, Alkyl mit 1, 2, 3, 4 oder 5 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
oder
- R(3): CHR(18)R(19);
R(18) Wasserstoff oder C_{z}H_{2z}-R(16), wobei R(16) wie oben angegeben definiert ist;
z 0, 1, 2 oder 3;
R(19) COOH, CONH₂, CONR(20)R(21), COOR(22), CH₂OH;
R(20) Wasserstoff, Alkyl mit 1, 2, 3, 4 oder 5 C-Atomen, CᵥH₂ᵥ₋CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
v 0, 1, 2 oder 3;
w 0, 1, 2 oder 3;
R(21) Wasserstoff oder Alkyl mit 1, 2, 3, 4 oder 5 C-Atomen;
R(22) Alkyl mit 1, 2, 3, 4 oder 5 C-Atomen;
- R(4): Wasserstoff, Alkyl mit 1, 2, 3, 4, 5 oder 6 C-Atomen oder CF₃;
oder
- R(3) und R(4): gemeinsam eine Kette von 4 oder 5 Methylengruppen, von denen eine Methylengruppe durch -O-, -S-, -NH-, -N(Methyl)- oder -N(Benzyl)- ersetzt sein kann;
- R(5), R(6), R(7) und R(8): unabhängig voneinander Wasserstoff, F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino;
- R(30) und R(31): unabhängig voneinander Wasserstoff oder Alkyl mit 1, 2 oder 3 C-Atomen;
oder
- R(30) und R(31): gemeinsam eine Kette von 2 Methylengruppen;
sowie ihre pharmazeutisch akzeptablen Salze.

Bevorzugt sind Verbindungen der Formel I, worin bedeuten:
- R(1): C(O)OR(9), SO₂R(10), COR(11) oder C(O)NR(12)R(13);
R(9) CₓH₂ₓ-R(14);
x 0, 1, 2, 3 oder 4,
wobei x nicht 0 sein kann, wenn R(14) OR(15) bedeutet;
R(14) Alkyl mit 1, 2, 3 oder 4 C-Atomen, Cycloalkyl mit 3, 4, 5, 6, 7, 8 oder 9 C-Atomen, CF₃, C₂F₅, OCF₃, OR(15), Phenyl, Furyl, Thienyl oder ein N-haltiger Heteroaromat mit 1, 2, 3, 4, 5, 6, 7, 8 oder 9 C-Atomen,
wobei Phenyl, Furyl, Thienyl und der N-haltige Heteroaromat unsubstituiert sind oder substituiert mit 1, 2 oder 3 Substituenten ausgewählt aus der Gruppe bestehend aus F, Cl, Br, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Alkyl mit 1, 2, 3, 4 oder 5 C-Atomen, Cycloalkyl mit 3, 4, 5 oder 6 C-Atomen, CF₃ oder Phenyl,
das unsubstituiert ist oder substituiert mit 1, 2 oder 3 Substituenten ausgewählt aus der Gruppe bestehend aus F, Cl, Br, CF₃, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(10), R(11) und R(12) unabhängig von einander wie R(9) definiert;
R(13) Wasserstoff, Alkyl mit 1, 2, 3 oder 4 C-Atomen oder CF₃;
- R(2): Wasserstoff, Alkyl mit 1, 2, 3 oder 4 C-Atomen oder CF₃;
- R(3): CyH₂y-R(16);
y 0, 1, 2, 3 oder 4,
wobei y nicht 0 sein kann, wenn R(16) OR(17) bedeutet;
R(16) Alkyl mit 1, 2, 3 oder 4 C-Atomen, Cycloalkyl mit 3, 4, 5, 6, 7, 8 oder 9 C-Atomen, CF₃, C₂F₅, OR(17), Phenyl, Furyl, Thienyl oder ein N-haltiger Heteroaromat mit 1, 2, 3, 4, 5, 6, 7, 8 oder 9 C-Atomen,
wobei Phenyl, Furyl, Thienyl und der N-haltige Heteroaroma unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(17) Alkyl mit 1, 2, 3, 4 oder 5 C-Atomen, Cycloalkyl mit 3, 4, 5 oder 6 C-Atomen, CF₃, Phenyl oder 2-, 3-, oder 4- Pyridyl,
wobei Phenyl oder 2-, 3-, oder 4- Pyridyl unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
oder
- R(3): CHR(18)R(19);
R(18) Wasserstoff oder C_{z}H₂ᵣR(16), wobei R(16) wie oben angegeben definiert ist;
z 0, 1, 2 oder 3;
R(19) CONH₂, CONR(20)R(21), COOR(22), CH₂OH;
R(20) Wasserstoff, Alkyl mit 1, 2, 3, 4 oder 5 C-Atomen, CᵥH₂ᵥ₋CF₃ oder C_{w}H_{2w}- Phenyl,
wobei der Phenylring unsubstituiert ist oder substituiert mit 1, 2 oder 3 Substituenten ausgewählt aus der Gruppe bestehend aus F, Cl, Br, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
v 0, 1, 2 oder 3;
w 0, 1, 2 oder 3;
R(21) Wasserstoff oder Alkyl mit 1, 2, 3, 4 oder 5 C-Atomen;
R(22) Alkyl mit 1, 2, 3, 4 oder 5 C-Atomen;
- R(4): Wasserstoff, Alkyl mit 1, 2, 3, 4, 5 oder 6 C-Atomen oder CF₃;
- R(5), R(6), R(7) und R(8): unabhängig voneinander Wasserstoff, F, Cl, Br, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino;
- R(30) und R(31): unabhängig voneinander Wasserstoff oder Alkyl mit 1, 2 oder 3 C-Atomen;
oder
- R(30) und R(31): gemeinsam eine Kette von 2 Methylengruppen;
sowie ihre pharmazeutisch akzeptablen Salze.

Besonders bevorzugt sind Verbindungen der Formel I, worin bedeuten:
- R(1): C(O)OR(9), SO₂R(10), COR(11) oderC(O)NR(12)R(13);
R(9) CₓH₂ₓ-R(14);
x 0, 1, 2, 3 oder 4,
wobei x nicht 0 sein kann, wenn R(14) OR(15) bedeutet;
R(14) Cycloalkyl mit 3, 4, 5, 6, 7, 8 oder 9 C-Atomen, CF₃, OR(15), Phenyl, Furyl, Thienyl oder ein N-haltiger Heteroaromat mit 3, 4 oder 5 C-Atomen,
wobei Phenyl, Furyl, Thienyl und der N-haltige Heteroaromat unsubstituiert sind oder substituiert mit 1 oder 2 Substituenten ausgewählt aus der Gruppe bestehend aus F, Cl, Br, CF₃, OCF₃, CN, COOMe, CONH₂, COMe, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(15) Alkyl mit 1 oder 2 C-Atomen, Cycloalkyl mit 3, 4, 5 oder 6 C-Atomen, CF₃ oder Phenyl,
das unsubstituiert ist oder substituiert mit 1 oder 2 Substituenten ausgewählt aus der Gruppe bestehend aus F, Cl, Br, CF₃, CN, COOMe, CONH₂, COMe, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(10), R(11) und R(12) unabhängig von einander wie R(9) definiert;
R(13) Wasserstoff;
- R(2): Wasserstoff oder Alkyl mit 1, 2 oder 3 C-Atomen;
- R(3): CHR(18)R(19);
R(18) Wasserstoff oder C_{z}H_{2z}-R(16);
z 0, 1, 2 oder 3;
R(19) CONH₂, CONR(20)R(21), COOR(22) oder CH₂OH;
R(20) Wasserstoff, Alkyl mit 1, 2, 3, 4 oder 5 C-Atomen, CᵥH₂ᵥ₋CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, CF₃, OCF₃, CN, COOMe, CONH₂, COMe, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
v 0, 1, 2 oder 3;
w 0, 1, 2 oder 3;
R(21) Wasserstoff oder Alkyl mit 1, 2, 3, 4 oder 5 C-Atomen;
R(22) Alkyl mit 1, 2, 3,4 oder 5 C-Atomen;
R(16) Alkyl mit 1, 2 oder 3 C-Atomen, Cycloalkyl mit 3, 4, 5, 6, 7, 8 oder 9 C-Atomen, CF₃, OR(17), Phenyl, Furyl, Thienyl oder ein N-haltiger Heteroaromat mit 3, 4 oder 5 C-Atomen,
wobei Phenyl, Furyl, Thienyl und der N-haltige Heteroaromat unsubstituiert sind oder substituiert mit 1 oder 2 Substituenten ausgewählt aus der Gruppe bestehend aus F, Cl, Br, CF₃, OCF₃, CN, COOMe, CONH₂, COMe, NH₂, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(17) Alkyl mit 1, 2, 3 oder 4 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, CF₃, OCF₃, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4): Wasserstoff oder Alkyl mit 1 oder 2 C-Atomen;
- R(5), R(6), R(7) und R(8): unabhängig voneinander Wasserstoff, F, Cl, Br, CF₃, CN, COOMe, CONH₂, COMe, NH₂, OH, Alkyl mit 1, 2 oder 3 C-Atomen, Alkoxy mit 1 oder 2 C-Atomen, Dimethylamino, Sulfamoyl, Methylsulfonyl oder Methylsulfonylamino;
- R(30) und R(31): unabhängig voneinander Wasserstoff oder Methyl;
oder
- R(30) und R(31): gemeinsam eine Kette von 2 Methylengruppen;
sowie ihre pharmazeutisch akzeptablen Salze.

Besonders bevorzugt sind auch Verbindungen der Formel I, worin bedeuten:
- R(1): C(O)OR(9), SO₂R(10), COR(11) oder C(O)NR(12)R(13);

- R(9): CₓH₂ₓ-R(14);
x 0, 1, 2, 3 oder 4,
wobei x nicht 0 sein kann, wenn R(14) OR(15) bedeutet;
R(14) Alkyl mit 1, 2, 3 oder 4 C-Atomen, Cycloalkyl mit 3, 4, 5, 6, 7, 8 oder 9 C-Atomen, CF₃, OR(15), Phenyl, Furyl, Thienyl oder ein N-haltiger Heteroaromat mit 3, 4 oder 5 C-Atomen,
wobei Phenyl, Furyl, Thienyl und der N-haltige Heteroaromat unsubstituiert sind oder substituiert mit 1 oder 2 Substituenten ausgewählt aus der Gruppe bestehend aus F, Cl, Br, CF₃, OCF₃, CN, COOMe, CONH₂, COMe, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(15) Alkyl mit 1 oder 2 C-Atomen, Cycloalkyl mit 3, 4, 5 oder 6 C-Atomen, CF₃ oder Phenyl,
das unsubstituiert ist oder substituiert mit 1 oder 2 Substituenten ausgewählt aus der Gruppe bestehend aus F, Cl, Br, CF₃, CN, COOMe, CONH₂, COMe, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(10), R(11) und R(12) unabhängig von einander wie R(9) definiert;
R(13) Wasserstoff;
- R(2): Wasserstoff oder Alkyl mit 1, 2 oder 3 C-Atomen;
- R(3): C_{y}H_{2y}-R(16);
y 0, 1, 2, 3 oder 4,
wobei y nicht 0 sein kann, wenn R(16) OR(17) bedeutet;
R(16) Alkyl mit 1, 2 oder 3 C-Atomen, Cycloalkyl mit 3, 4, 5, 6, 7, 8 oder 9 C-Atomen, CF₃, OR(17), Phenyl, Furyl, Thienyl oder ein N-haltiger Heteroaromat mit 3, 4 oder 5 C-Atomen,
wobei Phenyl, Furyl, Thienyl und der N-haltige Heteroaromat unsubstituiert sind oder substituiert mit 1 oder 2 Substituenten ausgewählt aus der Gruppe bestehend aus F, Cl, Br, CF₃, OCF₃, CN, COOMe, CONH₂, COMe, NH₂, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(17) Alkyl mit 1, 2, 3, 4 oder 5 C-Atomen, Cycloalkyl mit 3, 4, 5 oder 6 C-Atomen, CF₃, Phenyl oder 2-, 3- oder 4- Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4): Wasserstoff oder Alkyl mit 1 oder 2 C-Atomen;
- R(5), R(6), R(7) und R(8): unabhängig voneinander Wasserstoff, F, Cl, Br, CF₃, CN, COOMe, CONH₂, COMe, NH₂, OH, Alkyl mit 1, 2 oder 3 C-Atomen, Alkoxy mit 1 oder 2 C-Atomen, Dimethylamino, Sulfamoyl, Methylsulfonyl oder Methylsulfonylamino;
- R(30) und R(31): unabhängig voneinander Wasserstoff oder Methyl;
oder
- R(30) und R(31): gemeinsam eine Kette von 2 Methylengruppen;
sowie ihre pharmazeutisch akzeptablen Salze.

Ganz besonders bevorzugt sind Verbindungen der Formel I, worin bedeuten:
- R(1): C(O)OR(9), SO₂R(10), COR(11) oder C(O)NR(12)R(13);
R(9) CₓH₂ₓ-R(14);
x 0, 1, 2 oder 3;
R(14) Alkyl mit 1, 2, 3 oder 4 C-Atomen, Cycloalkyl mit 3, 4, 5, 6, 7, 8 oder 9 C-Atomen, CF₃, Phenyl oder Pyridyl,
wobei Phenyl und Pyridyl unsubstituiert sind oder substituiert mit 1 oder 2 Substituenten ausgewählt aus der Gruppe bestehend aus F, Cl, CF₃, OCF₃, OH. Alkyl mit 1, 2 oder 3 C-Atomen und Alkoxy mit 1 oder 2 C-Atomen;
R(10), R(11) und R(12) unabhängig von einander wie R(9) definiert;
R(13) Wasserstoff;
- R(2): Wasserstoff;
- R(3): C_{y}H_{2y}-R(16);
y 0, 1 oder 2;
R(16) Alkyl mit 1, 2 oder 3 C-Atomen, Cycloalkyl mit 5 oder 6 C-Atomen, CF₃, Phenyl oder Pyridyl
wobei Phenyl und Pyridyl unsubstituiert sind oder substituiert mit 1 oder 2 Substituenten ausgewählt aus der Gruppe bestehend aus F, Cl, CF₃, OCF₃, OH, Alkyl mit 1, 2 oder 3 C-Atomen und Alkoxy mit 1 oder 2 C-Atomen;
- R(4): Wasserstoff;
- R(5), R(6), R(7) und R(8): unabhängig voneinander Wasserstoff, F, CF₃, CN, COOMe, CONH₂, NH₂, OH, Alkyl mit 1, 2 oder 3 C-Atomen oder Alkoxy mit 1 oder 2 C-Atomen;
- R(30) und R(31): unabhängig voneinander Wasserstoff oder Methyl;
oder
- R(30) und R(31): gemeinsam eine Kette von 2 Methylengruppen;
sowie ihre pharmazeutisch akzeptablen Salze.

Speziell bevorzugt sind Verbindungen der Formel I, worin bedeuten:
- R(1): C(O)OR(9) oder COR(11);
R(9) CₓH₂ₓ-R(14);
x 0, 1, 2 oder 3;
R(14) Cycloalkyl mit 5 oder 6 C-Atomen oder Phenyl, wobei Phenyl unsubstituiert ist oder substituiert mit 1 oder 2 Substituenten ausgewählt aus der Gruppe bestehend aus F, Cl, CF₃, OCF₃, Alkyl mit 1, 2 oder 3 C-Atomen und Alkoxy mit 1 oder 2 C-Atomen;
R(11) wie R(9) definiert;
- R(2): Wasserstoff;
- R(3): CyH₂y-R(16);
y 0, 1 oder 2;
R(16) Alkyl mit 1, 2 oder 3 C-Atomen, Cycloalkyl mit 5 oder 6 C-Atomen, CF₃, Phenyl oder Pyridyl
wobei Phenyl und Pyridyl unsubstituiert sind oder substituiert mit 1 oder 2 Substituenten ausgewählt aus der Gruppe bestehend aus F, Cl, CF₃, OCF₃, Alkyl mit 1, 2 oder 3 C-Atomen und Alkoxy mit 1 oder 2 C-Atomen;
- R(4): Wasserstoff;
- R(5), R(6), R(7) und R(8): unabhängig voneinander Wasserstoff, F, CF₃, Alkyl mit 1, 2 oder 3 C-Atomen oder Alkoxy mit 1 oder 2 C-Atomen;
- R(30) und R(31): Wasserstoff;
sowie ihre pharmazeutisch akzeptablen Salze.

Alkylreste und Alkylenreste können geradkettig oder verzweigt sein. Dies gilt auch für die Alkylenreste der Formeln CₓH₂ₓ, C_{y}H_{2y}, C_{z}H_{2z}, CᵥH₂ᵥ und C_{w}H_{2w}. Alkylreste und Alkylenreste können auch geradkettig oder verzweigt sein, wenn sie substituiert sind oder in anderen Resten enthalten sind, z. B. in einem Alkoxyrest oder oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,1-Butylen, 1,4-Butylen, 1,5-Pentylen, 2,2-Dimethyl-1,3-propylen, 1,6-Hexylen, usw. sind Beispiele für Alkylenreste.

Cycloalkylreste können ebenfalls verzweigt sein. Beispiele für Cycloalkylreste mit 3 bis 11 C-Atomen sind Cyclopropyl, Cyclobutyl, 1-Methylcyclopropyl, 2-Methylcyclopropyl, Cyclopentyl, 2-Methylcyclobutyl, 3-Methylcyclobutyl, Cyclopentyl, Cyclohexyl, 2-Methylcyclohexyl, 3-Methylcyclohexyl, 4-Methylcyclohexyl, Menthyl, Cycloheptyl, Cyclooctyl usw.

Als N-haltige Heteroaromaten mit 1, 2, 3, 4, 5, 6, 7, 8 oder 9 C-Atomen gelten insbesondere 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder 5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3-oder 5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder -5-yl, 1,2,4-Thiadiazol-3-oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfaßt sind weiterhin die entsprechenden N-Oxide dieser Verbindungen, also z.B. 1-Oxy-2-, 3- oder 4-pyridyl.

Besonders bevorzugt sind die N-haltigen Heterocyclen Pyrrolyl, Imidazolyl, Chinolyl, Pyrazolyl, Pyridyl, Pyrazinyl, Pyrimidinyl und Pyridazinyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Monosubstituierte Phenylreste können in der 2-, der 3- oder der 4-Position substituiert sein, disubstituierte in der 2,3-, 2,4-, 2,5-, 2,6-, 3,4- oder 3,5-Position, trisubstituierte in der 2,3,4-, 2,3,5-, 2,3,6-, 2,4,5-, 2,4,6- oder 3,4,5-Position. Entsprechendes gilt sinngemäß analog auch für die N-haltigen Heteroaromaten, den Thiophen- oder die Furylrest.

Bei Di- bzw. Trisubstitution eines Restes können die Substituenten gleich oder verschieden sein.

Wenn R(3) und R(4) gemeinsam eine Kette von 4 oder 5 Methylengruppen bedeuten, von denen eine Methylengruppe durch -O-, -S-, -NH- etc. ersetzt sein kann, dann bilden diese Reste zusammen mit dem Stickstoffatom der Formel I einen 5 oder 6 gliedrigen Stickstoffheterocyclus, wie z.B. Pyrrolidin, Piperidin, Morpholin, Thiomorpholin etc.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate. Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. gehören also in enantiomerenreiner Form, sowohl als links- als auch als rechtsdrehende Antipoden, und auch in Form von Mischungen der beiden Enantiomeren in unterschiedlichen Verhältnissen oder in Form von Racematen zu der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Die Verbindungen der Formel I sind durch unterschiedliche chemische Verfahren herstellbar, die ebenfalls zu der vorliegenden Erfindung gehören. Einige typische Wege sind in den unten als Schema 1, 2, 3 und 4 bezeichneten Reaktionssequenzen skizziert. Die hierbei verwendeten Reste R(1) bis R(8) sind jeweils wie oben angegeben definiert, sofern nachfolgend nicht etwas anderes angegeben ist.

So erhält man beispielsweise eine Verbindung der Formel I gemäß Schema 1 ausgehend von Diphensäureanhydridderivaten der Formel II als kommerziellerhältlichen bzw. literaturbekannten Vorstufen. Reduktion der Verbindungen II mit Natriumborhydrid gefolgt von Umsetzung mit Kaliumphthalimid wie beschrieben in Tetrahedron 45 (1989) 1365-1376 liefert die Biphenylcarbonsäuren der Formel IV. Durch Kopplung mit Aminen der Formel HNR(3)R(4) gefolgt von Hydrazinolyse des Phthalimids erhält man die Aminomethylverbindungen der Formel VI, aus denen durch Umsetzung mit geeigneten Derivaten der Formel R(1)-X die erfindungsgemäßen Verbindungen der Formel I erhalten werden, worin R(2) Wasserstoff bedeutet und R(1), R(3), R(4), R(5), R(6), R(7) und R(8) die oben angegebenen Bedeutungen besitzen. Anschließende Alkylierung mit geeigneten Alkylierungsmitteln der Formel R(2)-Y, worin Y für eine nucleofuge Fluchtgruppe, z.B. Cl, Br oder I steht, liefert die entsprechenden Verbindungen der Formel I, worin R(2) Alkyl mit 1 bis 4 C-Atomen bedeutet.

Alternativ können die Biphenylcarbonsäuren der Formel IV auch durch Hydrazinolyse zu den Aminocarbonsäuren der Formel VII umgewandelt werden, die dann durch Reaktion der Aminogruppe mit Verbindungen der Formeln R(1)-X und R(2)-Y gefolgt von Amidierung der Carbonsäure mit Aminen der Formel HNR(3)R(4) zu erfindungsgemäßen Verbindungen der Formel I umgesetzt werden (Schema 2).

In manchen Fällen kann es sinnvoll sein, zunächst nach einer der zuvor genannten Methoden erfindungsgemäße Verbindungen der Formel Ia (Schema 3) herzustellen, in der R(9) für einen leicht abspaltbaren Rest, wie z.B. tert.-Butyl oder Benzyl steht. Nach Abspaltung der entsprechenden Schutzgruppe, z.B. mit Trifluoressigsäure für die Boc-Gruppe oder durch katalytische Hydrierung für den Benzyloxycarbonylrest, erhält man die Verbindungen der Formel IX, die dann wiederum durch Umsetzung mit Verbindungen der Formel R(1)-X in andere erfindungsgemäße Verbindungen der Formel I umgewandelt werden können.

Eine andere Möglichkeit zur Herstellung von erfindungsgemäßen Verbindungen besteht in der Palladium-katalysierten Kupplung eines Phenylbromids oder lodids der Formel X mit einer Phenylboronsäure der Formel XI (Suzuki-Kupplung; Schema 4), die z.B. in Gegenwart von Pd[(PPh)₃]₄ als Katalysator, Natriumcarbonat als Base und 1,2-Dimethoxyethan als Lösungsmittel durchgeführt werden kann.
Wenn R(9) für einen leicht abspaltbaren Rest, wie z.B. tert.-Butyl oder Benzyl steht, können die Verbindungen der Formel Ib dann wie oben und in Schema 3 beschrieben in andere erfindungsgemäße Verbindungen der Formel I umgewandelt werden.

Die benötigten Boronsäuren der Formel XI können aus den Verbindungen der Formel XII, in der Z für Wasserstoff, Brom oder lod steht, durch Ortholitiierung bzw. Metall-Halogenaustausch gefolgt von Umsetzung mit Borsäuretrimethylester erhalten werden .

Die oben genannten Umsetzungen der Verbindungen der Formeln VI, VII und IX mit Verbindungen der Formel R(1)-X entsprechen der bekannten Umwandlung eines Amins in ein Carbonsäureamid-, Sulfonsäureamid-, Carbamat-, Hamstoff oder Thiohamstoffderivat. Der Rest X steht hierbei für eine geeignete nucleofuge Abgangsgruppe, wie z.B. F, Cl, Br, Imidazol, O-Succinimid etc.

Zur Herstellung von Verbindungen der Formel I oder VIII in denen R(1) C(O)OR(9) bedeutet, also Carbamaten, werden z.B. Verbindungen der Formel R(1)-X verwendet, bei denen X für Chlor oder O-Succinimid steht, also Chloroformiate oder Succinimidocarbonate.

Zur Herstellung von Verbindungen der Formel Ioder VIII in denen R(1) SO₂R(10) bedeutet, also Sulfonamiden, werden in der Regel Verbindungen der Formel R(1)-X verwendet, bei denen X für Chlor steht, also Sulfonsäurechloride.

Zur Herstellung von Verbindungen der Formel I oder VIII in denen R(1) COR(11) bedeutet, also Carbonsäureamiden, werden z.B. Verbindungen der Formel R(1)-X verwendet, bei denen X für Chlor, Imidazol oder Acetoxy steht, also Carbonsäurechloride, Carbonsäureimidazolide oder gemischte Anhydride. Es können aber auch die freien Säuren der Formel R(1)-OH in Gegenwart geeigneter Kondensationsmittel wie Carbodiimiden oder Uroniumsalzen wie TOTU verwendet werden.

Zur Herstellung von Verbindungen der Formel I oder VIII in denen R(1) CONR(12)R(13) oder C(S)NR(12)R(13) bedeutet, also Harnstoffen oder Thiohamstoffen, können anstelle der Verbindungen der Formel R(1)-X auch Verbindungen der Formel R(12)N(=C=O), bzw. R(12)N(=C=S), also Isocyanate oder Thioisocyanate verwendet werden.

Die oben genannten Umsetzungen der Verbindungen der Formeln IV oder VIII mit Aminen der Formel HNR(3)R(4) entsprechen der bekannten Umwandlung einer Carbonsäure zu einem Carbonsäureamid. Zur Durchführung dieser Reaktionen sind in der Literatur zahlreiche Methoden beschrieben worden. Besonders vorteilhaft können sie durch Aktivierung der Carbonsäure, z.B. mit Dicyclohexylcarbodiimid (DCC), gegebenenfalls unter Zusatz von Hydroxybenzotriazol (HOBT) oder Dimethylaminopyridin (DMAP), oder mit O-[(Cyano(ethoxycarbonyl)methylen)amino]-1,1,3,3-tetramethyluroniumtetrafluoroborat (TOTU), durchgeführt werden. Es können aber auch zunächst nach bekannten Methoden reaktive Säurederivate synthetisiert werden, z.B. Säurechloride durch Umsetzung der Carbonsäuren der Formel IV oder VIII mit anorganischen Säurehalogeniden, wie z.B. SOCl₂, oder Säureimidazolide durch Umsetzung mit Carbonyldiimidazol, die dann anschließend, gegebenenfalls unter Zusatz einer Hilfsbase, mit den Aminen der Formel HNR(3)R(4) umgesetzt werden.

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepaßt werden.

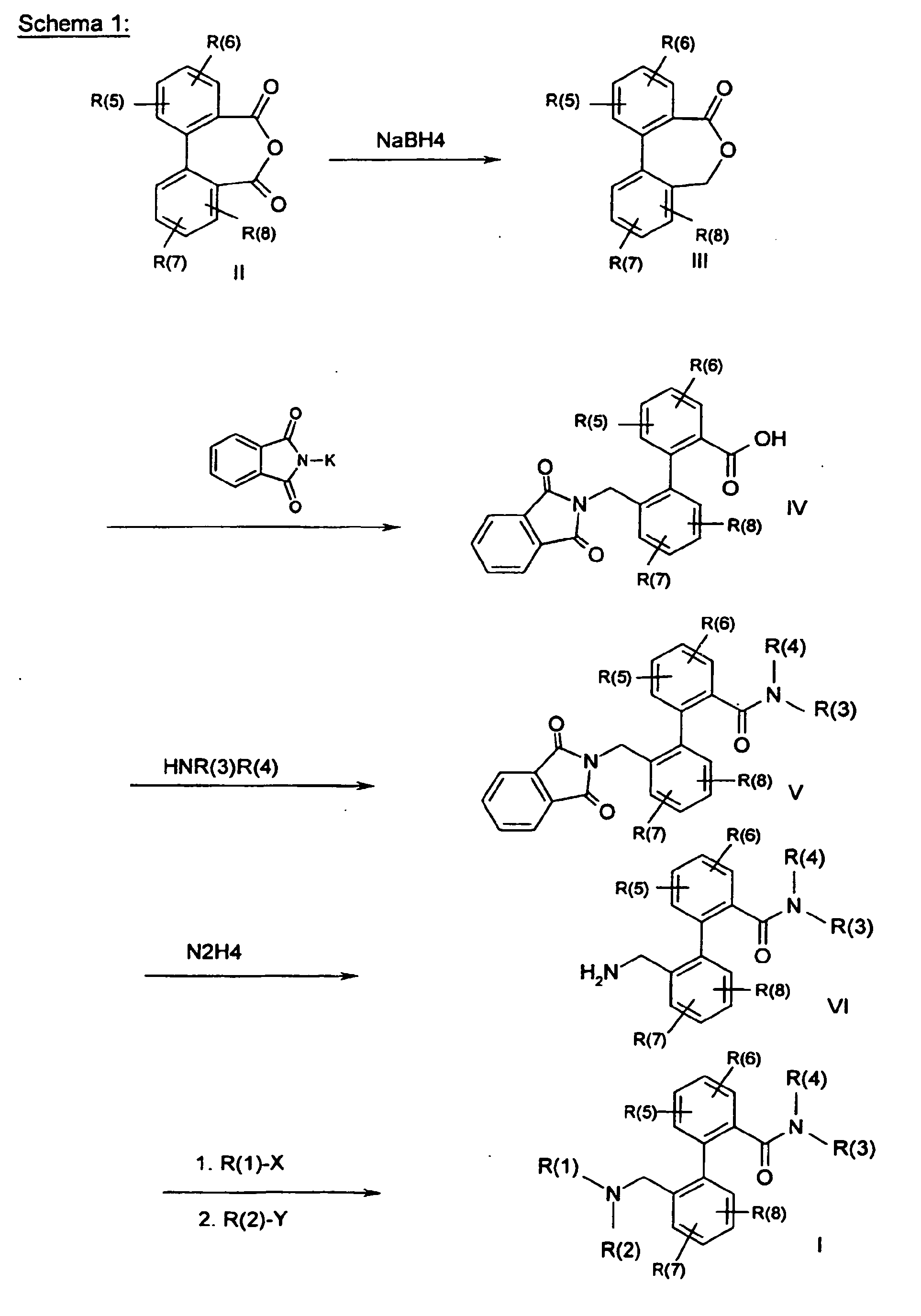

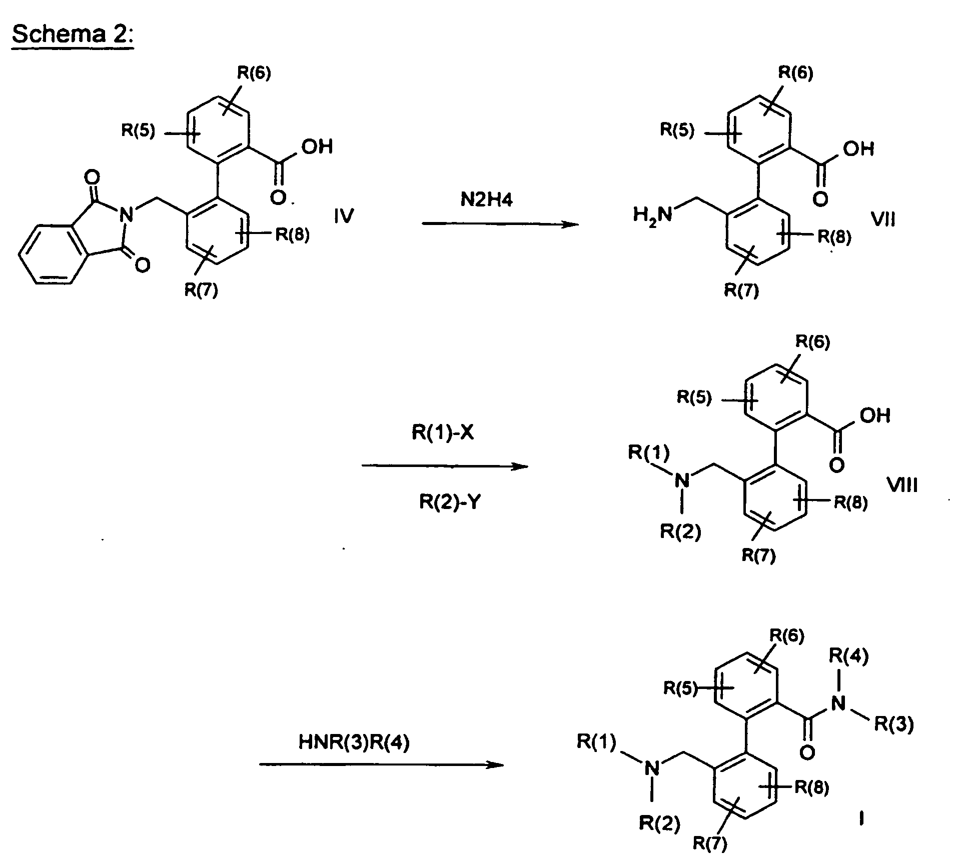

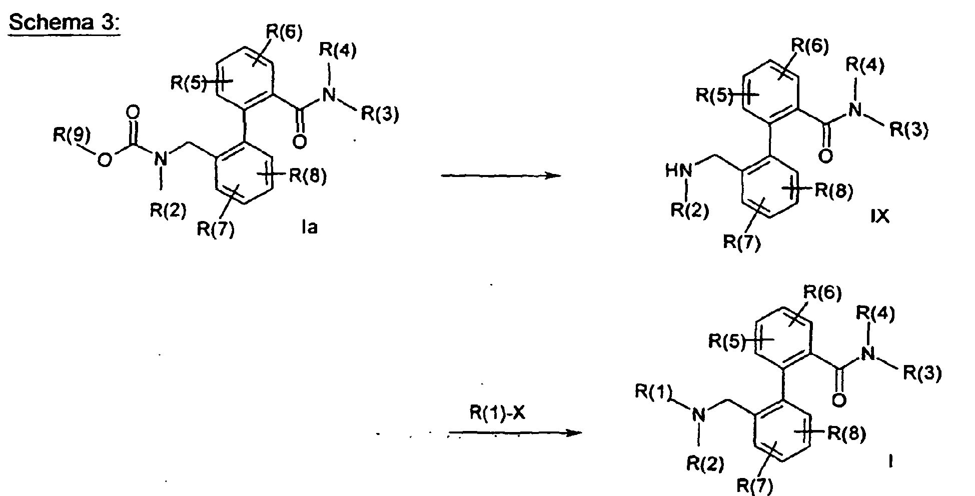

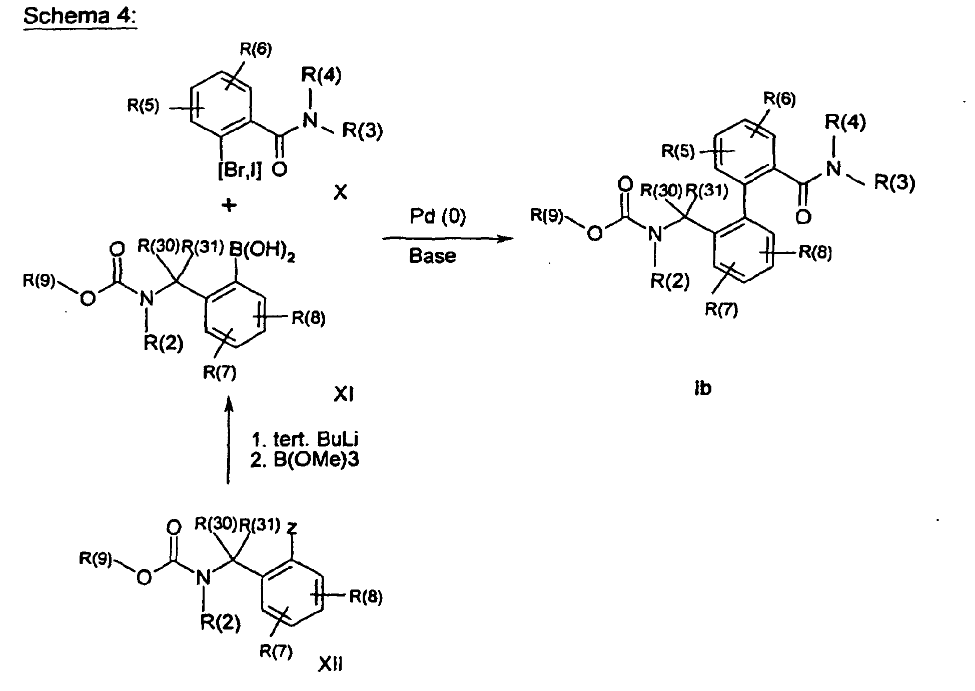

Die erfindungsgemäßen Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen.und/oder Hilfsstoffen und, wenn gewünscht, in Kombination mit anderen Arzneimittetwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauferkrankungen vorteilhafte Kombinationspartner kommen beispielsweise andere Antiarrhythmika, so Klasse I-, Klasse II- oder Klasse III-Antiarrhythmika, in Frage, wie beispielsweise IKₛ- oder IKᵣ-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Austausch-lnhibitoren, Calciumkanalantagonisten, Phosphodiesterasehemmer und andere positiv inotrop wirkende Stoffe, wie z. B. Digitalisglykoside, oder Diuretika.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wäßrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht.

Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

### Experimenteller Teil

### Liste der Abkürzungen

- CDI: Carbonyldiimidazol
- DIC: Diisopropylcarbodiimid
- DMAP: 4-Dimethylaminopyridin
- DMF: N,N-Dimethylformamid
- EDAC: N-Ethyl-N'-(3-dimethylaminopropyl)-carbodiimid Hydrochlorid
- EE: Essigsäureethylester
- F.p.: Schmelzpunkt (Wenn nicht anders angegeben sind die Schmelzpunkte der ungereinigten Rohprodukte angegeben; die Schmelzpunkte der jeweiligen Reinsubstanzen können durchaus deutlich höher liegen)
- HOBT: 1-Hydroxy-1H-benzotriazol
- i. Vak.: im Vakuum
- LM: Lösungsmittel
- Me: Methyl
- RT: Raumtemperatur
- THF: Tetrahydrofuran
- TOTU: O-[(Cyano(ethoxycarbonyl)methylen)amino]-1,1,3,3-tetramethyluroniumtetrafluoroborat

### Vorstufe 1: 7H-Dibenzo[c,e]oxepin-5-on

Zu einer Suspension von 50,0 g (0,22 mol) Diphensäureanhydrid in 220 ml DMF wurden bei 5°C 9,0 g (0,24 mol) Natriumboranat innerhalb von 10 min portionsweise zugefügt. Nach 1 h Rühren bei RT wurde die Reaktionsmischung auf 220 ml 6M Salzsäure gegossen, mit 750 ml Wasser verdünnt und 2 h nachgerührt. Der ausgefallene Niederschlag wurde abgesaugt und man erhielt 35,0 g 7H-Dibenzo[c,e]oxepin-5-on; Fp. 131°C.

### Vorstufe 2: 2'-Phthaloimidomethyl-biphenyl-2-carbonsäure

Eine Mischung aus 35 g (0,17 mol) 7H-Dibenzo[c,e]oxepin-5-on und 30,8 g (0,17 mol) Kaliumphthalimid in 330 ml DMF wurde 18 h auf 170°C erhitzt. Nach dem Abkühlen wurde der ausgefallene Niederschlag abgesaugt und in 160 ml Eisessig eingetragen. Nach 1 h Rühren wurde mit 650 ml Eiswasser verdünnt und das ausgefallene Produkt abgesaugt und im Vakuum getrocknet. Man erhielt 44,8 g 2'-Phthaloimidomethyl-biphenyl-2-carbonsäure; Fp. 198°C.

### Vorstufe 3: 2'-Aminomethyl-biphenyl-2-carbonsäure

Eine Suspension von 10,0 g (28 mmol) 2'-Phthaloimidomethyl-biphenyl-2-carbonsäure in 450 ml Methanol wurde mit 20 ml Hydrazinhydrat versetzt und 1,5 h auf 40°C erwärmt. Die Reaktionsmischung wurde eingeengt und der Rückstand in 250 ml Methylenchlorid aufgenommen. Nach Abfiltrieren des ungelösten 2,3-Dihydro-phthalazin-1,4-dions wurde die Mutterlauge eingeengt und man erhielt 4,8 g 2'-Aminomethyl-biphenyl-2-carbonsäure.

### Allgemeine Vorschrift zur Synthese von gemischten Succinimidocarbonaten aus Alkoholen (Vorstufen 4 a - 4 k)

Zu einer Lösung von 19,5 mmol des entsprechenden Alkohols und 1,2 g (9,8 mmol) DMAP in 30 ml Methylenchlorid und 30 ml Acetonitril werden bei 0°C 5,0 g (19,5 mmol) Disuccinimidocarbonat portionsweise zugegeben. Nach 2,5 bis 10 h Rühren bei RT werden 25 ml Wasser zugegeben und die organische Phase wird noch 2mal mit Wasser gewaschen. Nach Trocken und Einengen erhält man die entsprechenden Succinimidocarbonate, meist als kristalline Feststoffe.

### Vorstufe 4 a:

Nach der allgemeinen Vorschrift wurden 3,2 g 4-Fluorbenzyl-N-succinimidocarbonat erhalten; Fp. 89°C (Ether).

### Vorstufe 4 b:

Aus 11,7 mmol 4-Trifluormethylbenzylalkohol wurden entsprechend der allgemeinen Vorschrift 2,3 g 4-Trifluormethylbenzyl-N-succinimidocarbonat erhalten; Fp. 102°C (Ether).

### Vorstufe 4 c:

Aus 10,5 mmol α-Methyl-4-(trifluormethyl)-benzylalkohol wurden entsprechend der allgemeinen Vorschrift 1,6 g α-Methyl-4-(trifluormethyl)benzyl-N-succinimidocarbonat erhalten; Fp. 115°C (Ether).

### Vorstufe 4 d:

Aus 19,5 mmol 4,4,4-Tritluorbutanol wurden entsprechend der allgemeinen Vorschrift 4,0 g 4,4,4-Trifluorbutyl-N-succinimidocarbonat erhalten; F.p. 72°C (Ether).

### Vorstufe 4 e:

Aus 26,3 mmol α-Methyl-3-(trifluormethyl)-benzylalkohol wurden entsprechend der allgemeinen Vorschrift 5,1 g α-Methyl-3-(trifluormethyl)benzyl-N-succinimidocarbonat erhalten; Fp. 77°C (Ether).

### Vorstufe 4 f:

Aus 31,6 mmol α-Methyl-2,6-difluorbenzylalkohol wurden entsprechend der allgemeinen Vorschrift 1,6 g α-Methyl-2,6-difluorbenzyl-N-succinimidocarbonat erhalten; Fp. 108°C (Ether).

### Vorstufe 4 g:

Aus 25 mmol α-Methyl-2-(trifluormethyl)-benzylalkohol wurden entsprechend der allgemeinen Vorschrift 3,5 g α-Methyl-2-(trifluormethyl)benzyl-N-succinimidocarbonat erhalten.

### Vorstufe 4 h:

Aus 25 mmol (S)-1-Phenylethanol wurden entsprechend der allgemeinen Vorschrift 3,5 g (S)-α-Methyl-benzyl-N-succinimidocarbonat erhalten.

### Vorstufe 4 i:

Aus 25 mmol (R)-1-Phenylethanol wurden entsprechend der allgemeinen Vorschrift 3,5 g (R)-α-Methyl-benzyl-N-succinimidocarbonat erhalten.

### Vorstufe 4 j:

Aus 25 mmol α-Methyl-4-fluorbenzylalkohol wurden entsprechend der allgemeinen Vorschrift 4,3 g α-Methyl-4-fluorbenzyl-N-succinimidocarbonat erhalten.

### Vorstufe 4 k:

Aus 9,8 mmol (S)-1-Phenyl-1-butanol wurden entsprechend der allgemeinen Vorschrift 1,7 g (S)-α-Propyl-benzyl-N-succinimidocarbonat erhalten.

### Vorstufe 5 a: 2'-Aminomethyl-biphenyl-2-carbonsäure-phenethylamid

Aus 2'-Phthaloimidomethyl-biphenyl-2-carbonsäure (Vorstufe 2) wurde nach Aktivierung mit CDI und Umsetzung mit Phenethylamin 2'-Phthaloimidomethyl-biphenyl-2-carbonsäure-phenethylamid erhalten; Fp. 156°C.
5,0 g (10,9 mmol) des Produktes wurden in 200 ml Methanol gelöst und mit 5 ml Hydrazinhydrat versetzt. Nach 1 h Rühren bei 40°C wurde die Reaktionsmischung eingeengt und der Rückstand in Methylenchlorid aufgenommen. Nach Abfiltrieren des gebildeten 2,3-Dihydro-phthalazin-1,4-dions wurde die Mutterlauge eingeengt und der Rückstand durch Flashchromatographie mit Methylenchlorid/Methanol 20:1 gereinigt. Man erhielt 3 g 2'-Aminomethyl-biphenyl-2-carbonsäure-phenethylamid.

### Vorstufe 5 b: 2'-Aminomethyl-biphenyl-2-carbonsäure-benzylamid

Aus 2'-Phthaloimidomethyl-biphenyl-2-carbonsäure (Vorstufe 2) wurde nach Überführung in das Säurechlorid mit Thionylchlorid und Umsetzung mit Benzylamin 2'-Phthaloimidomethyl-biphenyl-2-carbonsäure-benzylamid erhalten. 1,2 g (2,7 mmol) des Produktes wurden in 55 ml Methanol gelöst und mit 1,35 ml Hydrazinhydrat versetzt. Nach 1 h Rühren bei 40°C wurde die Reaktionsmischung eingeengt und der Rückstand in Methylenchlorid aufgenommen. Nach Abfiltrieren des gebildeten 2,3-Dihydro-phthalazin-1,4-dions wurde die Mutterlauge eingeengt und der Rückstand durch Flashchromatographie mit Methylenchlorid/Methanol 30:1 gereinigt. Man erhielt 0,49 g 2'-Aminomethyl-biphenyl-2-carbonsäure-benzylamid.

### Vorstufe 5 c: 2'-Aminomethyl-biphenyl-2-carbonsäure-isopentylamid

Aus 3 g (8,4 mmol) 2'-Phthaloimidomethyl-biphenyl-2-carbonsäure (Vorstufe 2) wurde durch Umsetzung mit Isopentylamin in Gegenwart von HOBT und DIC 3,2 g 2'-Phthaloimidomethyl-biphenyl-2-carbonsäure-isopentylamid erhalten; Fp. 169°C. Das Produkt wurde in 100 ml Methanol gelöst und mit 5 ml Hydrazinhydrat versetzt. Nach 1 h Rühren bei 40°C wurde die abgekühlte Reaktionsmischung filtriert. Das Filtrat wurde eingeengt und der Rückstand in Methylenchlorid aufgenommen. Nach Waschen mit Wasser, Trocknen und Einengen erhielt man 1,8 g 2'-Aminomethyl-biphenyl-2-carbonsäure-isopentylamid.

### Vorstufe 5 d: 2'-Aminomethyl-biphenyl-2-carbonsäure-2-(2-pyridyl)-ethylamid

Aus 10 g (28 mmol) 2'-Phthaloimidomethyl-biphenyl-2-carbonsäure (Vorstufe 2) wurde durch Umsetzung mit 2-(2-Pyridyl)-ethylamin in Gegenwart von HOBT und DIC 13 g 2'-Phthaloimidomethyl-biphenyl-2-carbonsäure-2-(2-pyridyl)-ethylamid erhalten; Fp. 155°C. Das Produkt wurde in 300 ml Methanol suspendiert und mit 20 ml Hydrazinhydrat versetzt. Nach 1 h Rühren bei 40°C wurde die abgekühlte Reaktionsmischung filtriert. Das Filtrat wurde eingeengt und der Rückstand in EE aufgenommen. Das Produkt wurde 2mal mit 2M Salzsäure in die wäßrige Phase extrahiert. Anschließend wurde die wäßrige Phase mit Kaliumcarbonat alkalisch gestellt und 2 mal mit EE extrahiert. Nach Waschen mit Wasser, Trocknen und Einengen erhielt man 7,3 g 2'-Aminomethyl-biphenyl-2-carbonsäure-2-(2-pyridyl)-ethylamid.

### Vorstufe 6: 2'-(Benzyloxycarbonyl-aminomethyl)-biphenyl-2-carbonsäure

Zu einer Lösung von 455 mg (2 mmol) 2'-Aminomethyl-biphenyl-2-carbonsäure (Vorstufe 3) und 336 mg (4 mmol) Natriumhydrogencarbonat in 5 ml Dioxan und 5 ml Wasser wurden 500 mg (2 mmol) Benzyl-N-succinimidocarbonat gelöst in 2,5 ml Dioxan bei 0°C zugetropft. Nach 4 h Rühren bei RT wurde im Vakuum eingeengt, mit Wasser verdünnt, angesäuert und mit Essigester extrahiert. Man erhielt 590 mg 2'-(Benzyloxycarbonyl-aminomethyl)-biphenyl-2-carbonsaure.

### Vorstufe 7: 2'-(tert.-Butoxycarbonylaminomethyl)-biphenyl-2-carbonsäure

Zu einer Lösung von 12,0 g (53 mmol) 2'-Aminomethyl-biphenyl-2-carbonsäure (Vorstufe 3) in 130 ml 1,4-Dioxan und 65 ml Wasser wurden 65 ml 1M Natronlauge gegeben und nach vollständigem Auflösen wurden 12,6 g (58 mmol) Di-tert.-butyldicarbonat hinzugefügt. Nach 2 h Rühren bei RT wurde im Vakuum eingeengt, mit Wasser verdünnt und 2mal mit Methylenchlorid extrahiert. Die wäßrige Phase wurde mit 1 M Kaliumhydrogensulfatlösung angesäuert und mit Essigester extrahiert. Nach weitgehendem Einengen, Zugabe von n-Heptan und Stehenlassen über Nacht, fiel das Produkt aus und man erhielt 7,6 g 2'-(tert.-Butoxycarbonylaminomethyl)-biphenyl-2-carbonsäure; Fp. 136°C.

### Allgemeine Vorschrift zur Abspaltung der Boc-Schutzgruppe :

Zu einer Lösung von Trifluoressigsäure in Dichlormethan (30 %ig) wurde das N-Boc geschützte Aminomethylbiphenylderivat (1 g auf 10 ml der Lösung) hinzugegeben. Das Gemisch wurde bei Raumtemperatur für 30 Minuten gerührt und dann das Lösungsmittel am Rotationsverdampfer im Vakuum entfernt. Der Rückstand wurde in Essigester aufgenommen und mit gesättigter Natriumhydrogencarbonatlösung gewaschen. Die organische Phase wurde über Magnesiumsulfat getrocknet, das Lösungsmittel im Vakuum entfernt und man erhielt die entsprechenden 2'-Aminomethyl-biphenyl-2-carbonsäureamide.

### Vorstufe 8 a: 2'-Aminomethyl-biphenyl-2-carbonsäure-(2,4-difluorbenzyl)-amid

Die Verbindung wurde erhalten aus der Boc-geschützten Verbindung (Beispiel 8 c) gemäß der allgemeinen Vorschrift. Alternativ kann die Verbindung auch direkt als Trifluoracetat isoliert und weiter umgesetzt werden.

### Weitere Vorstufen 8:

Analog wurden aus den Boc-geschützen Verbindungen der Beispiele 8d - 8o und 10 a - 10 o die entsprechenden Amine freigesetzt.

### Allgemeine Vorschrift zur Umsetzung von Aminomethylbiphenylen mit Succinimidocarbonaten zu Carbamaten (Beispiele 1 a bis 1 u)

Zu einer Lösung von 0,45 mmol des jeweiligen 2'-Aminomethylbiphenyls und 38 mg (0,45 mmol) Natriumhydrogencarbonat in 2 ml Dioxan und 2 ml Wasser werden 0,45 mmol des jeweiligen Succinimidocarbonats gelöst in 2 ml Dioxan langsam zugetropft. Man rührt 2 bis 12 h bei RT, engt ein, verdünnt mit Wasser, extrahiert mit EE und wäscht die organische Phase mit Wasser. Nach Trocknen und Einengen erhält man die entsprechenden Carbamate.

### Beispiel 1 a: 2'-(4-Trifluormethylbenzyloxycarbonyl-aminomethyl)-biphenyl-2-carbonsäure-phenethylamid

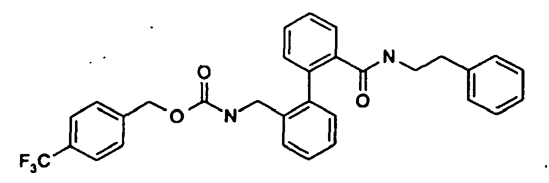

Aus 0,45 mmol 2'-Aminomethyl-biphenyl-2-carbonsäure-phenethylamid und 4-Trifluormethylbenzyl-N-succinimidocarbonat (Vorstufe 4b) wurden gemäß der allgemeinen Arbeitsvorschrift 226 mg 2'-(4-Triftuormethylbenzytoxycarbonyl-aminomethyl)-biphenyl-2-carbonsäure-phenethylamid erhalten. MS (ES+): m/z = 533 (M+1).

### Beispiel 1 b: 2'-(Benzyloxycarbonyl-aminomethyl)-biphenyl-2-carbonsäurephenethylamid

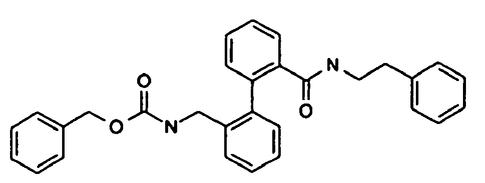

Aus 0,3 mmol 2'-Aminomethyl-biphenyl-2-carbonsäure-phenethylamid und Benzyl-N-succinimidocarbonat wurden gemäß der allgemeinen Arbeitsvorschrift 66 mg 2'-(Benzyloxycarbonyl-aminomethyl)-biphenyl-2-carbonsäure-phenethylamid als Öl erhalten. MS (ES+): m/z = 456 (M+1).

### Beispiel 1 c: 2'-(Methylsulfonylethyloxycarbonyl-aminomethyl)-biphenyl-2-carbonsäure-phenethylamid

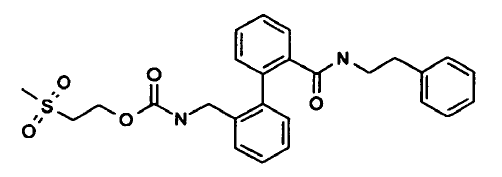

Aus 0,45 mmol 2'-Aminomethyl-biphenyl-2-carbonsäure-phenethylamid (Vorstufe 5a) und Methylsulfonylethyl-N-succinimidocarbonat wurden gemäß der allgemeinen Arbeitsvorschrift 164 mg 2'-(Methylsulfonylethyloxycarbonyl-aminomethyl)-biphenyl-2-carbonsäure-phenethylamid als Öl erhalten. MS (ES+): m/z = 481 (M+1).

### Beispiel 1 d: 2'-(4-Trifluormethylbenzyloxycarbonyl-aminomethyl)-biphenyl-2-carbonsäure-2-(2-pyridyl)-ethylamid

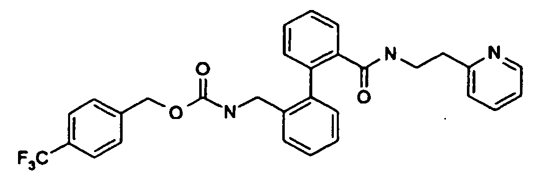

Aus 0,3 mmol 2'-Aminomethyl-biphenyl-2-carbonsäure-2-(2-pyridyl)-ethylamid (Vorstufe 5 d) und 4-Trifluormethylbenzyl-N-succinimidocarbonat (Vorstufe 4 b) wurden gemäß der allgemeinen Arbeitsvorschrift 170 mg 2'-(4-Trifluormethylbenzyloxycarbonyl-aminomethyl)-biphenyl-2-carbonsäure-2-(2-pyridyl)-ethylamid erhalten. MS (ES+): m/z = 534 (M+1).

### Beispiel 1 e: 2'-(4-Fluorbenzyloxycarbonyl-aminomethyl)-biphenyl-2-carbonsäure-2-(2-pyridyl)-ethylamid

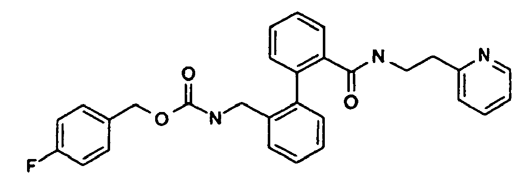

Aus 0,3 mmol 2'-Aminomethyl-biphenyl-2-carbonsäure-2-(2-pyridyl)-ethylamic (Vorstufe 5 d) und 4-Fluorbenzyl-N-succinimidocarbonat (Vorstufe 4 a) wurden gemäß der allgemeinen Arbeitsvorschrift 150 mg 2'-(4-Fluorbenzyloxycarbonyl-aminomethyl)-biphenyl-2-carbonsäure-2-(2-pyridyl)-ethylamid erhalten. MS (ES+): m/z = 484 (M+1).

### Beispiel 1 f: (±)-2'-(α-Methyl-4-(trifluormethyl)-benzyloxycarbonyl-aminomethyl)-biphenyl-2-carbonsäure-2-(2-pyridyl)-ethylamid

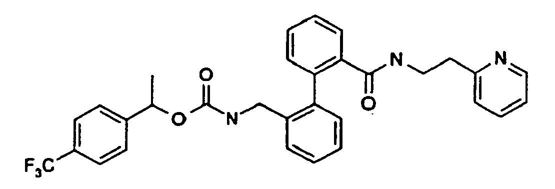

Aus 0,3 mmol 2'-Aminomethyl-biphenyl-2-carbonsäure-2-(2-pyridyl)-ethylamid (Vorstufe 5 d) und α-Methyl-4-(trifluormethyl)-benzyl-N-succinimidocarbonat (Vorstufe 4 c) wurden gemäß der allgemeinen Arbeitsvorschrift 170 mg 2'-(α-Methyl-4-(trifluormethyl)-benzyloxycarbonyl-aminomethyl)-biphenyl-2-carbonsäure-2-(2-pyridyl)-ethylamid als Racemat erhalten. MS (ES+): m/z = 548 (M+1).

### Beispiel 1 g: (S)-2'-(α-Methyl-4-(trifluormethyl)-benzyloxycarbonyl-aminomethyl)-biphenyl-2-carbonsäure-2-(2-pyridyl)-ethylamid

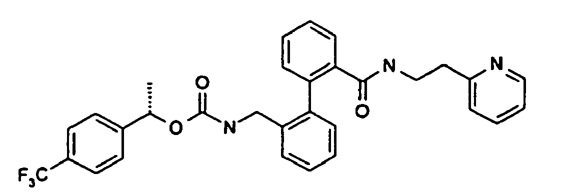

Das S-Enantiomer wurde erhalten aus dem entsprechenden Racemat (Beispiel 1 f) durch präparative HPLC über eine Chiralpak AD 250x4,6 Säule mit n-Hexan/Ethanol/Isopropanol (10:1:1, je 0,3% Trifluoressigsäure/Diethylamin) als Solvent.

### Beispiel 1 h: (R)-2'-(α-Methyl-4-(trifluormethyl)-benzyloxycarbonyl-aminomethyl)-biphenyl-2-carbonsäure-2-(2-pyridyl)-ethylamid

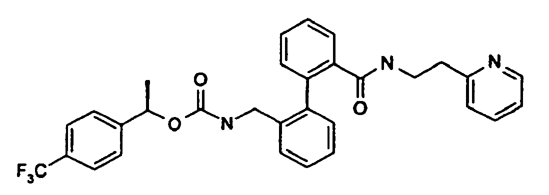

Das R-Enantiomer wurde erhalten aus dem entsprechenden Racemat (Beispiel 1f) durch präparative HPLC über eine Chiralpak AD 250x4,6 Säule mit n-Hexan/Ethanol/sopropanol (10:1:1, je 0,3% Trifluoressigsäure/Diethylamin) als Solvent.

### Beispiel 1 i: 2'-(4,4,4-Trifluorbutyloxycarbonyl-aminomethyl)-biphenyl-2-carbonsäure-2-(2-pyridyl)-ethylamid

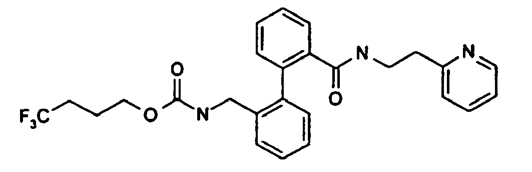

Aus 0,3 mmol 2'-Aminomethyl-biphenyl-2-carbonsäure-2-(2-pyridyl)-ethylamid (Vorstufe 5d) und 4,4,4-Trifluorbutyl-N-succinimidocarbonat (Vorstufe 4 d) wurden gemäß der allgemeinen Arbeitsvorschrift 140 mg 2'-(4,4,4-Trifluorbutyloxycarbonyl-aminomethyl)-biphenyl-2-carbonsäure-2-(2-pyridyl)-ethylamid erhalten. MS (ES+): m/z = 486 (M+1).

### Beispiel 1 j: (S)-2'-(α-Methyl-benzyloxycarbonyl-aminomethyl)-biphenyl-2-carbonsäure-2-(2-pyridyl)-ethylamid

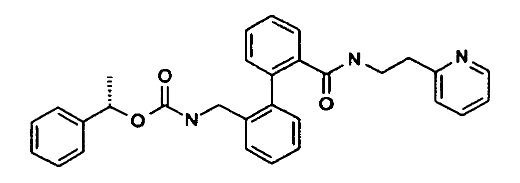

Aus 0,3 mmol 2'-Aminomethyl-biphenyl-2-carbonsäure-2-(2-pyridyl)-ethylamid (Vorstufe 5 d) und (S)-α-Methyl-benzyl-N-succinimidocarbonat (Vorstufe 4 h) wurden gemäß der allgemeinen Arbeitsvorschrift 60 mg (S)-2'-(α-Methyl-benzyloxycarbonyl-aminomethyl)-biphenyl-2-carbonsäure-2-(2-pyridyl)-ethylamid erhalten. MS (ES+): m/z = 480 (M+1).

### Beispiel 1 k: (R)-2'-(α-Methyl-benzyloxycarbonyl-aminomethyl)-biphenyl-2-carbonsäure-2-(2-pyridyl)-ethylamid

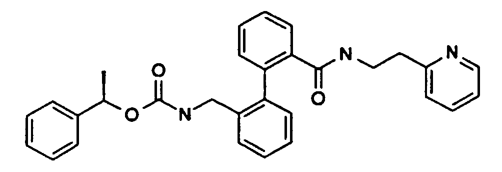

Aus 0,3 mmol 2'-Aminomethyl-biphenyl-2-carbonsäure-2-(2-pyridyl)-ethylamid (Vorstufe 5d) und (R)-α-Methyl-benzyl-N-succinimidocarbonat (Vorstufe 4i) wurden gemäß der allgemeinen Arbeitsvorschrift 60 mg (R)-2'-(α-Methyl-benryloxycarbonyl-aminomethyl)-biphenyl-2-carbonsäure-2-(2-pyridyl)-ethylamid erhalten. MS (ES+): m/z = 480 (M+1).

### Beispiele 1 l - 1 u

Gemäß der allgemeinen Arbeitvorschrift und analog den Beispielen 1 a - 1 k wurden folgende Verbindungen aus den jeweiligen Vorstufen erhalten:

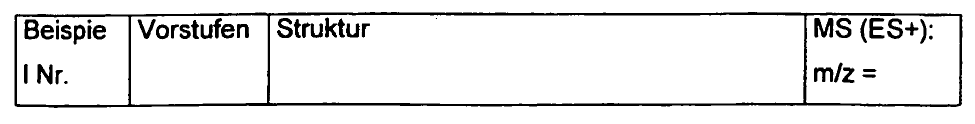

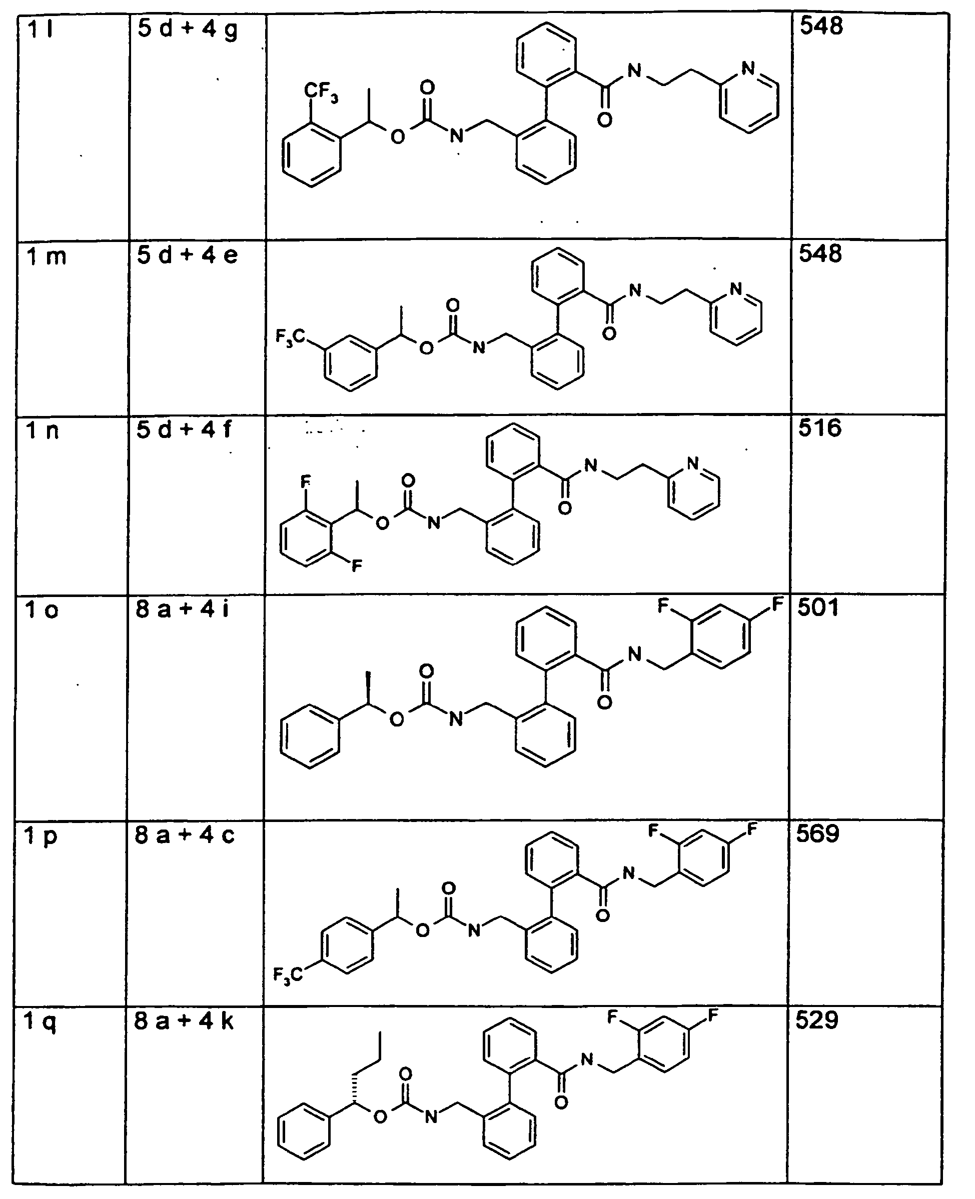

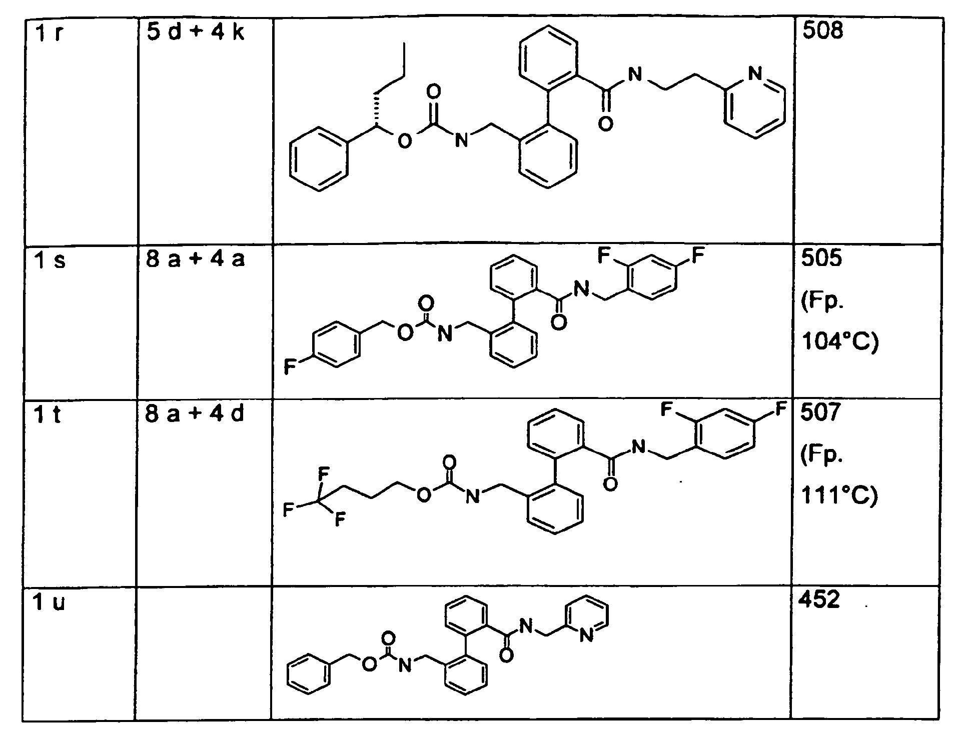

Allgemeine Vorschrift zur Umsetzung von Aminomethylbiphenylen mit Chlorameisensäureestem zu Carbamaten (Beispiele 2 a bis 2 m):
Zu einer Lösung von 0,3 mmol des jeweiligen 2'-Aminomethylbiphenyls und 37 mg (0,36 mmol) Triethylamin in 6 ml Methylenchlorid werden 0,32 mmol des jeweiligen Chlorameisensäureesters gelöst in 1 ml Methylenchlorid bei 5°C langsam zugetropft. Man läßt über Nacht bei RT rühren, gießt den Ansatz auf Wasser und wäscht die organische Phase noch einmal mit Wasser. Nach Einengen wird der Rückstand durch Flashchromatographie gereinigt.

### Beispiel 2 a: 2'-(Butoxycarbonyl-aminomethyl)-biphenyl-2-carbonsäure-2-(2-pyridyl)-ethylamid

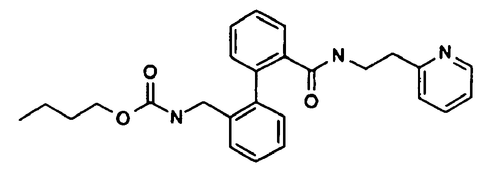

Aus 0,3 mmol 2'-Aminomethyl-biphenyl-2-carbonsäure-2-(2-pyridyl)-ethylamid (Vorstufe 5 d) und Chlorameisensäurebutylester wurden gemäß der allgemeinen Arbeitsvorschrift 69 mg 2'-(Butoxycarbonyl-aminomethyl)-biphenyl-2-carbonsäure-2-(2-pyridyl)-ethylamid als Öl erhalten. MS (ES+): m/z = 432 (M+1).

### Beispiel 2 b: 2'-(Benzyloxycarbonylamino-methyl)-biphenyl-2-carbonsäure-(3-methyl-butyl)-amid

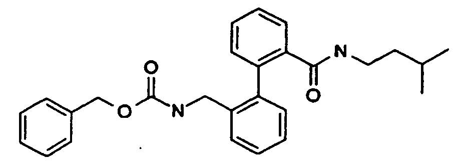

Aus 0,27 mmol 2'-Aminomethyl-biphenyl-2-carbonsäure-(3-methyl-butyl)-amid (Vorstufe 5 c) und Chlorameisensäurebenzylester wurden gemäß der allgemeinen Arbeitsvorschrift 44 mg 2'-(Benzyloxycarbonylamino-methyl)-biphenyl-2-carbonsäure-(3-methyl-butyl)-amid erhalten; Fp. 112°C. MS (ES+): m/z = 431 (M+1).

### Beispiel 2 c: 2'-(Benzyloxycarbonylamino-methyl)-biphenyl-2-carbonsäure-2-(2-pyridyl)-ethylamid

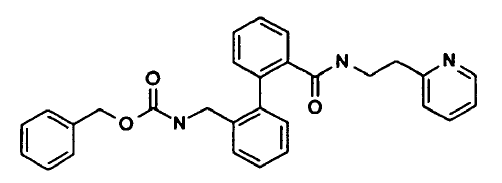

Aus 0,24 mmol 2'-Aminomethyl-biphenyl-2-carbonsäure-2-(2-pyridyl)-ethylamid (Vorstufe 5 d) und Chlorameisensäurebenzylester wurden gemäß der allgemeinen Arbeitsvorschrift 59 mg 2'-(Benzyloxycarbonylamino-methyl)-biphenyl-2-carbonsäure-2-(2-pyridyl)-ethylamid erhalten; Fp. 140°C (Heptan/EE). MS (ES+): m/z = 466 (M+1).

### Beispiel 2 d: 2'-(Butoxycarbonylamino-methyl)-biphenyl-2-carbonsäure-(3-methylbutyl)-amid

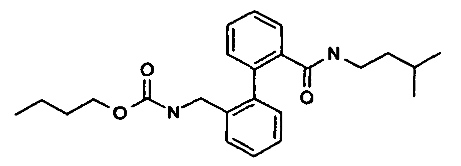

Aus 0,34 mmol 2'-Aminomethyl-biphenyl-2-carbonsäure-(3-methyl-butyl)-amid (Vorstufe 5 c) und Chlorameisensäurebutylester wurden gemäß der allgemeinen Arbeitsvorschrift 66 mg 2'-(Butoxycarbonylamino-methyl)-biphenyl-2-carbonsäure-(3-methyl-butyl)-amid als Harz erhalten. MS (ES+): m/z = 397 (M+1).

### Beispiel 2 e: 2'-(2-Chlorbenzyloxycarbonylamino-methyl)-biphenyl-2-carbonsäure-(3-methyl-butyl)-amid

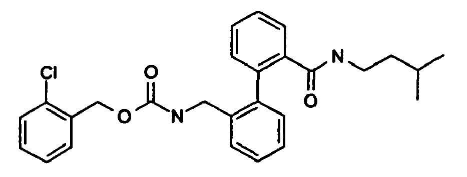

Aus 0,34 mmol 2'-Aminomethyl-biphenyl-2-carbonsäure-(3-methyl-butyl)-amid (Vorstufe 5c) und Chlorameisensäure-(2-chlorbenzyl)-ester wurden gemäß der allgemeinen Arbeitsvorschrift 75 mg 2'-(2-Chlorbenzyloxycarbonylamino-methyl)-biphenyl-2-carbonsäure-(3-methyl-butyl)-amid als Harz erhalten. MS (ES+): m/z = 465 (M+1).

### Beispiel 2 f. 2'-(Methoxycarbonylamino-methyl)-biphenyl-2-carbonsäure-(3-methylbutyl)-amid

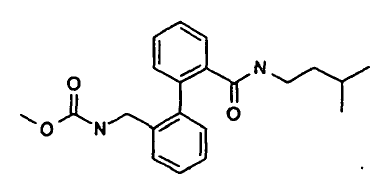

Aus 0,34 mmol 2'-Aminomethyl-biphenyl-2-carbonsäure-(3-methyl-butyl)-amid (Vorstufe 5 c) und Chlorameisensäuremethylester wurden gemäß der allgemeinen Arbeitsvorschrift gefolgt von Extraktion mit EE und Reinigung durch Flashchromatographie 29 mg 2'-(Methoxycarbonylamino-methyl)-biphenyl-2-carbonsäure-(3-methyl-butyl)-amid als Harz erhalten. MS (ES+): m/z = 355 (M+1).

### Beispiel 2 g: 2'-(Phenoxycarbonylamino-methyl)-biphenyl-2-carbonsäure-(3-methylbutyl)-amid

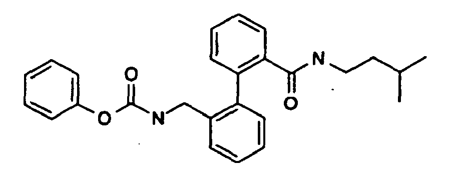

Aus 0,34 mmol 2'-Aminomethyl-biphenyl-2-carbonsäure-(3-methyl-butyl)-amid (Vorstufe 5 c) und Chlorameisensäurephenylester wurden gemäß der allgemeinen Arbeitsvorschrift gefolgt von Extraktion mit EE und Reinigung durch Flashchromatographie'55 mg 2`-(Phenoxycarbonylamino-methyl)-biphenyl-2-carbonsäure-(3-methyl-butyl)-amid als Harz erhalten. MS (ES+): m/z = 417 (M+1).

### Beispiel 2 h: 2'-(4-Carbomethoxy-phenoxycarbonylamino-methyl)-biphenyl-2-carbonsäure-(3-methyl-butyl)-amid

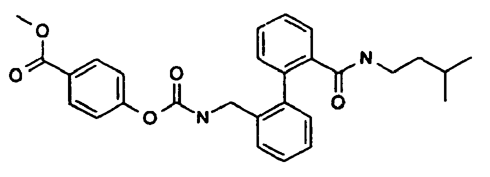

Aus 0,34 mmol 2'-Aminomethyl-biphenyl-2-carbonsäure-(3-methyl-butyl)-amid (Vorstufe 5 c) und Chlorameisensäure-(4-carbomethoxy)-phenylester wurden gemäß der allgemeinen Arbeitsvorschrift gefolgt von Extraktion mit EE und Reinigung durch Flashchromatographie 77 mg 2'-(4-Carbomethoxyphenoxycarbonylamino-methyl)-biphenyl-2-carbonsäure-(3-methyl-butyl)-amid als Harz erhalten. MS (ES+): m/z = 475 (M+1).

### Beispiel 2 i: 2'-(2,2-Dimethylpropoxycarbonylamino-methyl)-biphenyl-2-carbonsäure-phenethylamid

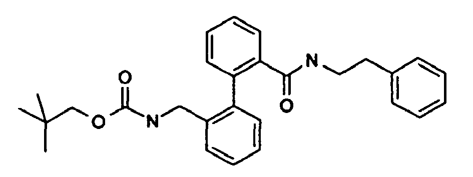

Aus 0,45 mmol 2'-Aminomethyl-biphenyl-2-carbonsäure-phenethylamid (Vorstufe 5 a) und Neopentylchloroformiat wurden gemäß der allgemeinen Arbeitsvorschrift gefolgt von Extraktion mit EE und Reinigung durch Flashchromatographie 156 mg 2'-(2,2-Dimethylpropoxycarbonylamino-methyl)-biphenyl-2-carbonsäurephenethylamid erhalten. MS (ES+): m/z = 445 (M+1).

### Beispiele 2 j -2 m

Analog Beispielen 2 a - 2 i wurden folgende Verbindungen erhalten:

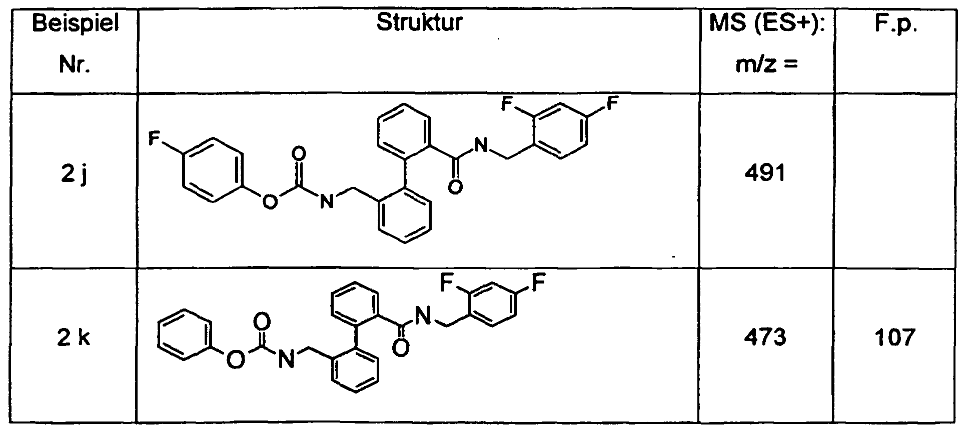

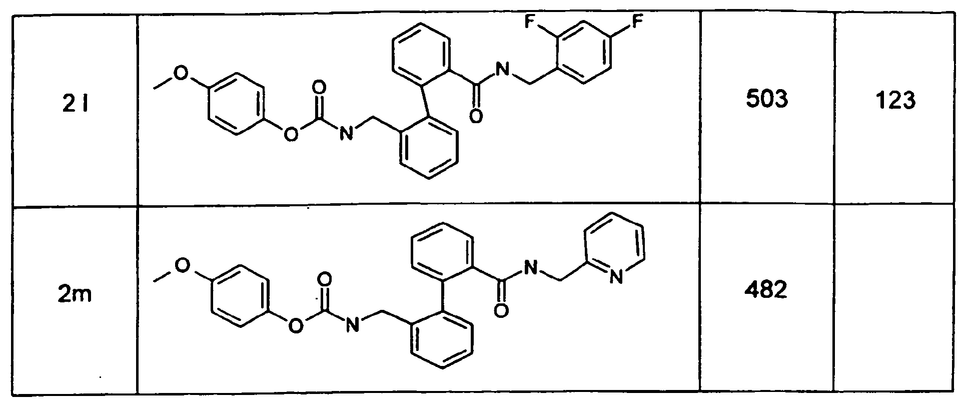

Allgemeine Vorschrift zur Umsetzung von Aminomethylbiphenylen mit Sulfonsäurechloriden zu Sulfonamiden (Beispiele 3 a bis 3 t):
Zu einer Lösung von 0,61 mmol des jeweiligen 2'-Aminomethylbiphenyls und 74 mg (0,73 mmol) Triethylamin in 5 ml Methylenchlorid werden 0,66 mmol des jeweiligen Sulfonsäurechlorids bei 0°C langsam zugetropft. Nach 12 h Rühren bei RT wird die Reaktionsmischung im Vakuum eingeengt, der Rückstand wird mit 25 ml Wasser 2 h verrührt und das auskristallisierte Produkt wird abgesaugt.

### Beispiel 3 a: 2'-(3-Trifluormethylphenylsulfonyl-aminomethyl)-biphenyl-2-carbonsäurephenethylamid

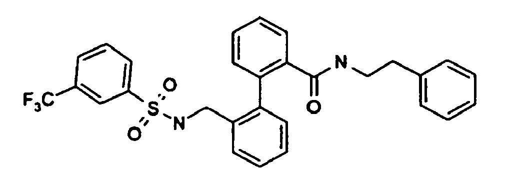

Aus 0,61 mmol 2'-Aminomethyl-biphenyl-2-carbonsäüre-phenethylamid (Vorstufe 5 a) und 3-Trifluormethylphenylsulfonylchlorid wurden gemäß der allgemeinen Arbeitsvorschrift 272 mg 2'-(3-Trifluormethylphenylsulfonyl-aminomethyl)-biphenyl-2-carbonsäurephenethylamid erhalten; Fp. 145°C. MS (ES+): m/z = 539 (M+1).

### Beispiel 3 b: 2'-(4-Acetylphenylsulfonyl-aminomethyl)-biphenyl-2-carbonsäurephenethylamid

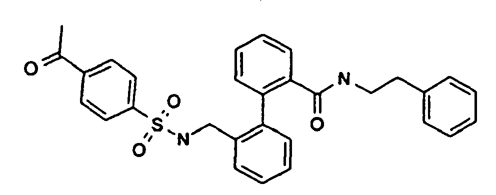

Aus 0,61 mmol 2'-Aminomethyl-biphenyl-2-carbonsäure-phenethylamid (Vorstufe 5 a) und 4-Acetylphenylsulfonylchlorid wurden gemäß der allgemeinen Arbeitsvorschrift 258 mg 2'-(4-Acetylphenylsulfonylaminomethyl)-biphenyl-2-carbonsäurephenethylamid erhalten; Fp. 145°C. MS (ES+): m/z = 513 (M+1).

### Beispiel 3 c: 2'-(3-Nitrophenylsulfonyl-aminomethyl)-biphenyl-2-carbonsäurephenethylamid

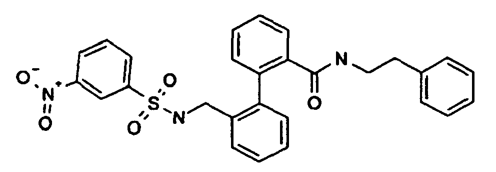

Aus 0,61 mmol 2'-Aminomethyl-biphenyl-2-carbonsäure-phenethylamid (Vorstufe 5 a) und 3-Nitrophenylsulfonylchlorid wurden gemäß der allgemeinen Arbeitsvorschrift 272 mg 2'-(3-Nitrophenylsulfonylaminomethyl)-biphenyl-2-carbonsäurephenethylamid erhalten; Fp. 145°C. MS (ES+): m/z = 516 (M+1).

### Beispiel 3 d: 2'-(Phenylsulfonyl-aminomethyl)-biphenyl-2-carbonsäurephenethylamid

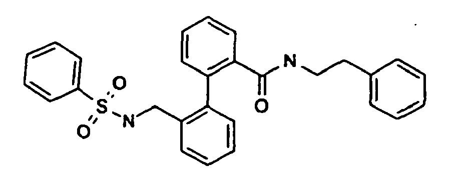

Aus 0,61 mmol 2'-Aminomethyl-biphenyl-2-carbonsäure-phenethylamid (Vorstufe 5 a) und Phenylsulfonylchlorid wurden gemäß der allgemeinen Arbeitsvorschrift 224 mg 2'-(Phenylsulfonyl-aminomethyl)-biphenyl-2-carbonsäurephenethylamid erhalten; Fp. 154°C. MS (ES+): m/z = 471 (M+1).

### Beispiel 3 e: 2'-(3-Fluorphenylsulfonyl-aminomethyl)-biphenyl-2-carbonsäurephenethylamid

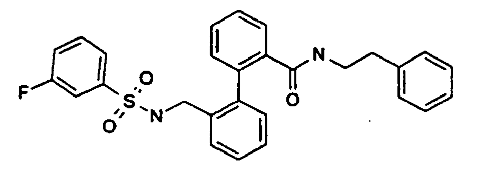

Aus 0,61 mmol 2'-Aminomethyl-biphenyl-2-carbonsäure-phenethylamid (Vorstufe 5 a) und 3-Fluorphenylsulfonylchlorid wurden gemäß der allgemeinen Arbeitsvorschrift 221 mg 2'-(3-Fluorphenylsulfonylaminomethyl)-biphenyl-2-carbonsäurephenethylamid erhalten; Fp. 153°C. MS (ES+): m/z = 489 (M+1).

### Beispiel 3 f: 2'-(4-Ethylphenylsulfonyl-aminomethyl)-biphenyl-2-carbonsäurephenethylamid

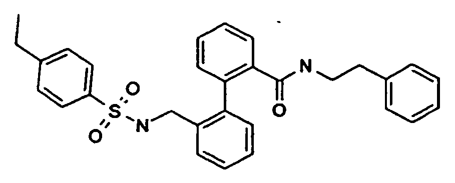

Aus 0,61 mmol 2'-Aminomethyl-biphenyl-2-carbonsäure-phenethylamid (Vorstufe 5 a) und 4-Ethylphenylsulfonylchlorid wurden gemäß der allgemeinen Arbeitsvorschrift 250 mg 2'-(4-Ethylphenylsulfonylaminomethyl)-biphenyl-2-carbonsäurephenethylamid erhalten; Fp. 163°C. MS (ES+): m/z = 499 (M+1).

### Beispiel 3 g: 2'-(3-Trifluormethylphenylsulfonyl-aminomethyl)-biphenyl-2-carbonsäurebenzylamid

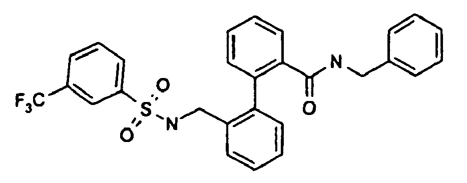

Aus 0,28 mmol 2'-Aminomethyl-biphenyl-2-carbonsäure-benzylamid (Vorstufe 5 b) und 3-Trifluormethylphenylsulfonylchlorid wurden gemäß der allgemeinen Arbeitsvorschrift 131 mg 2'-(3-Trifluormethylphenylsulfonyl-aminomethyl)-biphenyl-2-carbonsäurebenzylamid erhalten; Fp. 126°C. MS (ES+): m/z = 525 (M+1).

### Beispiel 3 h: 2'-(3-Acetylphenylsulfonyl-aminomethyl)-biphenyl-2-carbonsäurebenzylamid

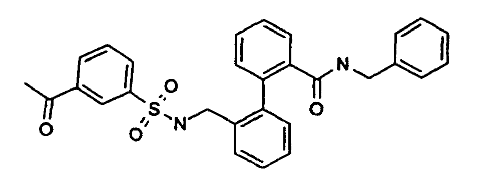

Aus 0,28 mmol 2'-Aminomethyl-biphenyl-2-carbonsäure-benzylamid (Vorstufe 5 b) und 3-Acetylphenylsulfonylchlorid wurden gemäß der allgemeinen Arbeitsvorschrift 110 mg 2'-(3-Acetylphenylsulfonylaminomethyl)-biphenyl-2-carbonsäurebenzylamid erhalten; Fp. 182°C. MS (ES+): m/z = 499 (M+1).

### Beispiel 3 i: 2'-(3-Nitrophenylsulfonyl-aminomethyl)-biphenyl-2-carbonsäurebenzylamid

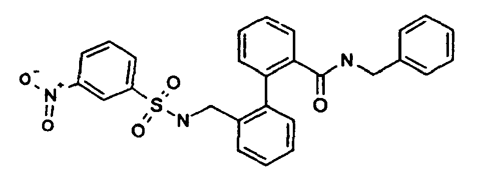

Aus 0,28 mmol 2'-Aminomethyl-biphenyl-2-carbonsäure-benzylamid (Vorstufe 5 b) und 3-Nitrophenylsulfonylchlorid wurden gemäß der allgemeinen Arbeitsvorschrift 115 mg 2'-(3-Nitrophenylsulfonylaminomethyl)-biphenyl-2-carbonsäurebenzylamid erhalten; Fp. 175°C. MS (ES+): m/z = 502 (M+1).

### Beispiel 3 j: 2'-(3-Phenylsulfonyl-aminomethyl)-biphenyl-2-carbonsäurebenzylamid

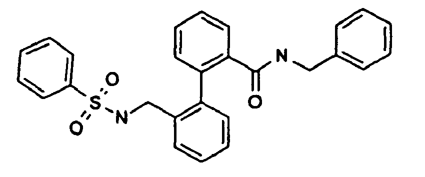

Aus 0,28 mmol 2'-Aminomethyl-biphenyl-2-carbonsäure-benzylamid (Vorstufe 5 b) und Phenylsulfonylchlorid wurden gemäß der allgemeinen Arbeitsvorschrift 95 mg 2'-(Phenylsulfonyl-aminomethyl)-biphenyl-2-carbonsäurebenzylamid erhalten; Fp. 162°C. MS (ES+): m/z = 457 (M+1).

### Beispiel 3 k: 2'-(3-Fluorphenylsulfonyl-aminomethyl)-biphenyl-2-carbonsäurebenzylamid

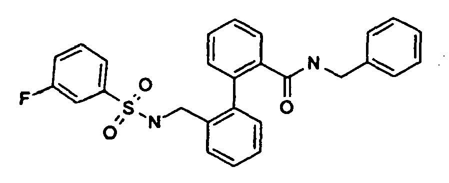

Aus 0,28 mmol 2'-Aminomethyl-biphenyl-2-carbonsäure-benzylamid (Vorstufe 5 b) und 3-Fluorphenylsulfonylchlorid wurden gemäß der allgemeinen Arbeitsvorschrift 112 mg 2'-(3-Fluorphenylsulfonylaminomethyl)-biphenyl-2-carbonsäurebenzylamid erhalten; Fp. 147°C. MS (ES+): m/z = 475 (M+1).

### Beispiel 3 l: 2'-(Phenylsulfonylamino-methyl)-biphenyl-2-carbonsäure-(3-methylbutyl)-amid

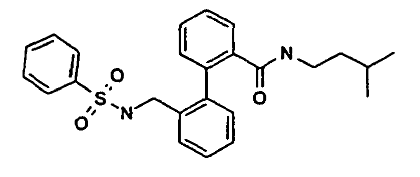

Aus 0,34 mmol 2'-Aminomethyl-biphenyl-2-carbonsäure-(3-methyl-butyl)-amid (Vorstufe 5 c) und Phenylsulfonylchlorid wurden gemäß der allgemeinen. Arbeitsvorschrift 100 mg 2'-(Phenylsulfonylamino-methyl)-biphenyl-2-carbonsäure-(3-methyl-butyl)-amid erhalten; Fp. 127°C. MS (ES+): mlz = 437 (M+1).

### Beispiel 3 m: 2'-(4-Fluorphenylsulfonylamino-methyl)-biphenyl-2-carbonsäure-(3-methyl-butyl)-amid

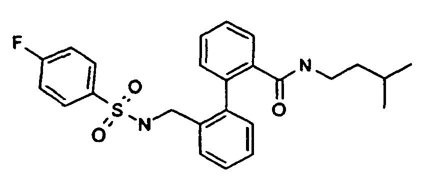

Aus 0,34 mmol 2'-Aminomethyl-biphenyl-2-carbonsäure-(3-methyl-butyl)-amid (Vorstufe 5 c) und 4-Fluorphenylsulfonylchlorid wurden gemäß der allgemeinen Arbeitsvorschrift 122 mg 2'-(4-Fluorphenylsulfonylamino-methyl)-biphenyl-2-carbonsäure-(3-methyl-butyl)-amid erhalten; Fp. 149°C. MS (ES+): m/z = 455 (M+1).

### Beispiel 3 n: 2'-(3-Fluorphenylsulfonylamino-rnethyl)-biphenyl-2-carbonsäure-(3-methyl-butyl)-amid

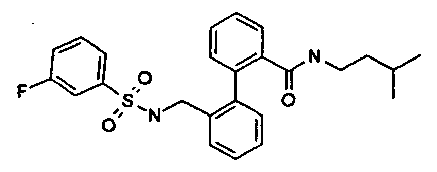

Aus 0,34 mmol 2'-Aminomethyl-biphenyl-2-carbonsäure-(3-methyl-butyl)-amid (Vorstufe 5 c) und 3-Fluorphenylsulfonylchlorid wurden gemäß der allgemeinen Arbeitsvorschrift 118 mg 2'-(3-Fluorphenylsulfonylamino-methyl)-biphenyl-2-carbonsäure-(3-methyl-butyl)-amid erhalten; Fp. 141°C. MS (ES+): m/z = 455 (M+1).

### Beispiel 3 o: 2'-(lsopropylsulfonylamino-methyl)-biphenyl-2-carbonsäure-(3-methylbutyl)-amid

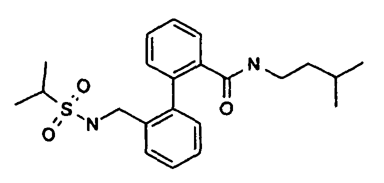

Aus 0,34 mmol 2'-Aminomethyl-biphenyl-2-carbonsäure-(3-methyl-butyl)-amid (Vorstufe 5 c) und Isopropylsulfonylchlorid wurden gemäß der allgemeinen Arbeitsvorschrift gefolgt von Reinigung durch Flashchromatograpie 16 mg 2'-(Isopropylsulfonylamino-methyl)-biphenyl-2-carbonsäure-(3-methyl-butyl)-amid als Öl erhalten. MS (ES+): m/z = 403 (M+1).

### Beispiel 3 p: 2'-(Phenylsulfonylamino-rnethyl)-biphenyl-2-carbonsäure-2-(2-pyridyl)-ethylamid

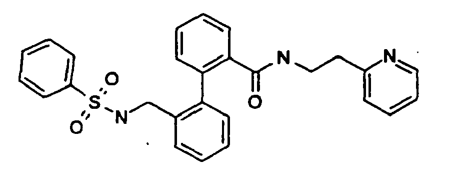

Aus 0,3 mmol 2`-Aminomethyl-biphenyl-2-carbonsäure-2-(2-pyridyl)-ethylamid (Vorstufe 5 d) und Phenylsulfonylchlorid wurden gemäß der allgemeinen Arbeitsvorschrift 117 mg 2'-(Phenylsulfonylamino-methyl)-biphenyl-2-carbonsäure-2-(2-pyridyl)-ethylamid erhalten; Fp. 131°C. MS (ES+): m/z = 472 (M+1).

### Beispiel 3 q: 2'-(4-Fluorphenylsulfonylamino-methyl)-biphenyl-2-carbonsäure-2-(2-pyridyl)-ethylamid

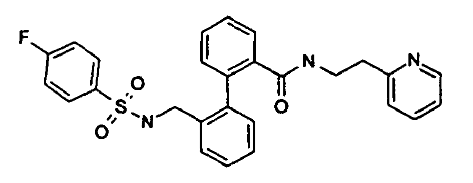

Aus 0,3 mmol 2'-Aminomethyl-biphenyl-2-carbonsäure-2-(2-pyridyl)-ethylamid (Vorstufe 5 d) und 4-Fluorphenylsulfonylchlorid wurden gemäß der allgemeinen Arbeitsvorschrift 106 mg 2'-(4-Fluorphenylsulfonylamino-methyl)-biphenyl-2-carbonsäure-2-(2-pyridyl)-ethylamid erhalten; Fp. 130°C. MS (ES+): m/z = 490 (M+1).

### Beispiel 3 r: 2'-(3-Fluorphenylsulfonylamino-methyl)-biphenyl-2-carbonsäure-2-(2-pyridyl)-ethylamid

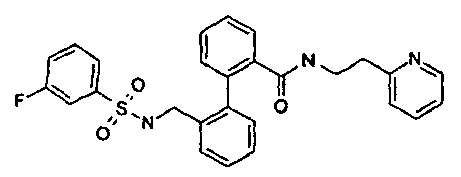

Aus 0,3 mmol 2'-Aminomethyl-biphenyl-2-carbonsäure-2-(2-pyridyl)-ethylamid (Vorstufe 5 d) und 3-Fluorphenylsulfonylchlorid wurden gemäß der allgemeinen Arbeitsvorschrift 102 mg 2'-(3-Fluorphenylsulfonylamino-methyl)-biphenyl-2-carbonsäure-2-(2-pyridyl)-ethylamid erhalten; Fp. 123°C. MS (ES+): m/z = 490 (M+1).

### Beispiel 3 s: 2'-(Isopropylsulfonylamino-methyl)-biphenyl-2-carbonsäure-2-(2-pyridyl)-ethylamid

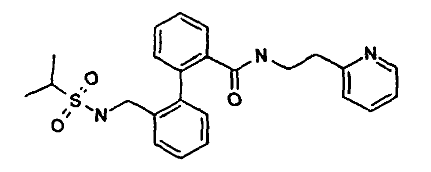

Aus 0,3 mmol 2'-Aminomethyl-biphenyl-2-carbonsäure-2-(2-pyridyl)-ethylamid (Vorstufe 5 d) und Isopropylsulfonylchlorid wurden gemäß der allgemeinen Arbeitsvorschrift und anschließender Extraktion mit EE 40 mg 2'-(Isopropylsulfonylamino-methyl)-biphenyl-2-carbonsäure-2-(2-pyridyl)-ethylamid als Öl erhalten. MS (ES+): m/z = 438 (M+1).

Analog Beispiel 3 a - 3 s wurde folgende Verbindung erhalten:

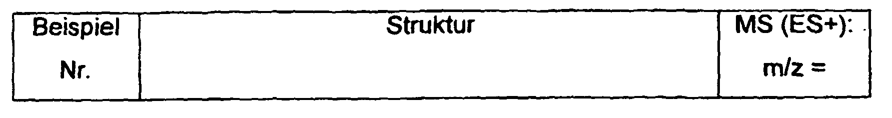

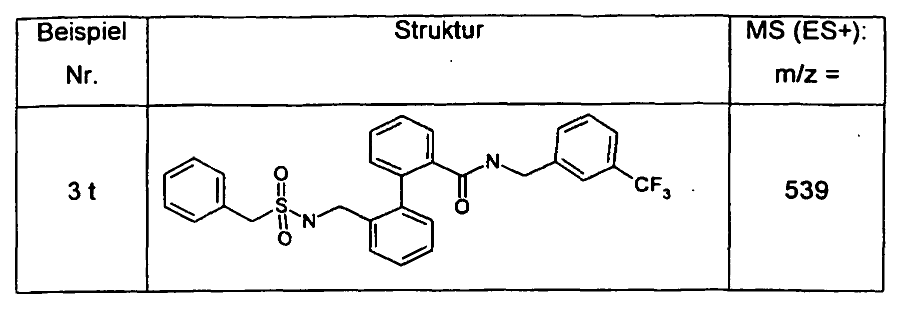

Allgemeine Vorschrift zur Umsetzung von Aminomethylbiphenylen mit Carbonsäurechloriden zu Carbonamiden (Beispiele 4 a bis 4 I):
Zu einer Lösung von 0,34 mmol des jeweiligen 2'-Aminomethylbiphenyls und 41 mg (0,41 mmol) Triethylamin in 5 ml Methylenchlorid werden 0,36 mmol des jeweiligen Sulfonsäurechlorids bei 0°C langsam zugetropft. Nach 3 h Rühren bei RT wird die Reaktionsmischung im Vakuum eingeengt, der Rückstand wird mit 25 ml Wasser verrührt und das ausgefallene Produkt wird abgesaugt oder durch Extraktion mit EE isoliert.

### Beispiel 4 a: 2'-(Benzoylamino-methyl)-biphenyl-2-carbonsäure-(3-methyl-butyl)-amid

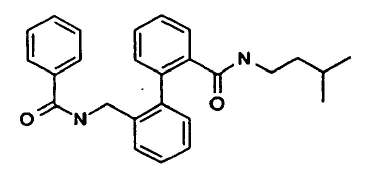

Aus 0,34 mmol 2'-Aminomethyl-biphenyl-2-carbonsäure-(3-methyl-butyl)-amid (Vorstufe 5 c) und Benzoylchlorid wurden gemäß der allgemeinen Arbeitsvorschrift 75 mg 2'-(Benzoylamino-methyl)-biphenyl-2-carbonsäure-(3-methyl-butyl)-amid erhalten; Fp. 147°C. MS (ES+): m/z = 401 (M+1).

### Beispiel 4 b: 2'-(Benzoylamino-methyl)-biphenyl-2-carbonsäure-2-(2-pyridyl)-ethylamid

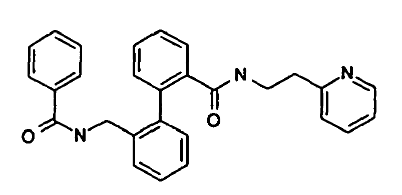

Aus 0,3 mmol 2'-Aminomethyl-biphenyl-2-carbonsäure-2-(2-pyridyl)-ethylamid (Vorstufe 5 d) und Benzoylchlorid wurden gemäß der allgemeinen Arbeitsvorschrift 98 mg 2'-(Benzoylamino-methyl)-biphenyl-2-carbonsäure-2-(2-pyridyl)-ethylamid erhalten; Fp. 135°C. MS (ES+): m/z = 436 (M+1).

### Beispiel 4 c: 2'-{[2-(4-Methoxy-phenyl)-acetylamino]-methyl}-biphenyl-2-carbonsäure-2,4-difluor-benzylamid

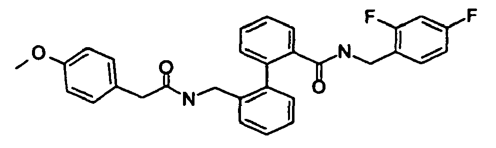

Aus 0,5 mmol 2'-Aminomethyl-biphenyl-2-carbonsäure-(2,4-difluorbenzyl)-amid (Vorstufe 8 a) und 4-Methoxyphenylacetylchlorid wurden gemäß der allgemeinen Arbeitsvorschrift 160 mg 2'-{[2-(4-Methoxy-phenyl)-acetylamino)-methyl}-biphenyl-2-carbonsäure-2,4-difluor-benzylamid erhalten; Fp. 138°C. MS (ES+): m/z = 501 (M+1).

Analog Beispiel 4 a - 4 c wurden folgende Verbindungen erhalten:

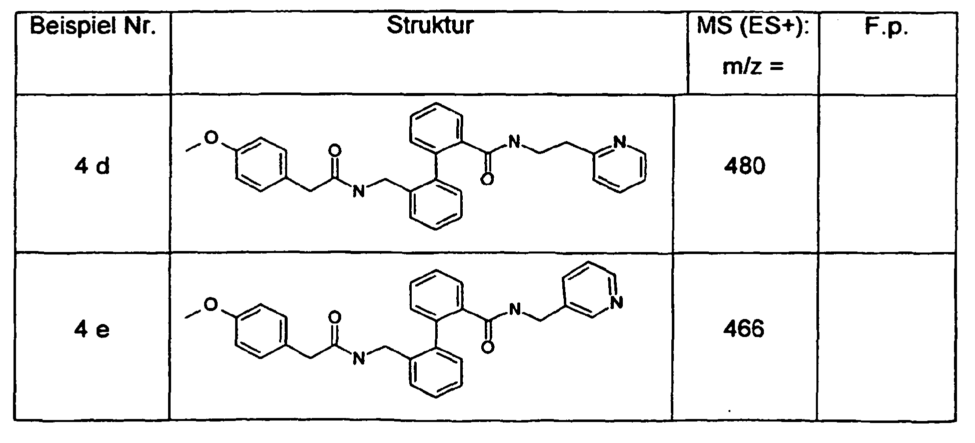

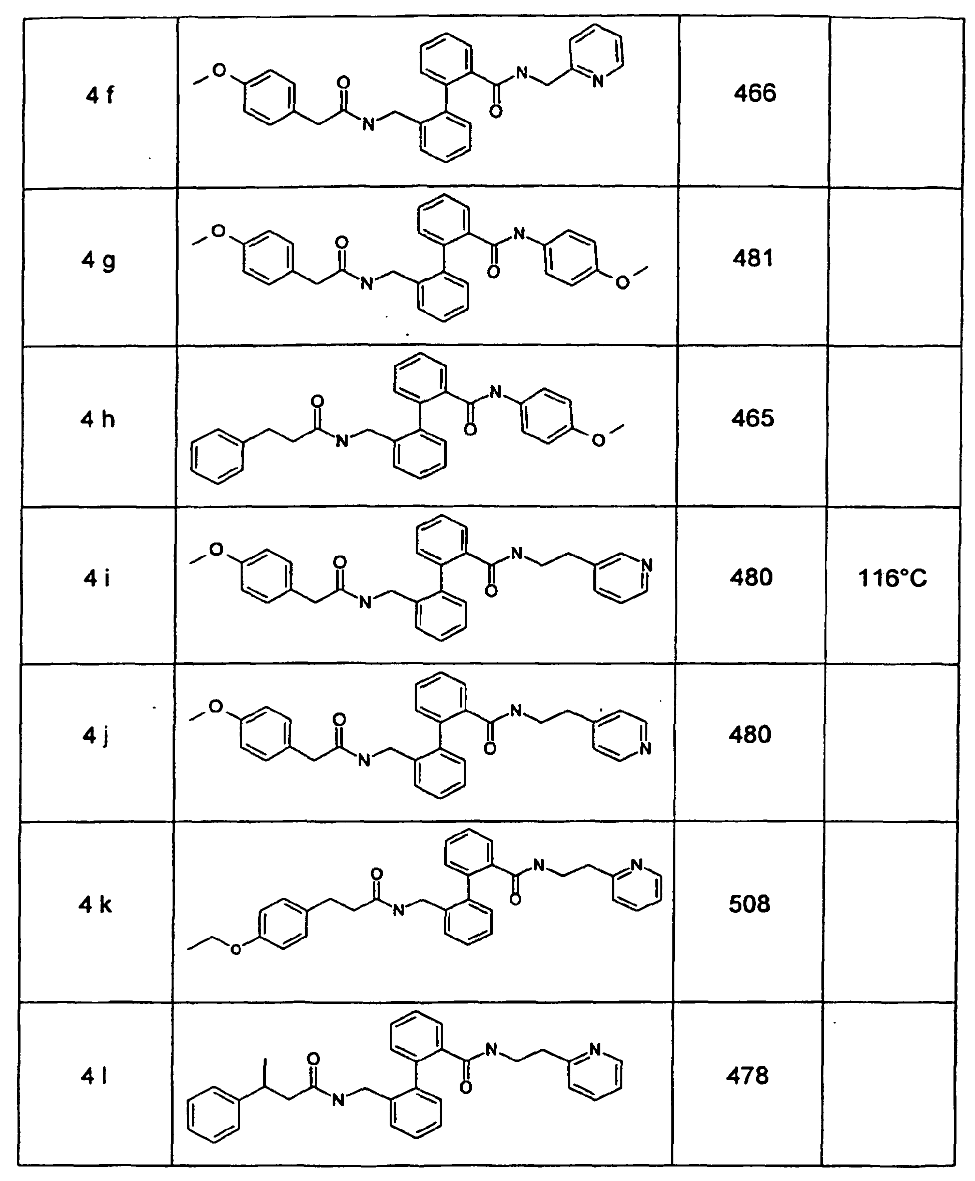

Allgemeine Vorschrift zur Umsetzung von Aminomethylbiphenylen mit Isocyanaten zu Harnstoffen (Beispiel 5 a - 5 e):
Zu einer Lösung von 0,34 mmol des jeweiligen 2'-Aminomethylbiphenyls und 41 mg (0,41 mmol) Triethylamin in 5 ml Methylenchlorid werden 0,36 mmol des jeweiligen Isocyanates gelöst in 0,5 ml Methylenchlorid bei 0°C langsam zugetropft. Nach 3 h Rühren bei RT wird die Reaktionsmischung im Vakuum eingeengt, der Rückstand wird mit 25 ml Wasser verrührt und das ausgefallene Produkt wird abgesaugt oder durch Extraktion mit EE isoliert.

### Beispiel 5 a: 2'-[(3-Phenyl-ureido)-methyl]-biphenyl-2-carbonsäure-(3-methyl-butyl)-amid

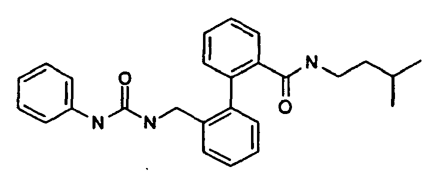

Aus 0,34 mmol 2'-Aminomethyl-biphenyl-2-carbonsäure-(3-methyl-butyl)-amid (Vorstufe 5 c) und Phenylisocyanat wurden gemäß der allgemeinen Arbeitsvorschrift 85 mg 2'-[(3-Phenyl-ureido)-methyl]-biphenyl-2-carbonsäure-(3-methyl-butyl)-amid
erhalten; Fp. 194°C. MS (ES+): m/z = 416 (M+1).

### Beispiel 5 b: 2'-[(3-Phenyl-ureido)-methyl]-biphenyl-2-carbonsäure-2-(2-pyridyl)-ethylamid

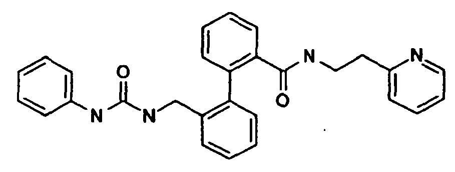

Aus 0,3 mmol 2'-Aminomethyl-biphenyl-2-carbonsäure-2-(2-pyridyl)-ethylamid (Vorstufe 5 d) und Phenylisocyanat wurden gemäß der allgemeinen Arbeitsvorschrift 101 mg 2'-[(3-Phenyl-ureido)-methyl]-biphenyl-2-carbonsäure-2-(2-pyridyl)-ethylamid erhalten; Fp. 99°C. MS (ES+): m/z = 451 (M+1).

### Beispiele 5 c - 5 e

Analog wurden folgende Verbindungen aus 2'-Aminomethyl-biphenyl-2-carbonsäure-2-(2-pyridyl)-ethylamid (Vorstufe 5 d) und den entsprechenden Isocyanaten erhalten:

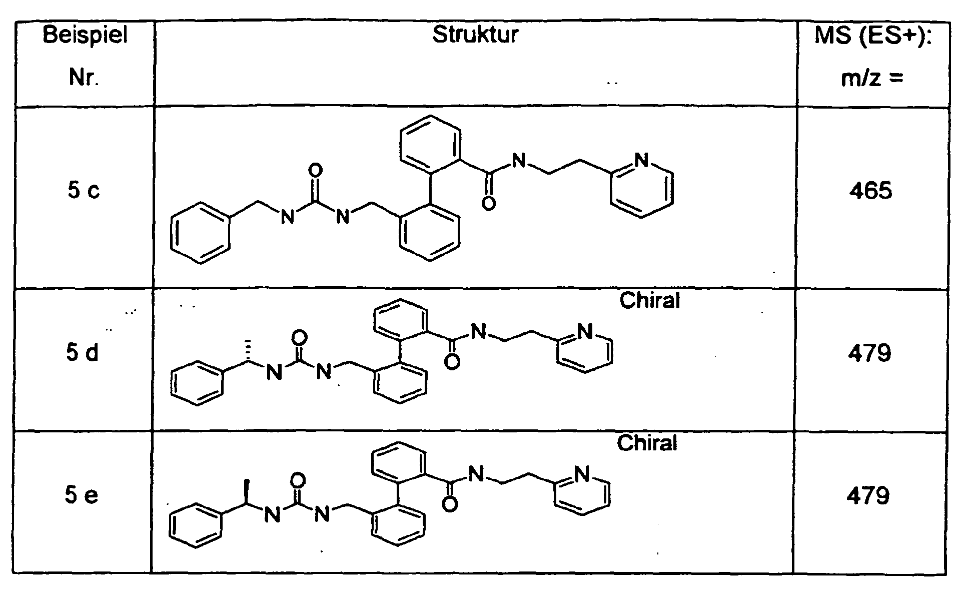

Allgemeine Vorschrift zur Umsetzung von Biphenylcarbonsäuren mit Aminen zu Amiden (Beispiele 6 a - 6 h):
Zu einer Lösung von 0,28 mmol der entsprechenden Biphenylcarbonsäure, 0,3 mmol HOBT und 0,3 mmol DIC in 5 ml THF werden bei 0°C 0,3 mmol des jeweiligen Amins zugetropft und es wird 12 bei RT gerührt. Die Reaktionsmischung wird mit EE verdünnt und mit verdünnter Salzsäure und Natriumbicarbonatlösung gewaschen. Nach Trocknen über Magnesiumsulfat und Einengen im Vakuum erhält man das entsprechende Amid.

### Beispiel 6 a: 2'-(Benzyloxycarbonylamino-methyl)-biphenyl-2-carbonsäure-benzylmethylamid

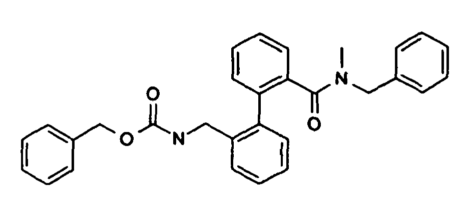

Aus 0,28 mmol 2'-(Benzyloxycarbonylamino-methyl)-biphenyl-2-carbonsäure (Vorstufe 6) und Benzyl-methylamin wurden gemäß der allgemeinen Arbeitsvorschrift 89 mg 2'-(Benzyloxycarbonylamino-methyl)-biphenyl-2-carbonsäure-benzyl-methylamid erhalten. MS (ES+): m/z = 465 (M+1).

### Beispiel 6 b: 2'-(Benzyloxycarbonylamino-methyl)-biphenyl-2-carbonsäurecyclohexylamid

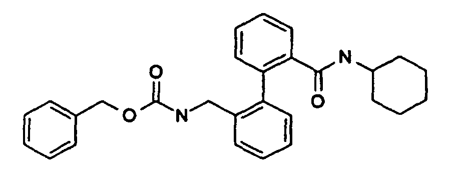

Aus 0,28 mmol 2'-(Benzyloxycarbonylamino-methyl)-biphenyl-2-carbonsäure (Vorstufe 6) und Cyclohexylamin wurden gemäß der allgemeinen Arbeitsvorschrift 99 mg 2'-(Benzyloxycarbonylamino-methyl)-biphenyl-2-carbonsäure-cyclohexylamid erhalten. MS (ES+): m/z = 443 (M+1).

### Beispiel 6 c: 2'-(Benzyloxycarbonylamino-methyl)-biphenyl-2-carbonsäurephenylamid

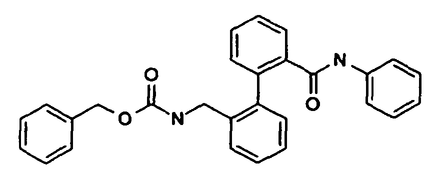

Aus 0,28 mmol 2'-(Benzyloxycarbonylamino-methyl)-biphenyl-2-carbonsäure (Vorstufe 6) und Anilin wurden gemäß der allgemeinen Arbeitsvorschrift 66 mg 2'-(Benzyloxycarbonylamino-methyl)-biphenyl-2-carbonsäure-phenylamid erhalten. MS (ES+): m/z = 437 (M+1).

### Beispiel 6 d: 2'-(Benzyloxycarbonylamino-methyl)-biphenyl-2-carbonsäure-{N-methyl-N-[2-(2-pyridyl)-ethyl]}-amid

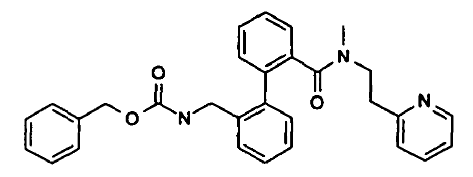

Aus 0,28 mmol 2'-(Benzyloxycarbonylamino-methyl)-biphenyl-2-carbonsäure (Vorstufe 6) und 2-[2-(Methytammoethyl)]pyridin wurden gemäß der allgemeinen Arbeitsvorschrift und anschließender Reinigung durch Flash-Chromatograpie 54 mg 2'-(Benzyloxycarbonylamino-methyl)-biphenyl-2-carbonsäure-{N-methyl-N-[2-(2-pyridyl)-ethyl]}-amid erhalten.
MS (ES+): m/z = 480 (M+1).

### Beispiel 6 e: 2'-(Benzyloxycarbonylamino-methyl)-biphenyl-2-carbonsäuredibutylamid

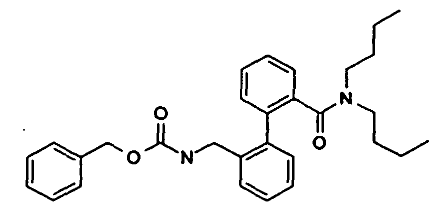

Aus 0,28 mmol 2'-(Benzyloxycarbonylamino-methyl)-biphenyl-2-carbonsäure (Vorstufe 6) und Dibutylamin wurden gemäß der allgemeinen Arbeitsvorschrift und anschließender Reinigung durch Flash-Chromatograpie 82 mg 2'-(Benzyloxycarbonylamino-methyl)-biphenyl-2-carbonsäure-dibutylamid erhalten. MS (ES+): m/z = 473 (M+1).

### Beispiel 6 f: 2'-(Benzyloxycarbonylamino-methyl)-biphenyl-2-carbonsäure-2-(2-pyridyl)-ethylamid

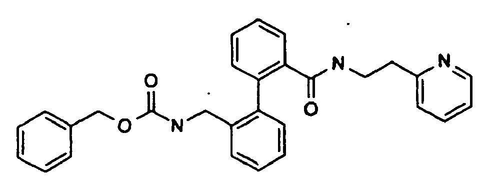

Aus 0,28 mmol 2'-(Benzyloxycarbonylamino-methyl)-biphenyl-2-carbonsäure (Vorstufe 6) und 2-(2-Pyridyl)-ethylamin wurden gemäß der allgemeinen Arbeitsvorschrift und anschließender Reinigung durch Flash-Chromatograpie 85 mg 2'-(Benzyloxycarbonylamino-methyl)-biphenyl-2-carbonsäure-2-(2-pyridyl)-ethylamid erhalten. Fp. 140°C (Heptan/EE); MS (ES+): m/z = 466 (M+1).

### Beispiel 6 g: 2'-(Benzyloxycarbonylamino-methyl)-biphenyl-2-carbonsäure-(2,4-difluorbenzyl)-amid

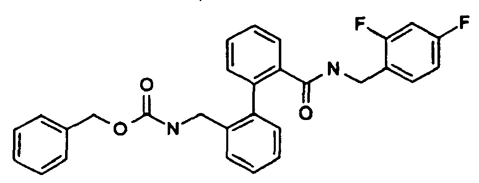

Aus 0,28 mmol 2'-(Benzyloxycarbonylamino-methyl)-biphenyl-2-carbonsäure (Vorstufe 6) und 2,4-Difluorbenzylamin wurden gemäß der allgemeinen Arbeitsvorschrift und anschließender Reinigung durch Flash-Chromatograpie 99 mg 2'-(Benzyloxycarbonylamino-methyl)-biphenyl-2-carbonsäure-(2,4-difluorbenzyl)-amid erhalten. MS (ES+): m/z = 487 (M+1).

### Beispiel 6 h: 2'-(Benzyloxycarbonylamino-methyl)-biphenyl-2-carbonsäure-(2,2,2-trifluorethyl)-amid

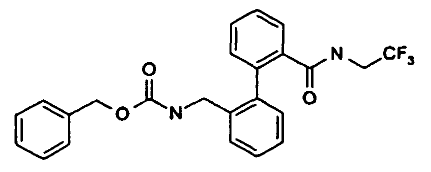

Aus 0,28 mmol 2'-(Benzyloxycarbonylamino-methyl)-biphenyl-2-carbonsäure (Vorstufe 6) und 2,2,2-Trifluorethylamin wurden gemäß der allgemeinen Arbeitsvorschrift und anschließender Reinigung durch Flash-Chromatograpie 19 mg 2'-(Benzyloxycarbonylamino-methyl)-biphenyl-2-carbonsäure-(2,2,2-trifluorethyl)-amid erhalten. MS (ES+): m/z = 443 (M+1).

### Beispiel 7 a: 2'-(Benzyloxycarbonylamino-methyl)-biphenyl-2-carbonsäure-2-[2-(1-oxypyridyl)]-ethylamid

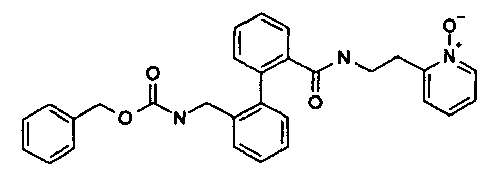

Zu einer Lösung von 85 mg (0,18 mmol) 2'-(Benzyloxycarbonylaminomethyl)-biphenyl-2-carbonsäure-2-(2-pyridyl)-ethylamid (Beispiel 6f) in 13 ml Methylenchlorid wurden bei 0°C 47 mg m-Chlorperbenzoesäure gelöst in 2 ml Methylenchlorid zugetropft und die Reaktionsmischung wurde 12 h bei RT gerührt. Die organische Phase wurde 2mal mit Natriumbicarbonatlösung gewaschen, über Magnesiumsulfat getrocknet und im Vakuum eingeengt. Man erhielt 79 mg 2'-(Benzyloxycarbonylamino-methyl)-biphenyl-2-carbonsäure-2-[2-(1-oxypyridyl)]-ethylamid. MS (ES+): m/z = 482 (M+1).

Analog Beispiel 7a wurden folgende Verbindungen aus den entsprechenden Pyridinen erhalten:

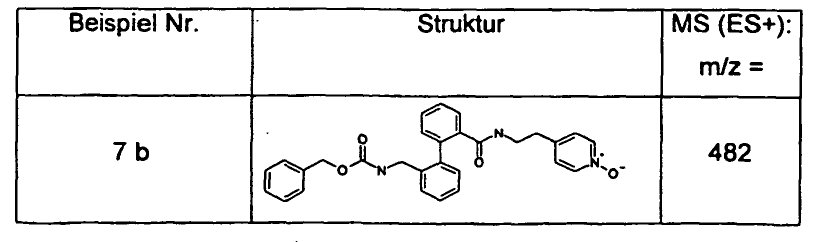

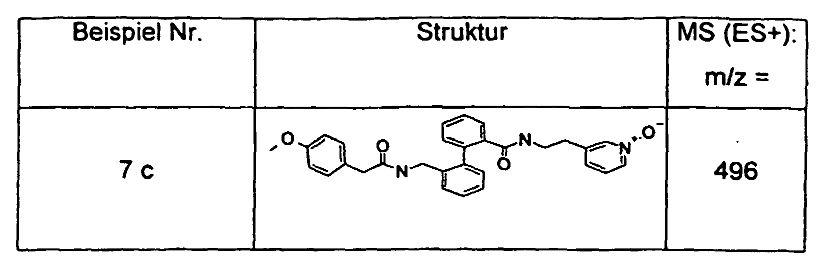

Allgemeine Vorschrift zur Umsetzung von Biphenylcarbonsäuren mit Aminen zu Amiden (Beispiele 8 a - 8 ac):
Zu einer Lösung von 0,42 mmol der entsprechenden Biphenylcarbonsäure, 0,44 mmol HOBT und 0,44 mmol EDAC in 5 ml THF werden bei 0°C 0,44 mmol des jeweiligen Amins zugetropft und es wird 4 bis 12 h bei RT gerührt. Die Reaktionsmischung wird mit EE verdünnt und mit verdünnter Salzsäure und Natriumbicarbonatlösung gewaschen. Nach Trocknen über Magnesiumsulfat und Einengen im Vakuum erhält man das entsprechende Amid.

### Beispiel 8 a: [2'-(1-Carbamoyl-3-methyl-butylcarbamoyl)-biphenyl-2-ylmethyl]-carbaminsäurebenzylester

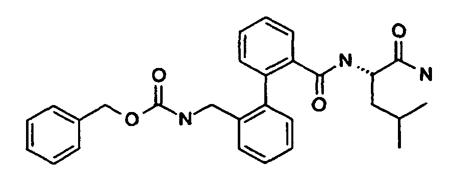

Aus 0,28 mmol 2'-(Benzyloxycarbonylamino-methyl)-biphenyl-2-carbonsäure (Vorstufe 6) und L-Leucinamidhydrochlorid / Triethylamin wurden gemäß der allgemeinen Arbeitsvorschrift 180 mg [2'-(1-Carbamoyl-3-methyl-butylcarbamoyl)-biphenyl-2-ylmethyl]-carbaminsäurebenzylester erhalten. MS (ES+): m/z = 474 (M+1).

### Beispiel 8 b: 2-{[2'-(Benzyloxycarbonylamino-methyl)-biphenyl-2-carbonyl]-amino}-3-phenyl-propionsäuremethylester

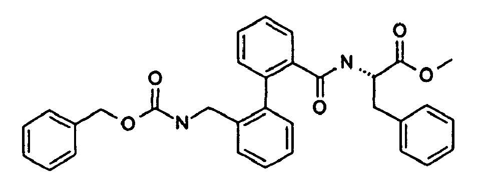

Aus 0,28 mmol 2'-(Benzyloxycarbonylamino-methyl)-biphenyl-2-carbonsäure (Vorstufe 6) und L-Phenylalaninmethylesterhydrochlorid /Triethylamin wurden gemäß der allgemeinen Arbeitsvorschrift 230 mg 2-{[2'-(Benzyloxycarbonylamino-methyl)-biphenyl-2-carbonyl]-amino}-3-phenyl-propionsäuremethylester erhalten. MS (ES+): m/z = 523 (M+1).

### Beispiel 8 c: 2'-(tert.-Butoxycarbonylamino-methyl)-biphenyl-2-carbonsäure-(2,4-difluorbenzyl)-amid

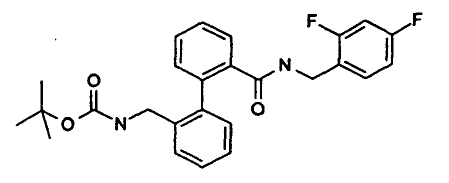

Aus 10 mmol 2'-(tert.-Butoxycarbonylaminomethyl)-biphenyl-2-carbonsäure (Vorstufe 7) und 2,4-Difluorbenzylamin wurden gemäß der allgemeinen Arbeitsvorschrift 3,8 g 2'-(tert.-Butoxycarbonylamino-methyl)-biphenyl-2-carbonsäure-(2,4-difluorbenzyl)-amid erhalten. MS (ES+): m/z = 453 (M+1).

### Beispiele 8 d - 8 p:

Aus 2'-(tert.-Butoxycarbonylaminomethyl)-biphenyl-2-carbonsäure (Vorstufe 7) und den entsprechenden Aminen wurden analog Beispiel 8a - 8c folgende Produkte erhalten:

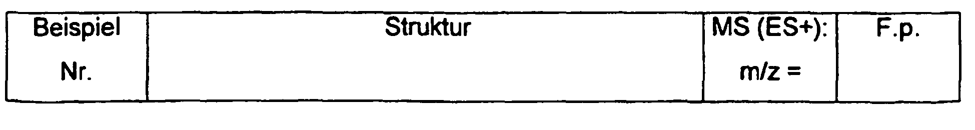

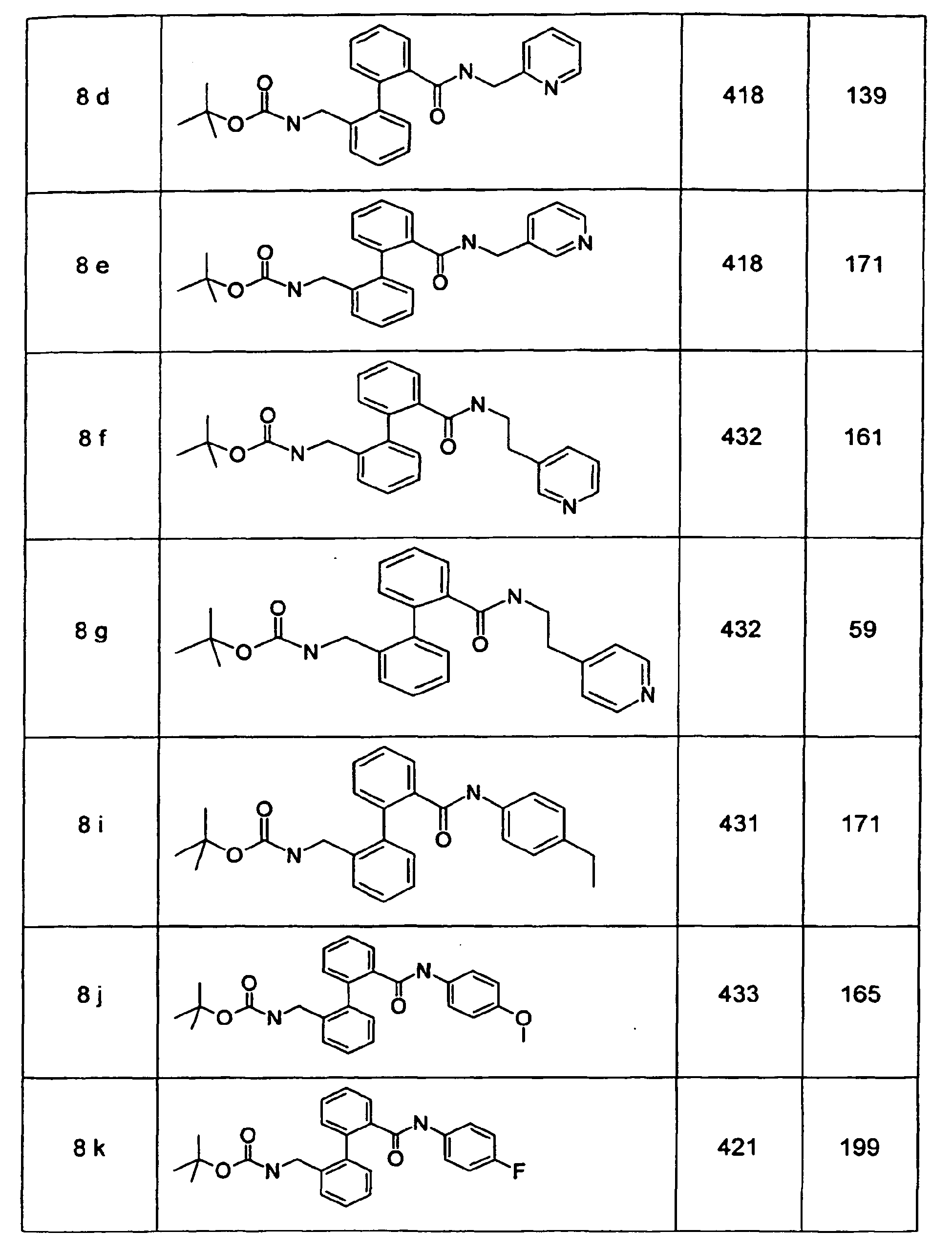

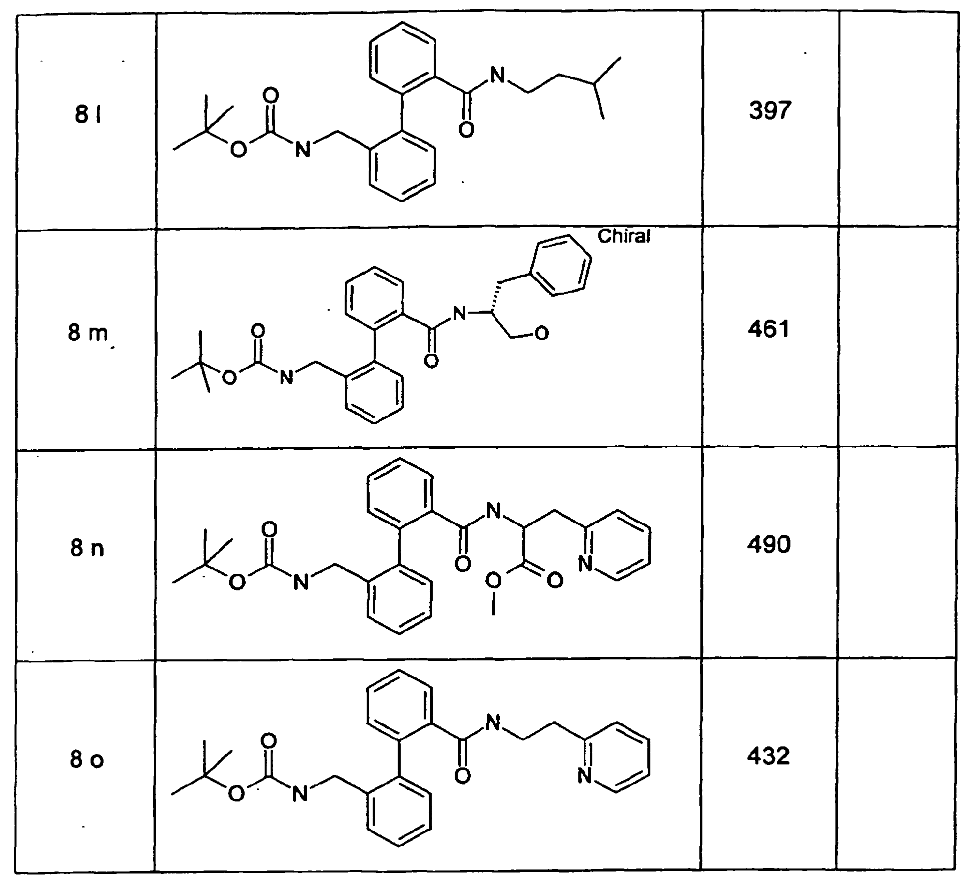

### Beispiele 8 p - 8 ac

Aus 2'-(Benzyloxycarbonylamino-methyl)-biphenyl-2-carbonsäure (Vorstufe 6) und den entsprechenden Aminen wurden analog Beispiel 8a - 8 c folgende Produkte erhalten:

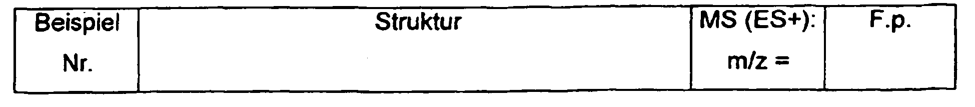

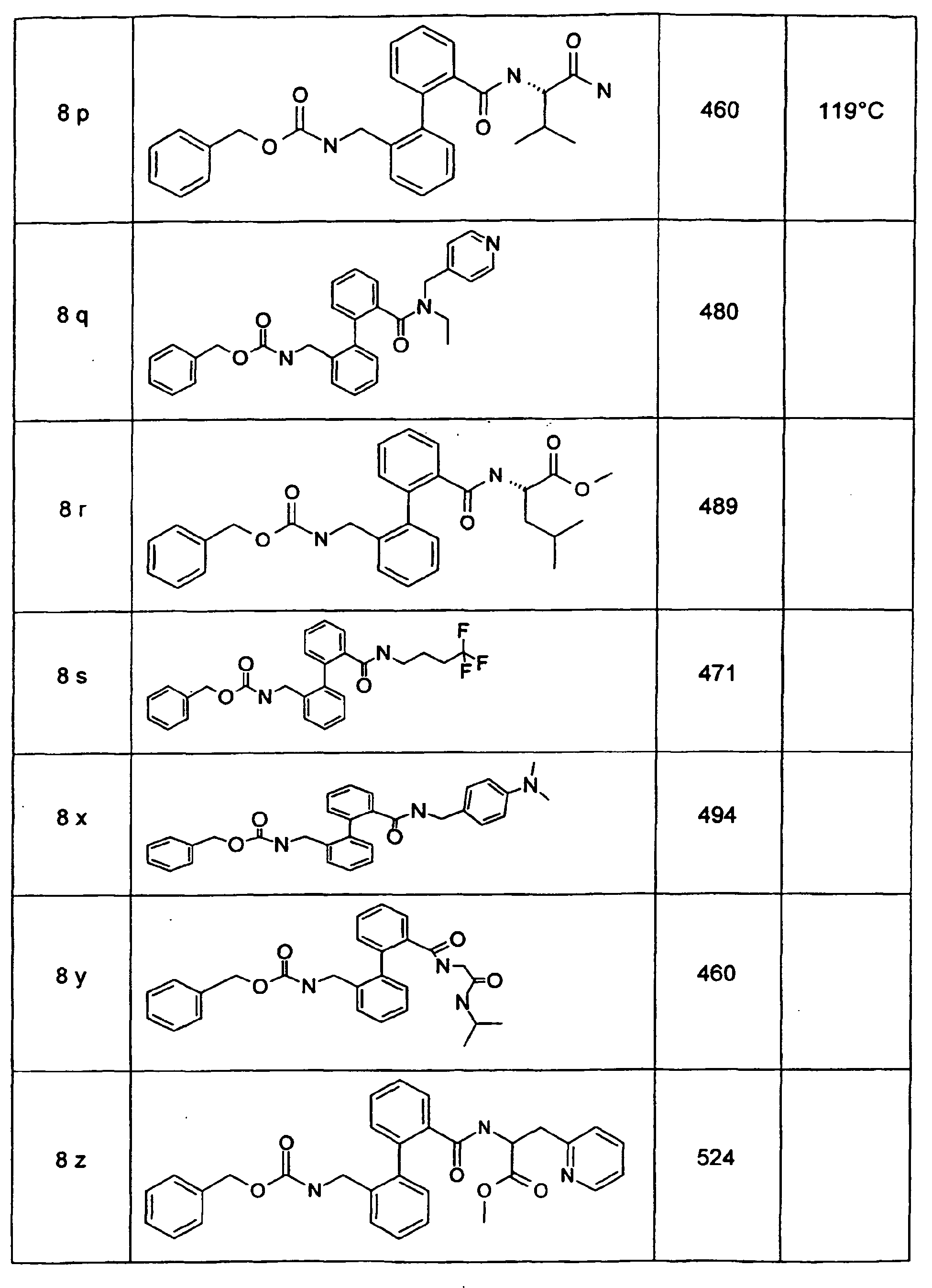

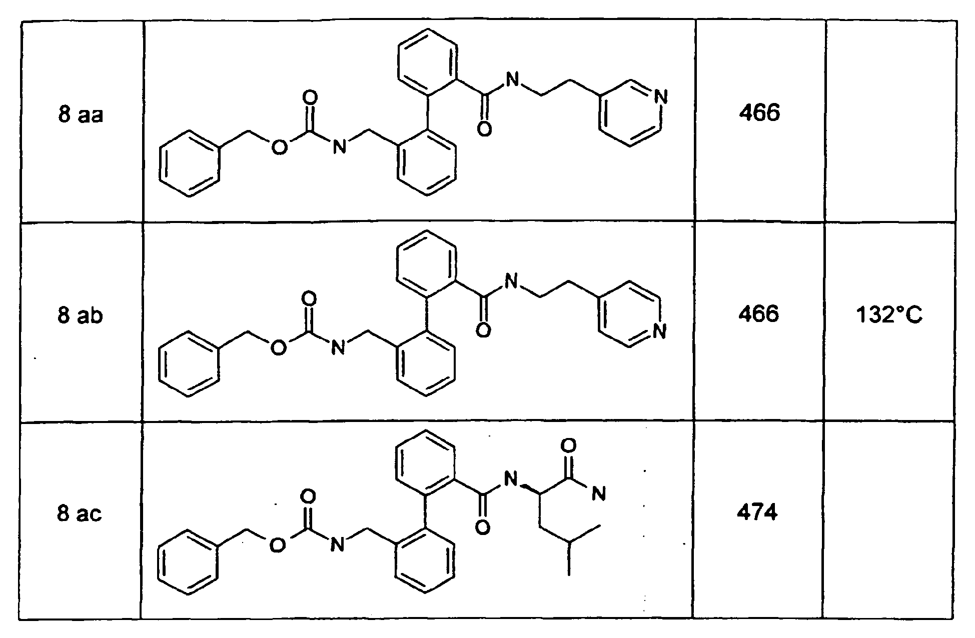

Allgemeine Vorschrift zur Umsetzung von Aminomethylbiphenylen mit Isothiocyanaten zu Thioharnstoffen (Beispiele 9 a - 9 i):
Zu einer Lösung von 0,34 mmol des jeweiligen 2'-Aminomethylbiphenyls und 41 mg (0,41 mmol) Triethylamin in 5 ml Methylenchlorid werden 0,36 mmol des jeweiligen Isothiocyanates gelöst in 0,5 ml Methylenchlorid bei 0°C langsam zugetropft. Nach 3 h Rühren bei RT wird die Reaktionsmischung im Vakuum eingeengt, der Rückstand wird mit 25 ml Wasser verrührt und das ausgefallene Produkt wird abgesaugt oder durch präparative HPLC gereinigt.

Auf diese Weise wurden u. a. folgende Produkte erhalten:

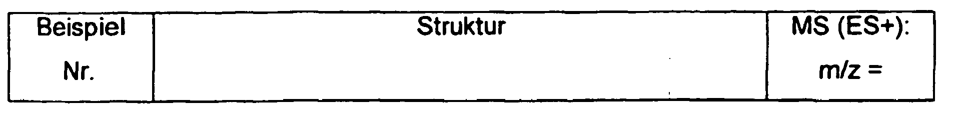

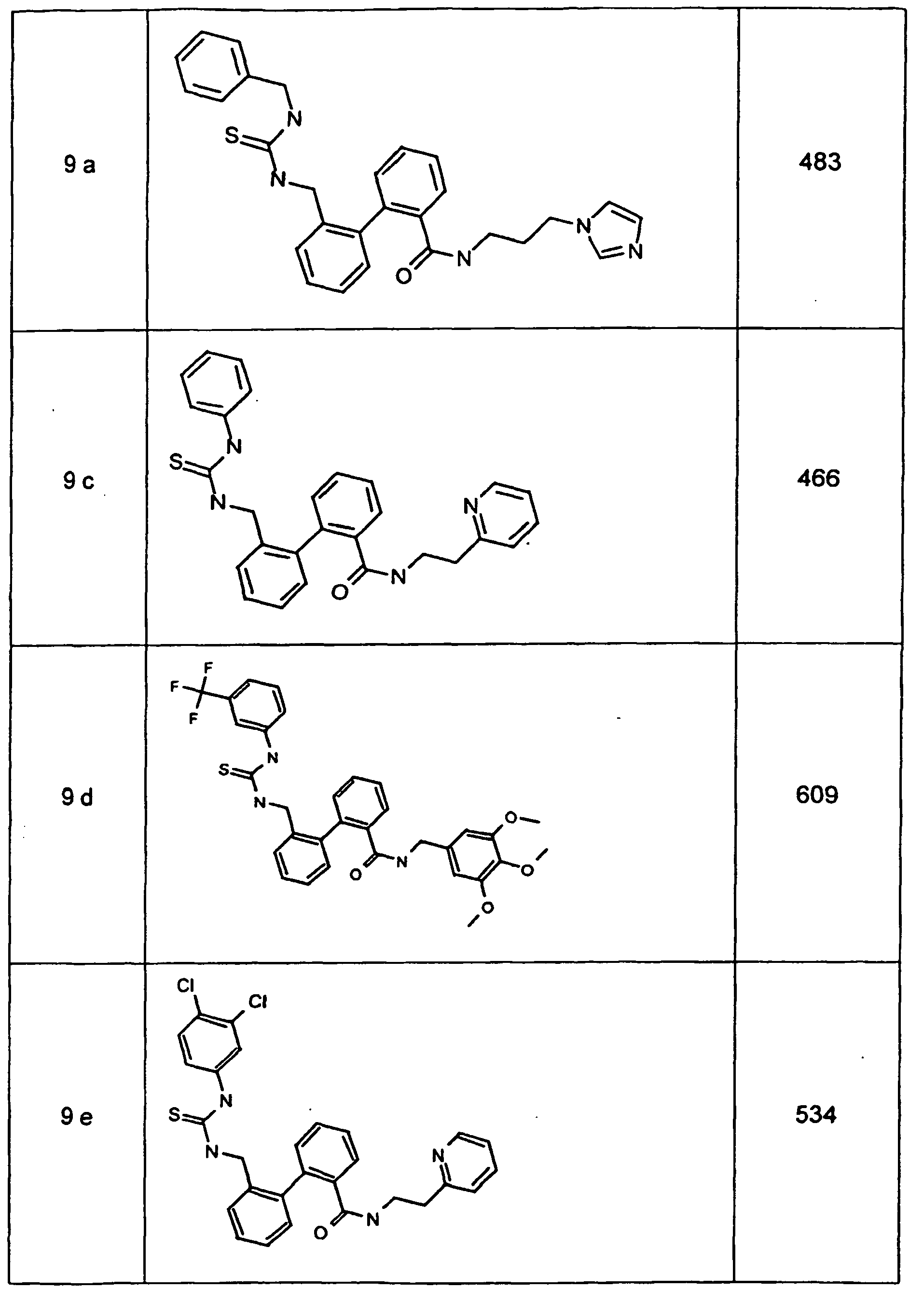

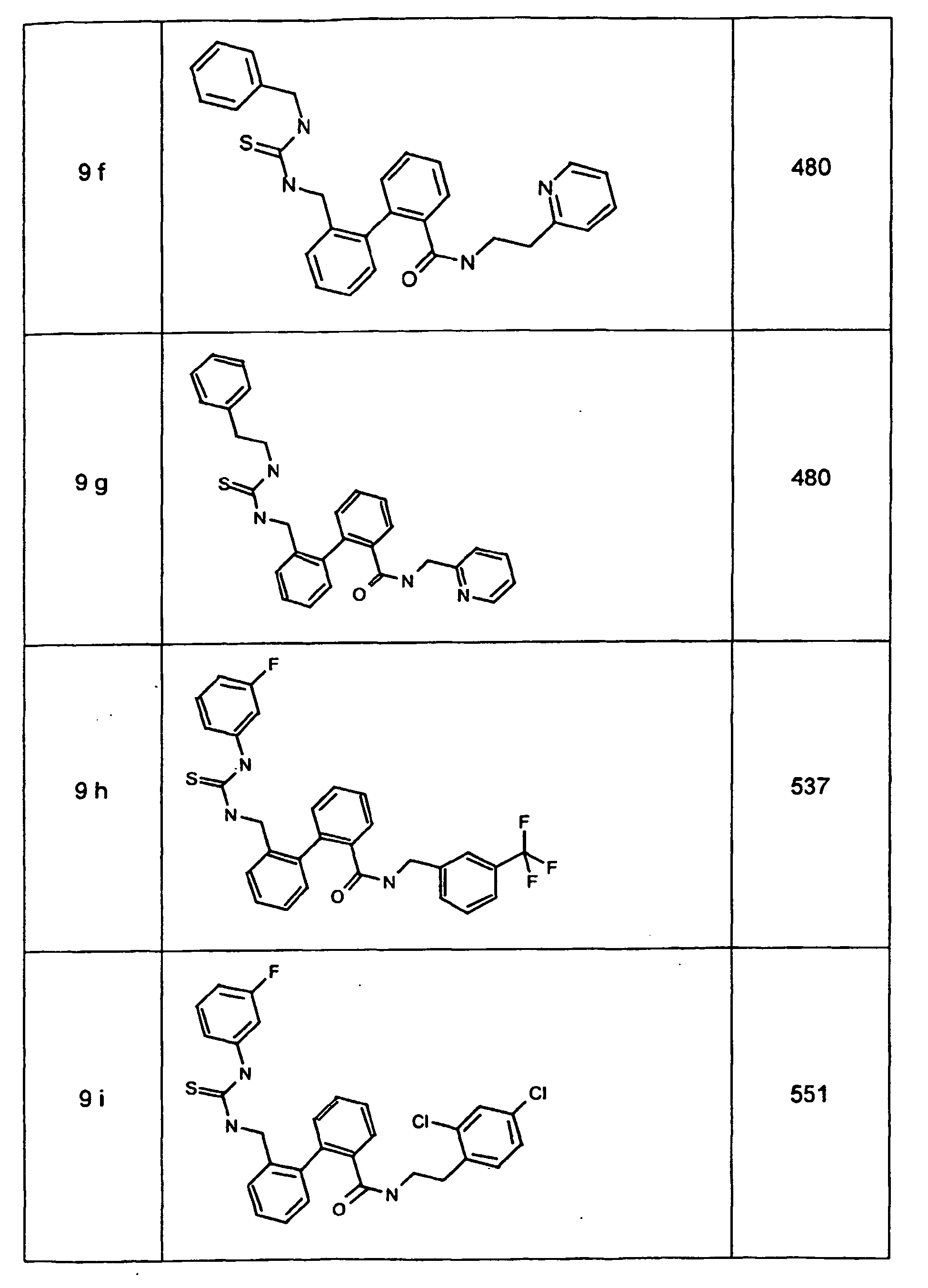

### Beispiele 10 a - 10 o

Durch Kopplung von 2'-(tert.-Butyloxycarbonyl-aminomethyl)-biphenyl-2-carbonsäure (Vorstufe 7) mit entsprechenden Aminen analog den für Beispiel 6 oder 8 beschriebenen Methoden wurden u. a. folgende Produkte erhalten:

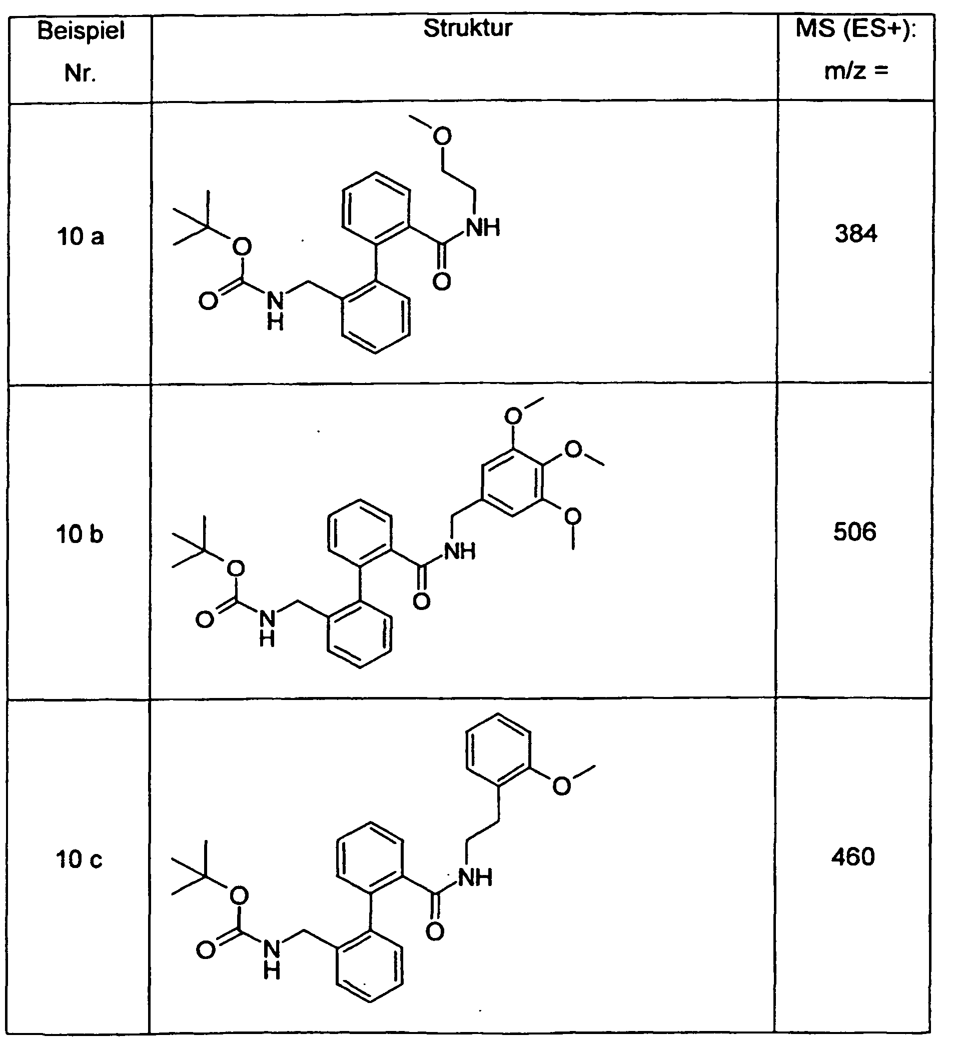

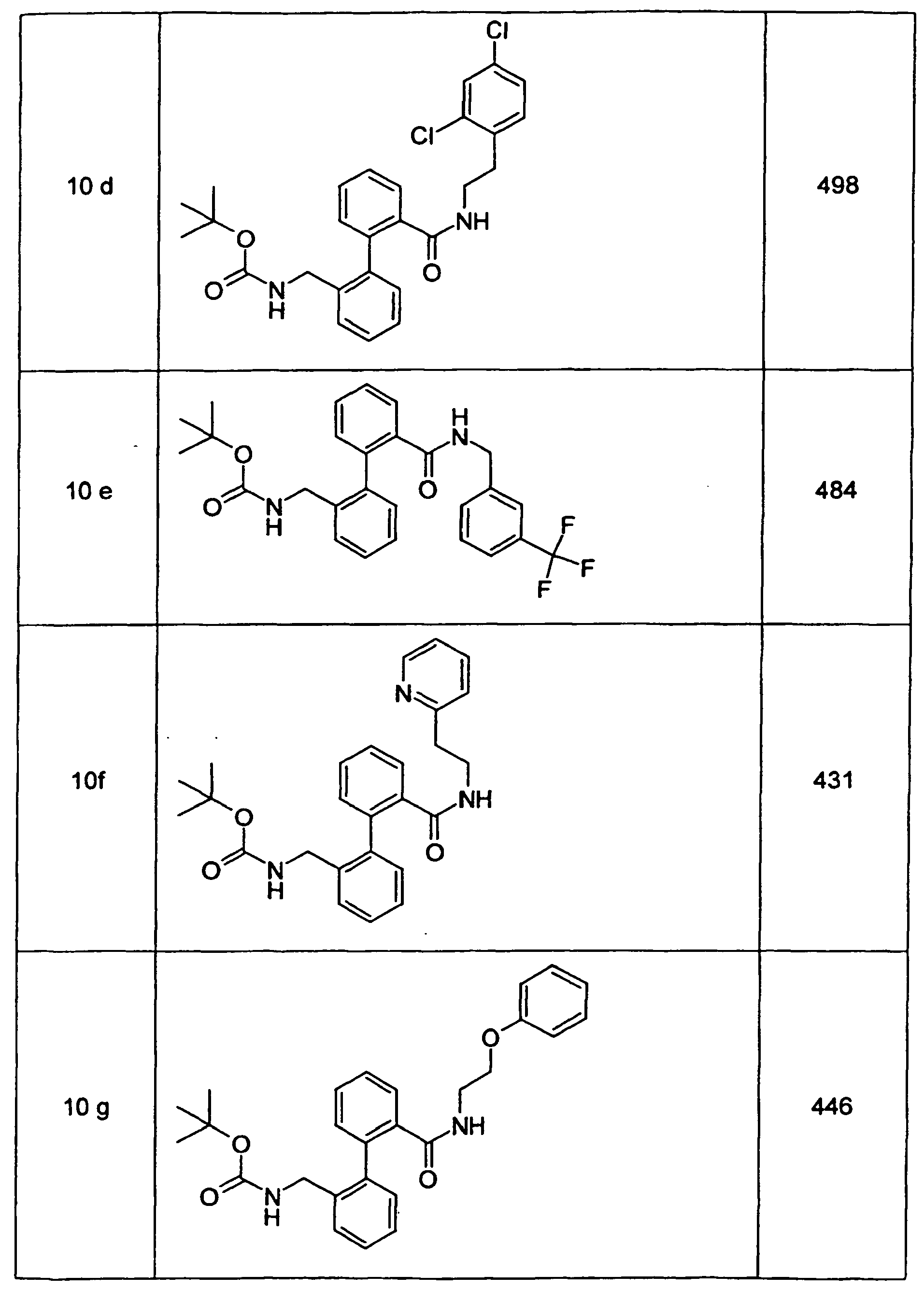

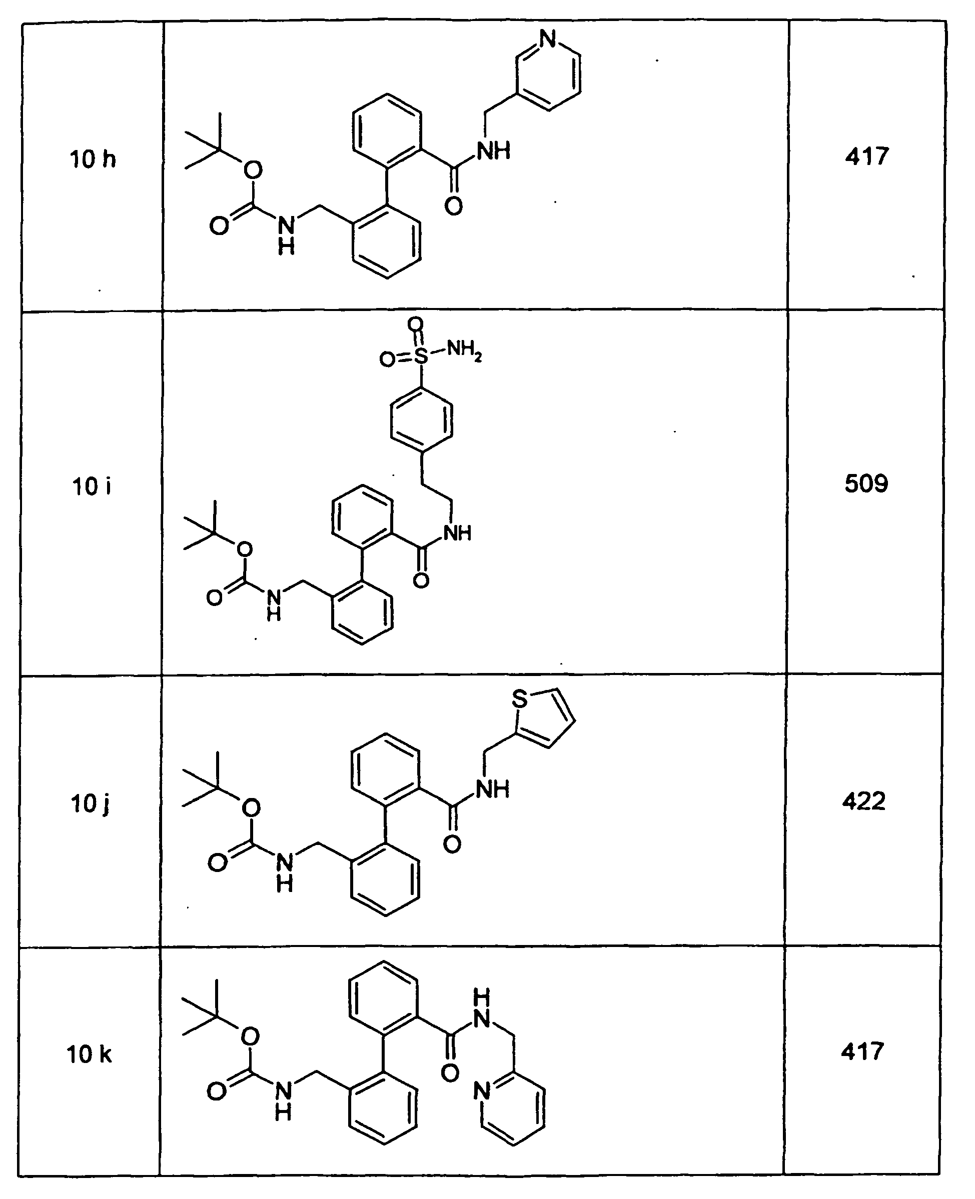

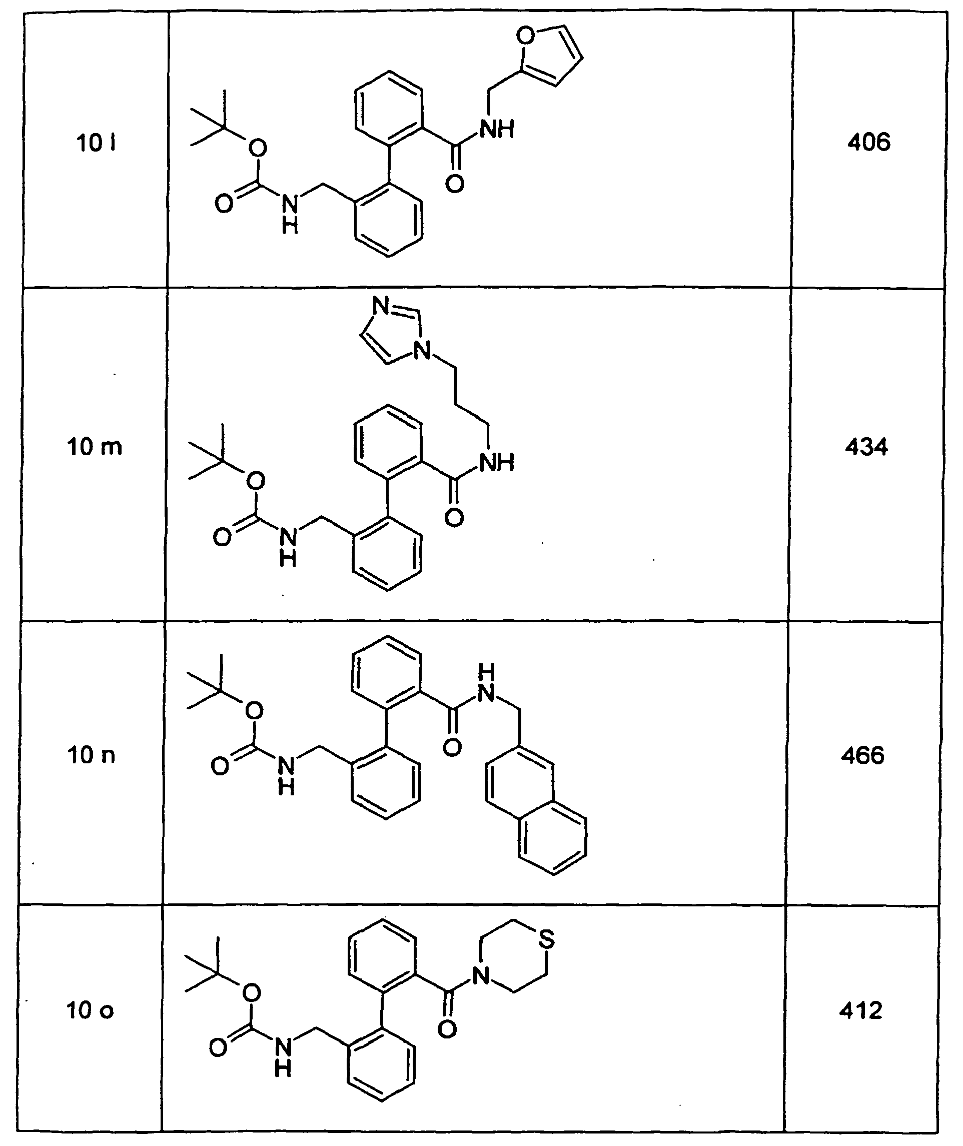

### Beispiel 11 a - 11 r:

Allgemeine Vorschrift zur Umwandlung der Boc-Derivate der Beispiele 10 zu Harnstoffen:
Zur Abspaltung der Boc-Schutzgruppe wurden 1 g der entsprechenden Verbindung des Beispiels 10 zu 10 ml einer 30- prozentigen Lösung von TFA in Dichlormethan gegeben. Das Gemisch wurde 30 min bei RT gerührt und das Lösungsmittel am Rotationsverdampfer im Vakuum entfernt. Der Rückstand wurde in Essigester aufgenommen und mit gesättigter Natriumhydrogencarbonatlösung gewaschen. Die organische Phase wurde über Magnesiumsulfat getrocknet, filtriert und das Lösungsmittel im Vakuum entfernt. Die erhaltenen 2'-Aminomethyl-biphenyl-2-carbonsäureamide wurden anschließend gemäß der Vorschrift für die Beispiele 5 mit Isocyanaten zu den entsprechenden Harnstoffen umgesetzt.

Auf diese Weise wurden u. a. folgende Produkte erhalten:

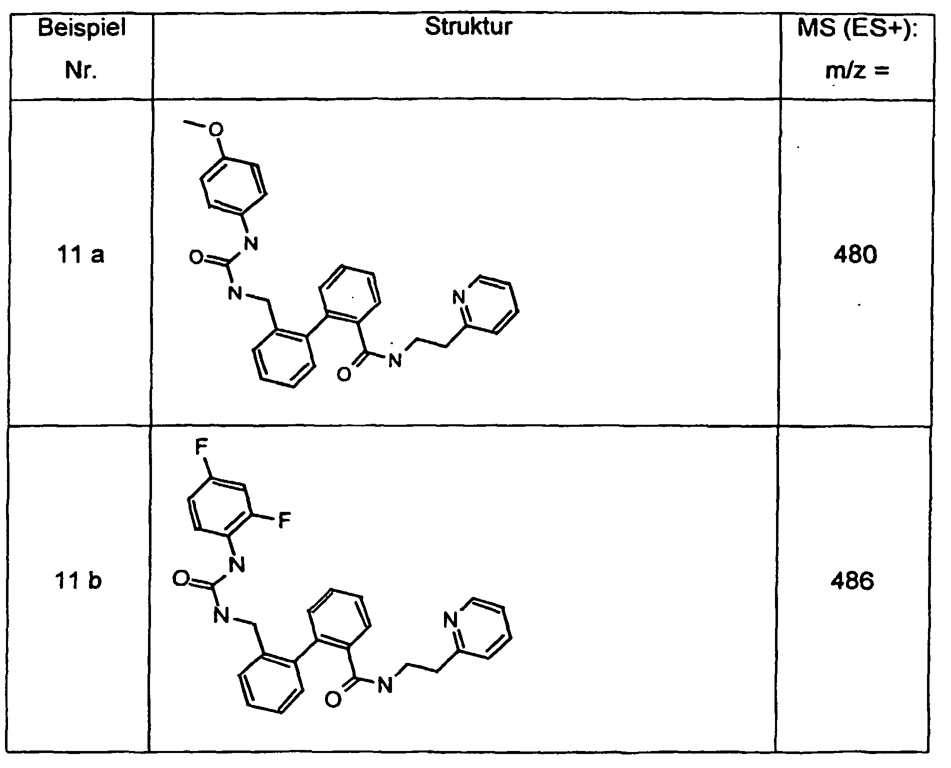

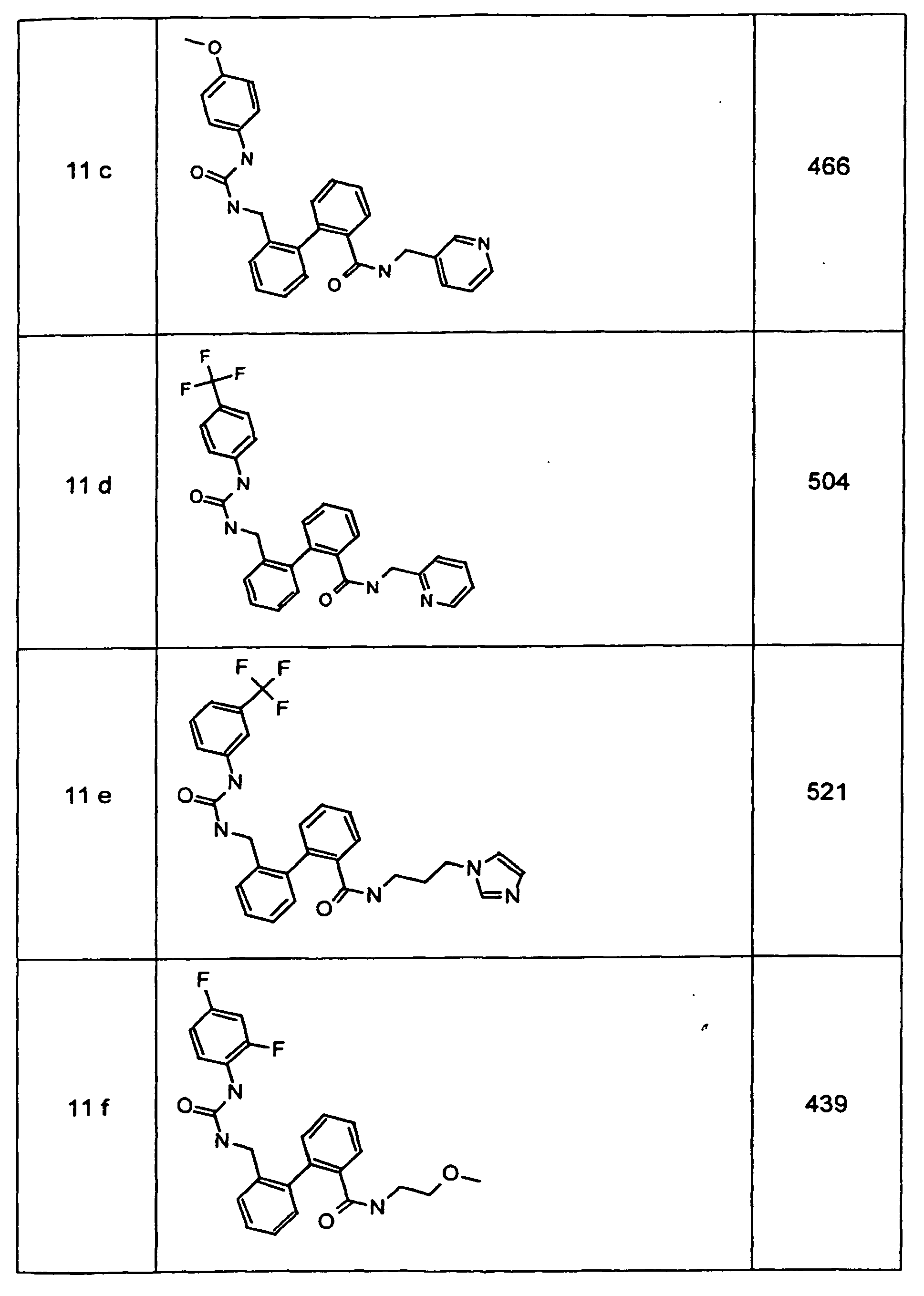

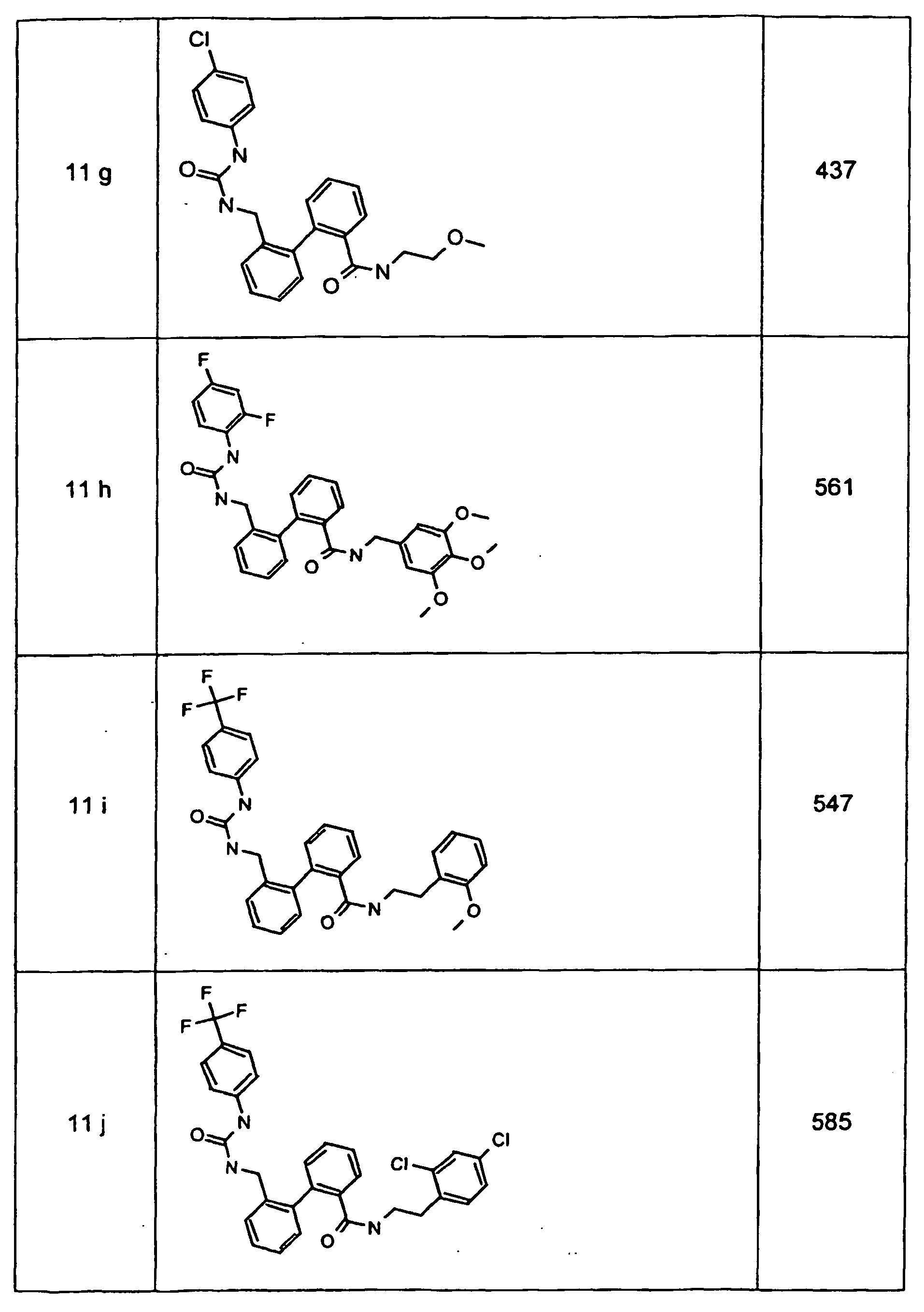

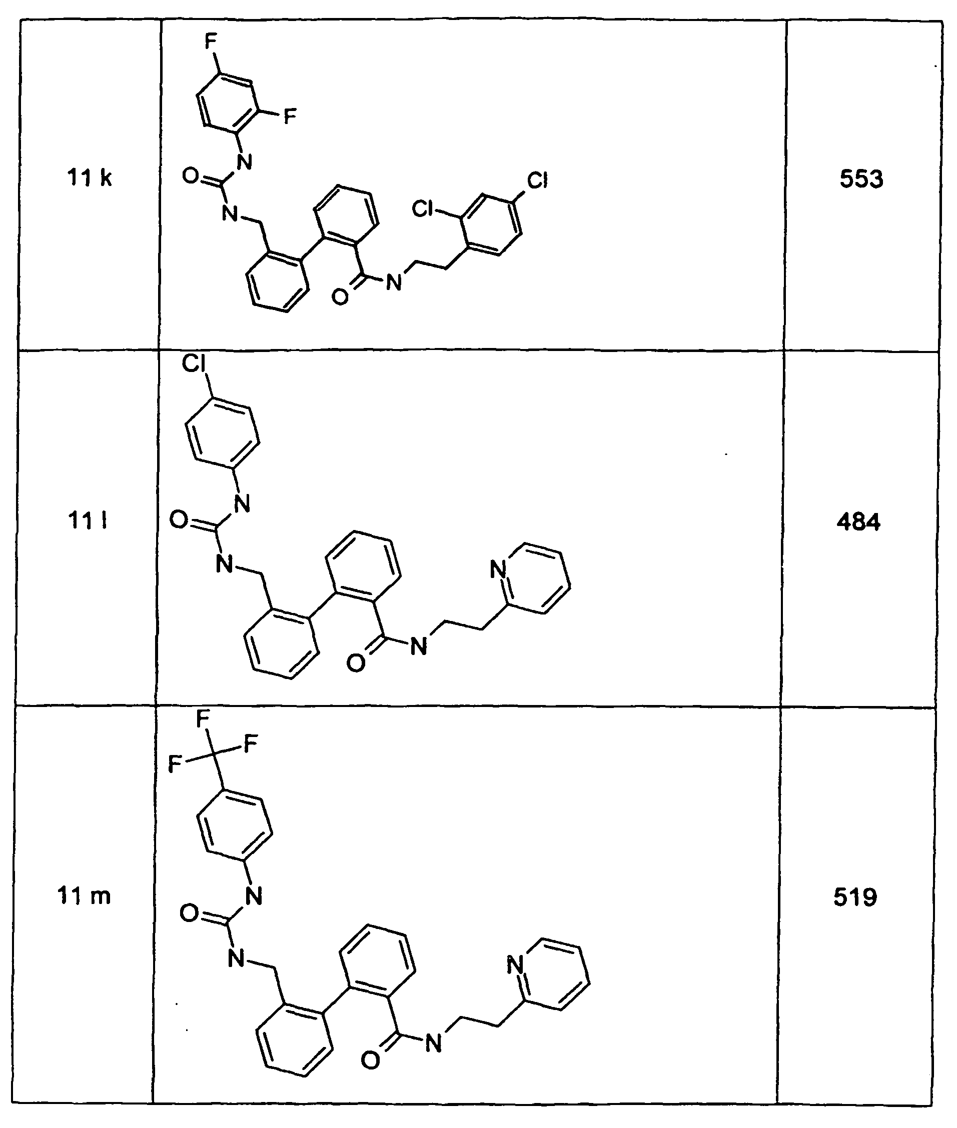

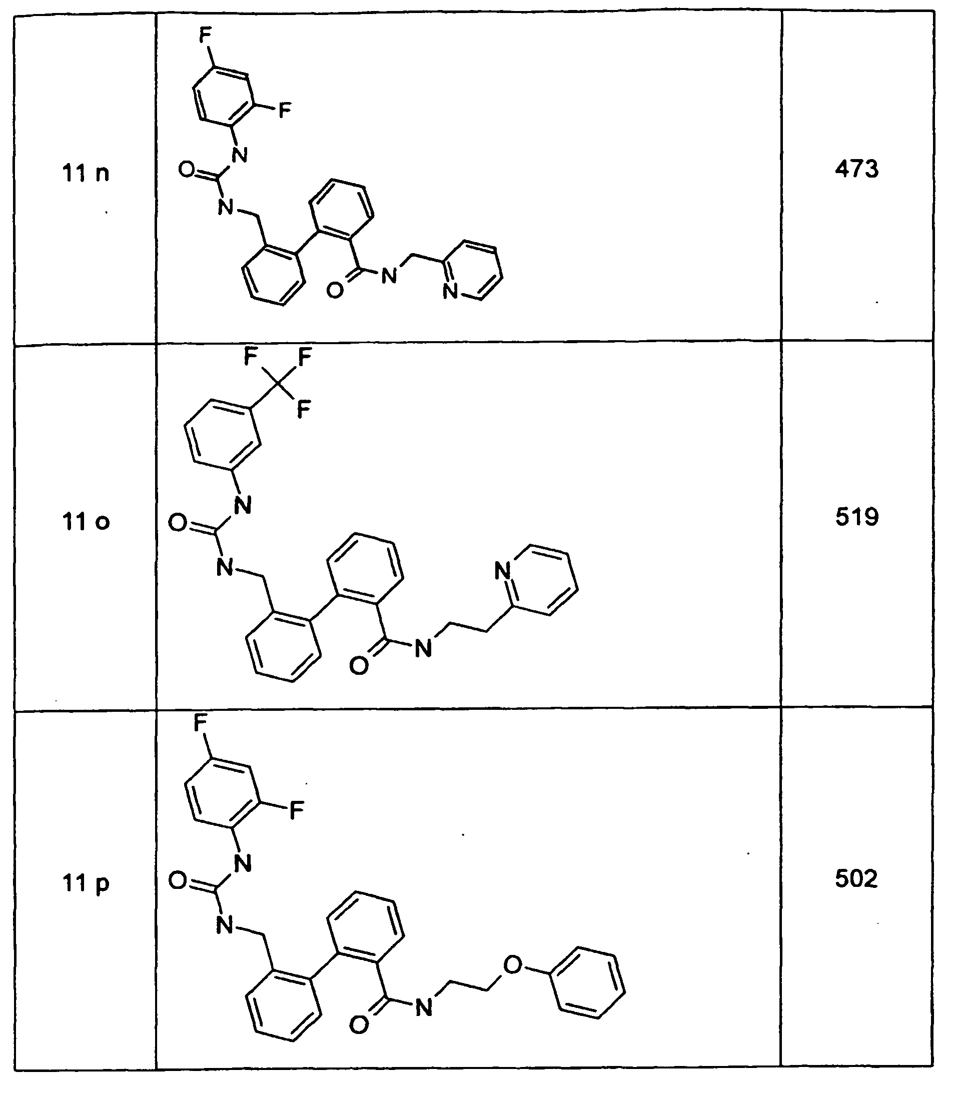

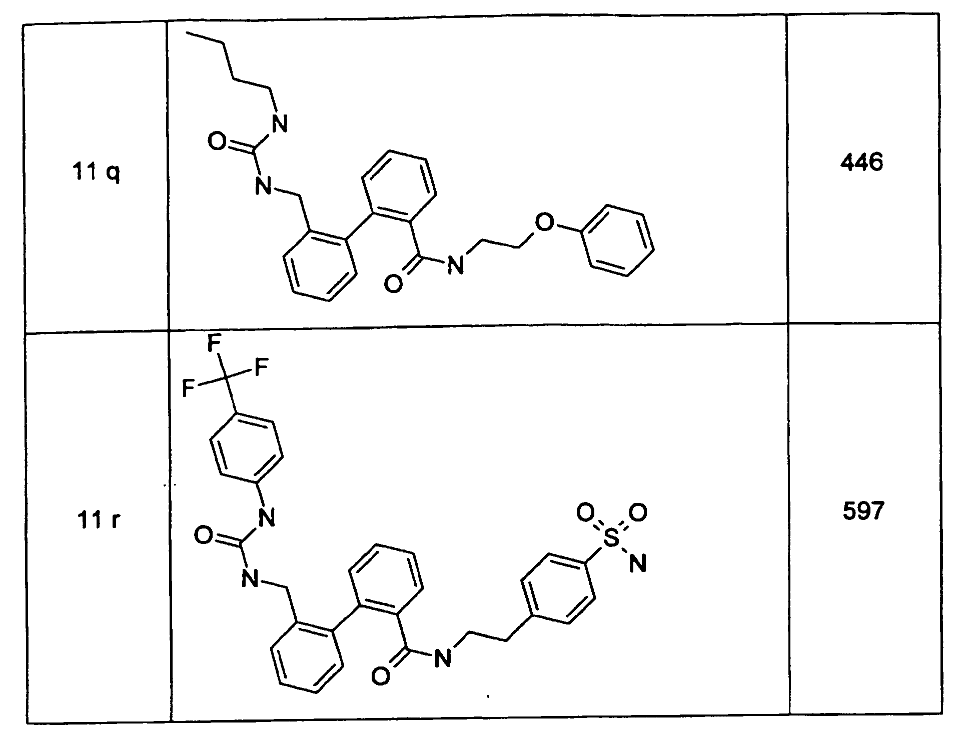

Allgemeine Arbeitsvorschriften zur Herstellung erfindungsgemäßer Verbindungen mittels Festphasensynthese:
Die Mengenangaben in den Vorschriften beziehen sich jeweils immer auf die Harzbeladung, die UV-photometrisch nach Abspaltung der Fmoc-Schutzgruppe ermittelt wurden (siehe zum Beispiel "The Combinatorial Chemistry Catalog", Novabiochem).

### Allgemeine Vorschrift zur Kupplung von α-Fmoc- Aminosäuren an Rink - Amidharz

Man gab zu Rink - Amid Polystyrolharz (Beladung 1,2 mmol/g) eine Lösung von je 1,5 Equivalenten HOBT, TOTU, DIPEA und der α-Fmoc Aminosäure in DMF (5 ml/g Harz) und ließ das Gemisch 12 h bei Raumtemperatur schütteln. Das Harz wurde abfiltriert und 3 mal mit je 10 ml DMF, einmal mit 10 ml Toluol, einmal mit 10 ml Methanol und 3 mal mit 10 ml Dichlormethan gewaschen. Die Bestimmung der Beladung nach der Fmoc-Methode ergab eine Beladung von 0,9 mmol/g Träger.

### Abspaltung der Fmoc-Schutzgruppe

Zur Abspaltung der Fmoc-Schutzgruppe wurde das Harz 5 min in DMF bei Raumtemperatur vorgequollen. Nach Zugabe einer Lösung von DMF/Piperidin (4 ml/g Harz, 1 : 1) wurde 20 min bei Raumtemperatur geschüttelt. Die Lösung wurde abgesaugt und der Vorgang wiederholt. Die Abspaltung einer analytischen Probe ergab vollständige Umsetzung nach HPLC/MS Untersuchung. Nach vollständiger Umsetzung wurde das Harz dreimal mit Dichlormethan gewaschen und direkt in die Kupplung eingesetzt.

### Allgemeine Arbeitsvorschrift zur Kupplung der harzgebundenen Aminosäuren mit der 2'- Phthalimidomethyl-biphenyl-2-carbonsäure (Vorstufe 2)

Man gab zu 100 mg Harz beladen mit der Aminosäure (0,6-0,8 mmol/g) eine Lösung von 12,2 mg (0,09 mmol) HOBT, 29,5 mg (0,09 mmol) TOTU, 16 µl (0,09 mmol) DIPEA und 0,09 mmol der 2'- Phthalimidomethyl-biphenyl-2-carbonsäure (Vorstufe 2) in 5 ml DMF und ließ das Gemisch 12 h bei Raumtemperatur schütteln. Das Harz wurde abfiltriert und 3 mal mit je 10 ml DMF, einmal mit mit 10 ml Toluol, einmal mit 10 ml Methanol und 3 mal mit 10 ml Dichlormethan gewaschen.

### Allgemeine Vorschrift zur Abspaltung der Phthalimido - Schutzgruppe am Träger

Man gab zu 1 g Harz beladen mit der Fmoc geschützten Aminoverbindung 5 ml einer 10%igen Lösung von Hydrazin in DMF und ließ das Gemisch 2 h bei Raumemperatur schütteln. Das Harz wurde abgesaugt. Das Harz wurde danach je dreimal mit je 10 ml DMF und Dichlormethan gewaschen. Die Abspaltung einer analytischen Probe ergab vollständige Umsetzung nach HPLC/MS Untersuchung.

### Allgemeine Vorschrift zur Kupplung mit Sulfonsäurechloriden

Man gab zu 100 mg Harz beladen mit der funktionalisierten 2'- Aminomethylbiphenyl-2-carbonsäure eine Lösung 0.16 ml (0.027 mmol) DIPEA und 0.027 mmol des Sulfonsäurechlorides in 5 ml DMF und ließ das Gemisch 12 h bei Raumtemperatur schütteln. Das Harz wurde abfiltriert und 3 mal mit je 10 ml DMF, einmal mit 10 ml Toluol, einmal mit 10 ml Methanol und 3 mal mit 10 ml Dichlormethan gewaschen.

### Allgemeine Arbeitsvorschrift zur Abspaltung vom Harz

Zur Abspaltung wurde das Harz in Dichormethan/Trifluoressigsäure (3 ml / 0,1 g Harz) suspendiert und für 1h geschüttelt. Das Harz wurde filtriert und mit 1 ml Dichlormethan gewaschen. Die vereinigte Abspaltlösung wurde in Rotationskonzentrator eingeengt. Der Rückstand wurde in Dichlormethan aufgenommen und mit Dichlormethan und Essigester über Kieselgel chromatographiert oder durch präparative HPLC gereinigt.

Auf diese Weise wurden unter anderem folgende Produkte erhalten:

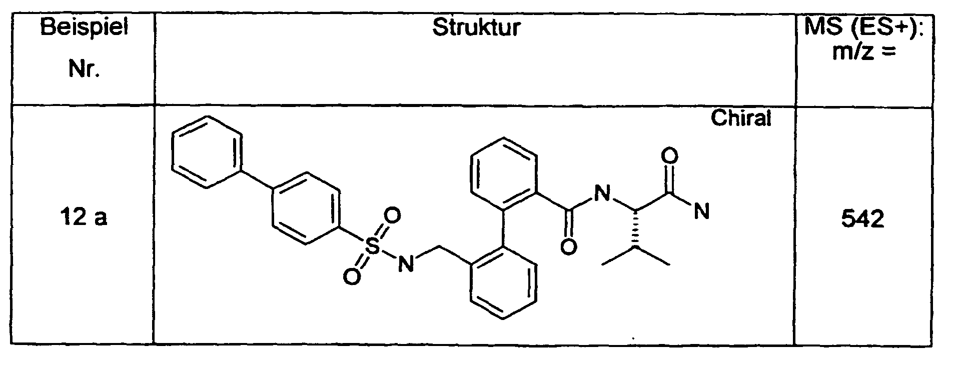

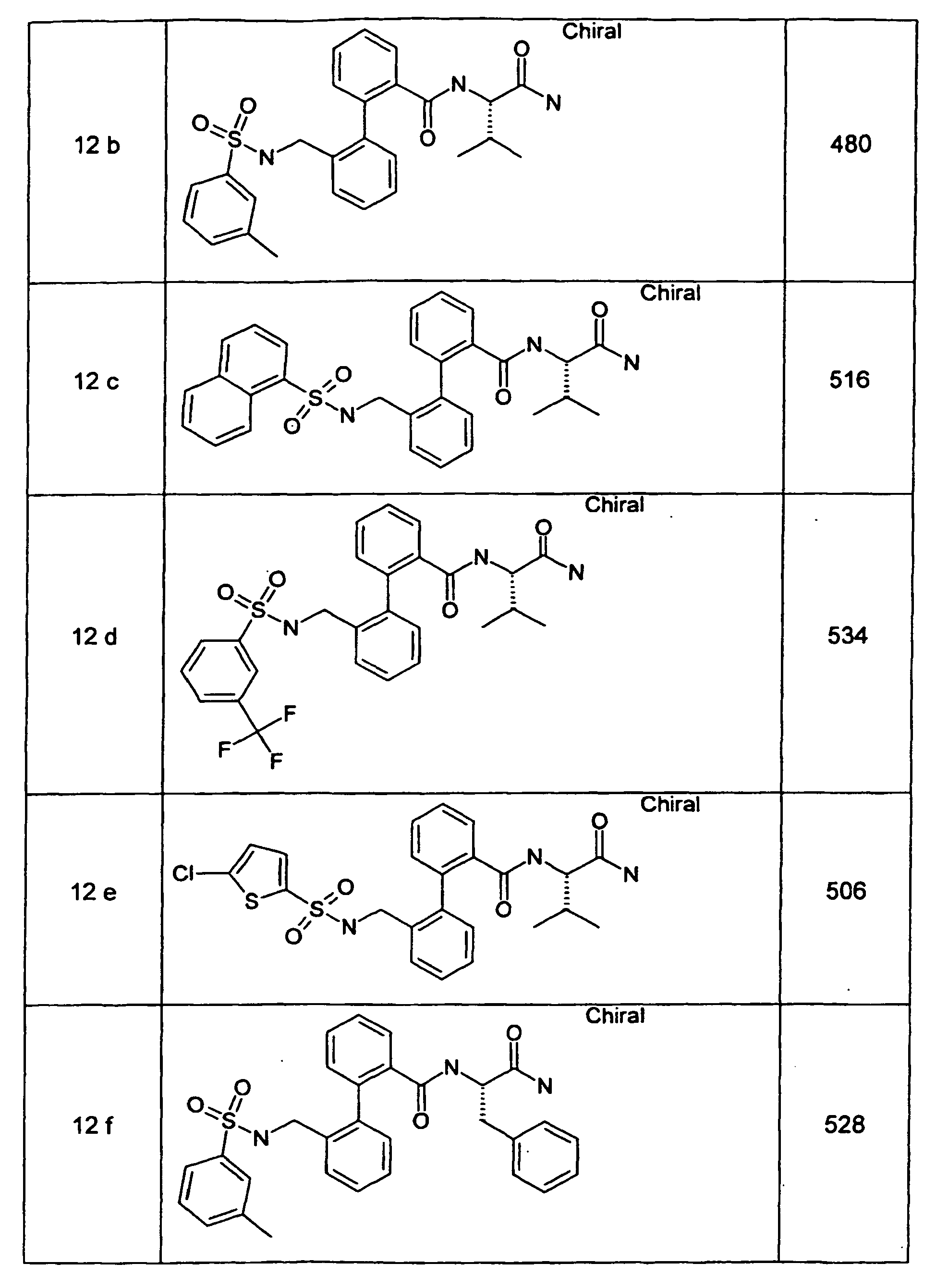

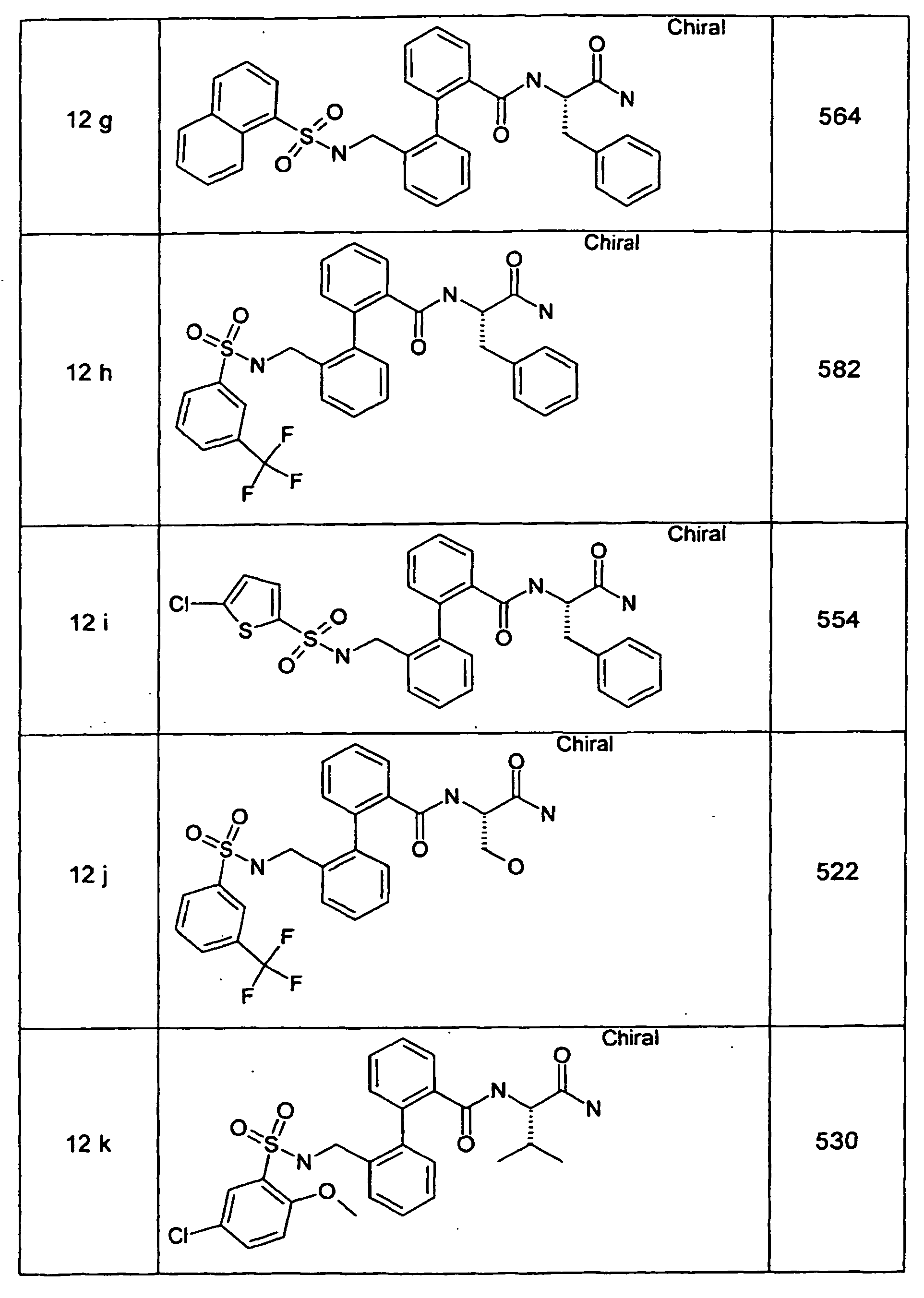

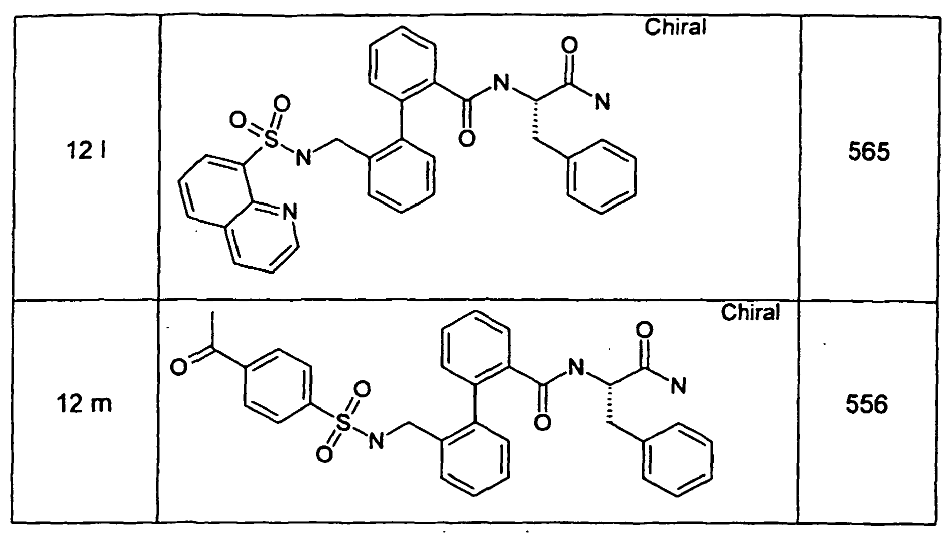

### Beispiel 13 a: 2-{[2'-(Benzyloxycarbonylamino-methyl)-biphenyl-2-carbonyl]-amino}-4-methyl-pentansäure

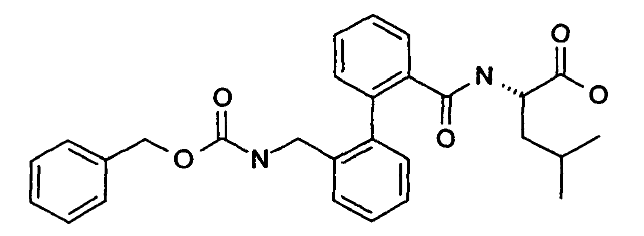

Die Verbindung wurde erhalten aus dem Methylester des Beispiels 8 r durch Verseifung mit Kaliumhydroxid in Methanol/Wasser bei 60°C.

### Beispiele 13 b - 13 e

Durch Kupplung der Carbonsäure des Beispiels 13 a mit den entsprechenden Aminen gemäß der in Beispiel 8 angegebenen allgemeinen Methode wurden die folgenden Verbindungen erhalten:

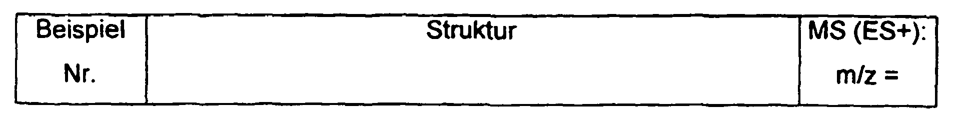

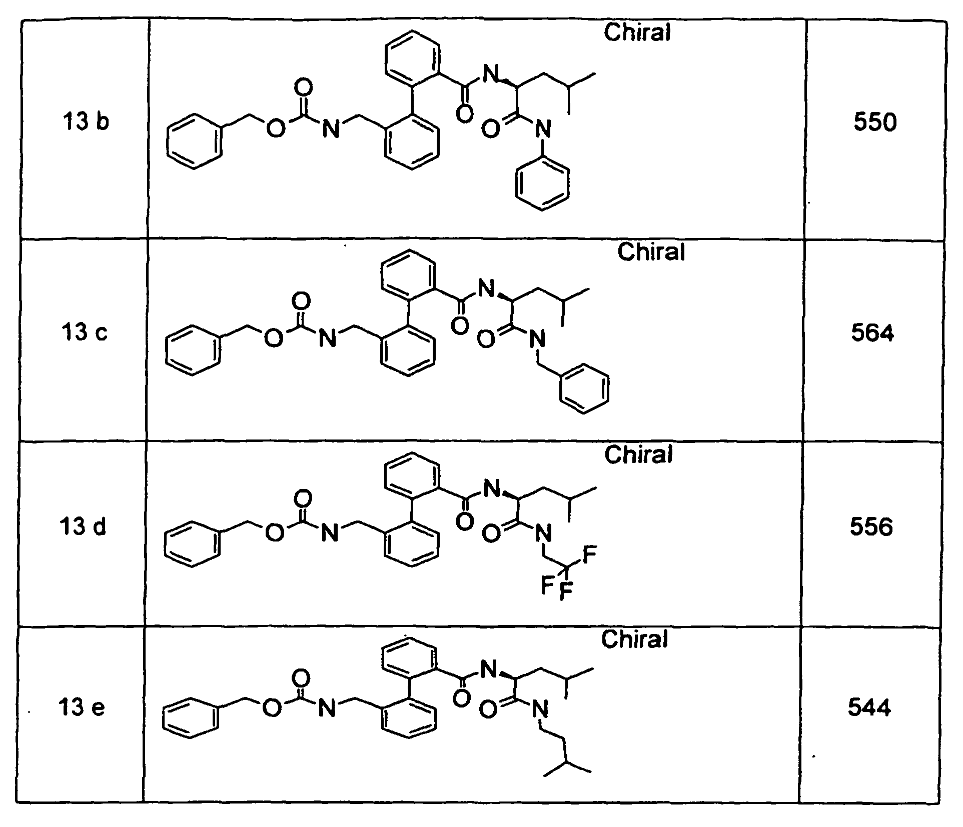

Durch hydrogenolytische Abspaltung der Z-Schutzgruppe der Verbindung des Beispiels 13 c und anschließende Umsetzung mit den entsprechenden Säurechloriden wurden folgende Verbindungen erhalten:

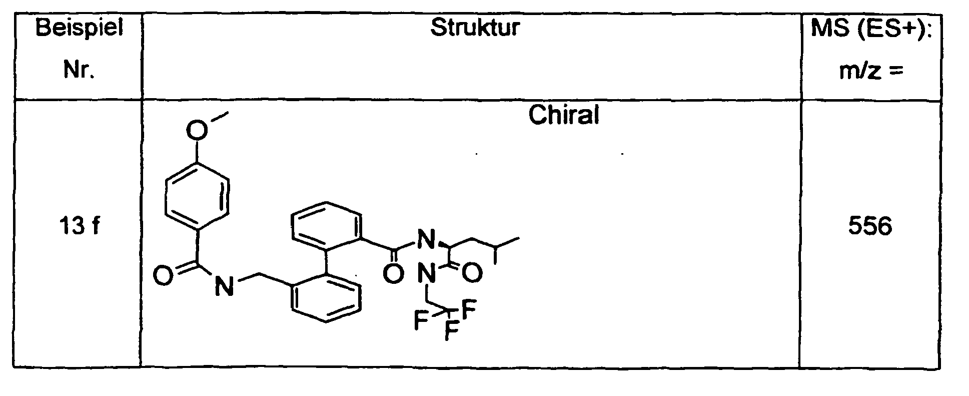

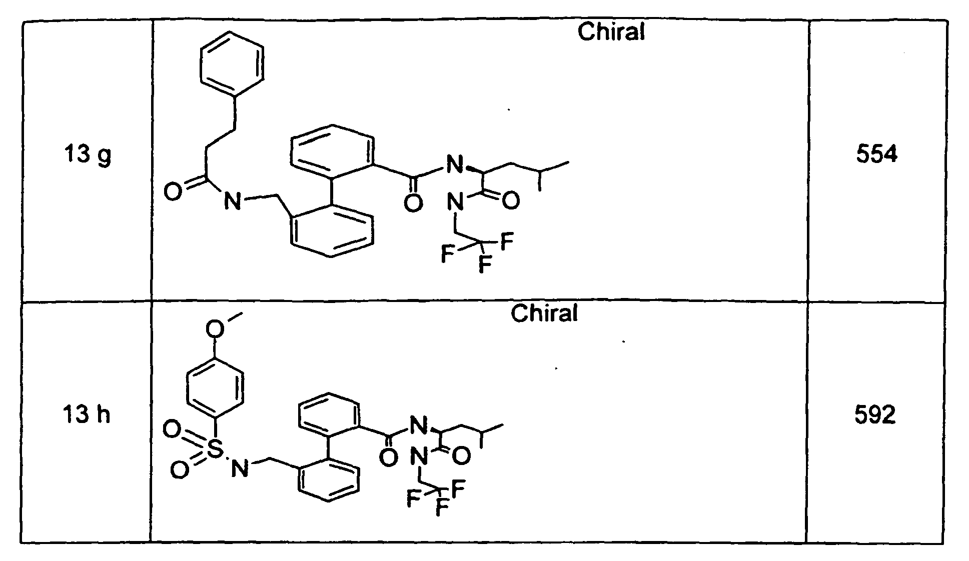

Ausgehend von der Verbindung des Beispiels 8 z wurde analog der Beispiele 13 a - 13 e durch Hydrolyse und Umsetzung mit Isopropylamin folgende Verbindung erhalten:

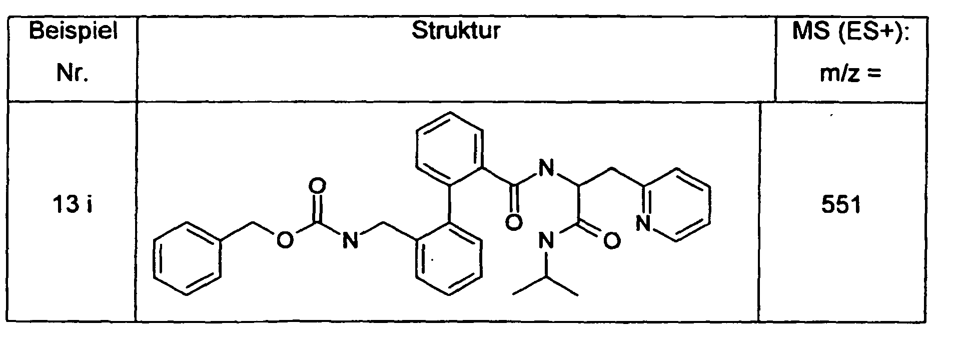

Allgemeine Vorschrift zur Kupplung von 2'-Aminomethyl-biphenyl-2-carbonsäure-(2,4-difluorbenzyl)-amid mit Carbonsäuren zu Carbonamiden (Beispiele 14 a - 14 f): 0,27 mmol der entsprechenden Carbonsäure wurden mit 0,27 mmol HOBT und 0,27 mmol EDAC in 1 ml THF 30 min bei RT gerührt. Dann wurden 0,26 mmol 2'-Aminomethyl-biphenyl-2-carbonsäure-(2,4-difluorbenzyl)-amid-trifluoracetat gelöst in 1 ml THF hinzugegeben und es wurde über Nacht bei RT gerührt. Die Reaktionsmischung wurde mit EE verdünnt und mit Natriumbicarbonatlösung und Wasser gewaschen. Nach Einengen der organischen Phase wurden die Produkte über präparative HPLC gereinigt.

Auf diese Weise wurden die folgenden Verbindungen hergestellt:

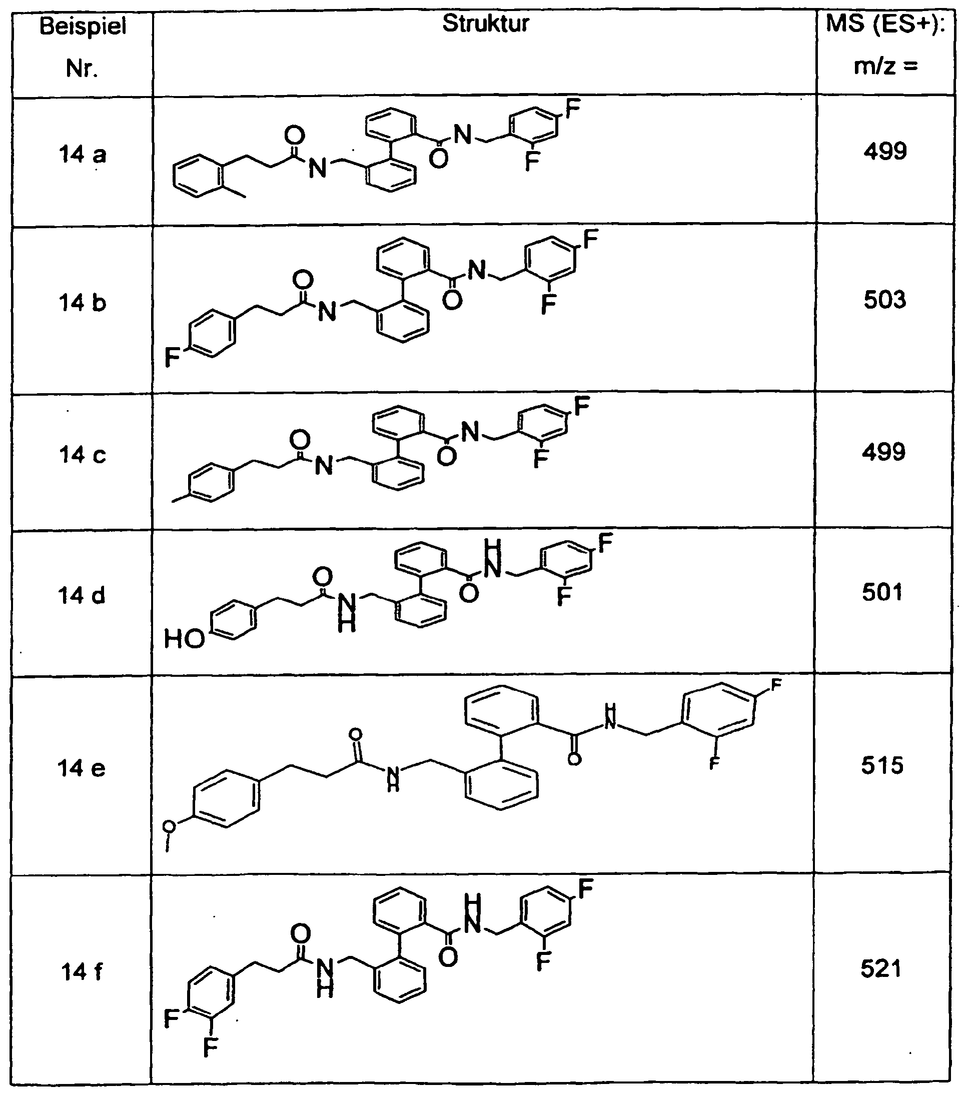

### Allgemeine Vorschrift zur Synthese von Biphenylen durch Suzuki-Kupplung (Beispiele 15 a -15 b)

Zu mit Argon begastem Dimethoxyethan (10 ml) wurden 58 mg (0,05 mmol) Tetrakis-triphenylphosphin-palladium und 1 mmol des entsprechenden Bromids zugegeben. Nach 10 min wurden 1,5 mmol der entsprechenden Boronsäure zugesetzt und zuletzt 1 ml einer 2 molaren Natriumcarbonatlösung (2 mmol). Es wurde 18 h zum Rückfluss unter Argon erhitzt, abgekühlt und mit 30 ml Methylenchlorid verdünnt. Die Mischung wurde mit Wasser und gesättigter Kochsalzlösung gewaschen, über Natriumsulfat getrocknet, eingeengt und durch Chromatographie über Kieselgel gereinigt.

### Beispiel 15 a: 2'-(tert.-Butoxycarbonylamino-methyl)-4-nitro-biphenyl-2-carbonsäure-(3-methyl-butyl)-amid

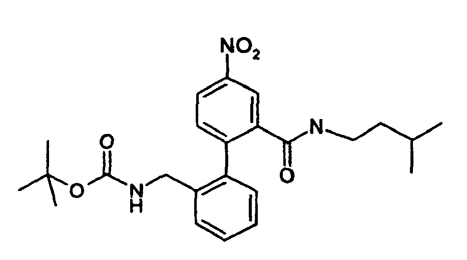

Nach der allgemeinen Vorschrift wurden 350 mg (79% Ausbeute) der nitro-substituierten Verbindung als gelber Feststoff erhalten.

### Beispiel 15 b: 2'-(t-Butoxycarbonylamino-methyl)-4-methoxy-biphenyl-2-carbonsäure-(3-methyl-butyl)-amid

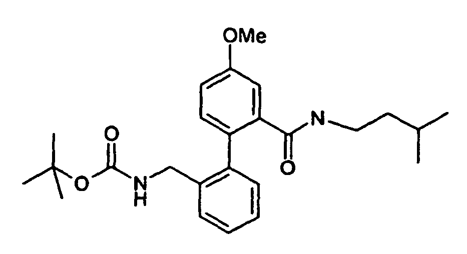

Nach der allgemeinen Vorschrift wurden 170 mg (41% Ausbeute) der methoxy-substituierten Verbindung als zähes helles Öl erhalten.

### Beispiel 16 a: 2'-(tert-Butoxycarbonylamino-methyl)-4-amino-biphenyl-2-carbonsäure-(3-methyl-butyl)-amid

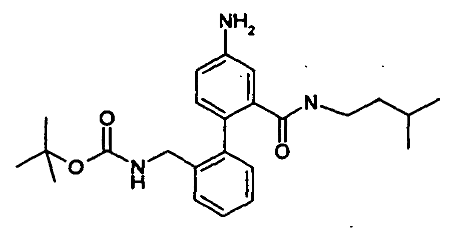

330 mg (0,75 mmol) der nitro-substituierten Verbindung des Beispiels 15a wurden in Ethylacetat gelöst und mit einer Spatelspitze 10 % Palladium auf Kohle unter Wasserstoffatmosphäre (1 bar) hydriert. Nach 2 h wurde über Celite filtriert und die klare Lösung eingeengt. Ausbeute: 260 mg (84%).

### Beispiel 16 b: 2'-(Benzyloxycarbonylamino-methyl)-4-hydroxy-biphenyl-2-carbonsäure-(3-methyl-butyl)-amid

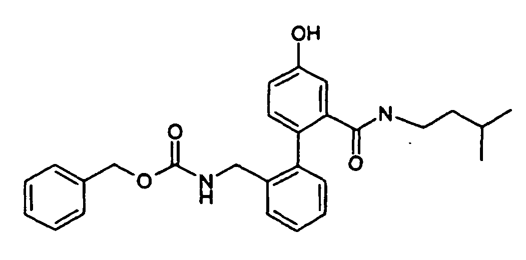

150 mg (0,35 mmol) der methoxy-substituierten Verbindung des Beispiels 15 b wurden in 5 ml wasserfreiem Methylenchlorid gelöst und bei -70°C mit 1,4 ml (1,4 mmol) einer 1 molaren Lösung von Bortribromid in n-Hexan langsam versetzt. Nach 10 min wurde die Reaktionsmischung langsam auf 0°C erwärmt. Nach 2 h bei dieser Temperatur wurde mit gesättigter Natriumhydrogencarbonatlösung neutralisiert, mit insgesamt 40 ml Methylenchlorid extrahiert, über Natriumsulfat getrocknet und eingengt. Vom erhaltenen Rohprodukt (88 mg) 2'-Aminomethyl-4-hydroxy-biphenyl-2-carbonsäure (3-methyl-butyl)-amid wurden 30 mg (0,1 mmol) in 3 ml Methylenchlorid gelöst, mit 11 mg (0,11 mmol) Triethylamin und 27 mg (0,11 mmol) Benzyloxycarbonyloxysuccinimid versetzt. Nach 3 h wurde mit Methylenchlorid verdünnt, mit Wasser gewaschen, die organische Phase über Natriumsulfat getrocknet und per RP-HPLC gereinigt. Man erhielt 8 mg 2'-(Benzyloxycarbonylamino-methyl)-4-hydroxy-biphenyl-2-carbonsäure-(3-methyl-butyl)-amid als dunkles Öl.

### Beispiel 17a: {1-[2'-(3-Methyl-butylcarbamoyl)-biphenyl-2-yl]-ethyl}-carbaminsäuretert.-Butylester

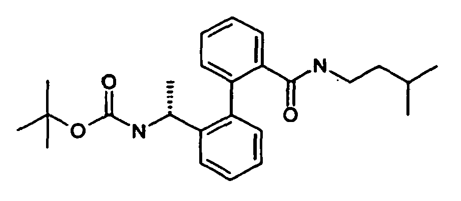

2.2 g (10 mmol) N-Boc-(R)-Phenethylamin wurden in 50 ml wasserfreiem THF gelöst, auf -78°C gekühlt und tropfenweise mit 14 ml (1.5 M Lösung in Pentan, 21 mmol) tert.Butyllithium versetzt. Während 2 h wurde auf -20°C erwärmt, anschliessend 4.5 ml (40 mmol) Borsäuretrimethylester zugegeben und auf RT erwärmt. Die Lösung wurde auf 0°C gekühlt, mit 10% HCl bis auf pH 6 angesäuert, die wässrige Phase mit Dichlormethan extrahiert, die vereinigten organischen Phasen mit gesättigter NaCl-Lösung gewaschen, getrocknet und eingeengt. Man erhielt die Boronsäure als einen hellgelben festen Schaum der ohne weitere Reinigung verwendet wurde.
Die Suzuki-Kuplung wurde gemäss der allgemeinen Arbeitsvorschrift (siehe Beispiel 15) mit 1 mmol 2-Brom-N-(3-methyl-butyl)-benzamid durchgeführt und ergab nach chromatographischer Reinigung 85 mg (0.2 mmol) des Biphenyls.

### Beispiele 17 b - 17 e:

Analog Beispiel 17 a wurde das Enantiomer 17 b erhalten. Durch Abspaltung der Boc-Gruppe und Überführung in die entsprechenden Carbamate wurden die Verbindungen 17a und 17b in die Verbindungen der Beispiele 17 c - 17 e umgewandelt.

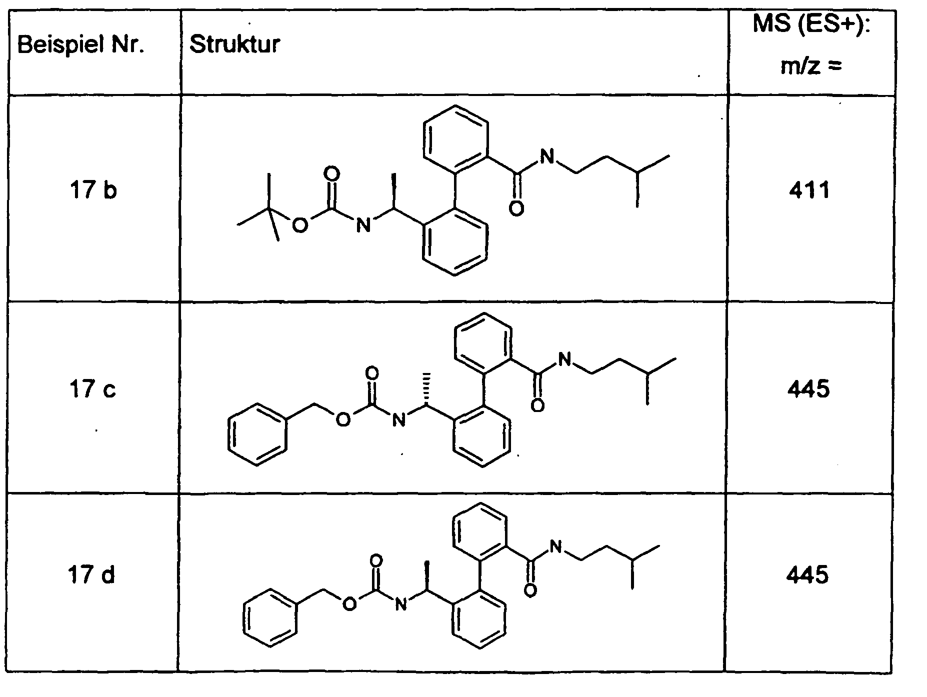

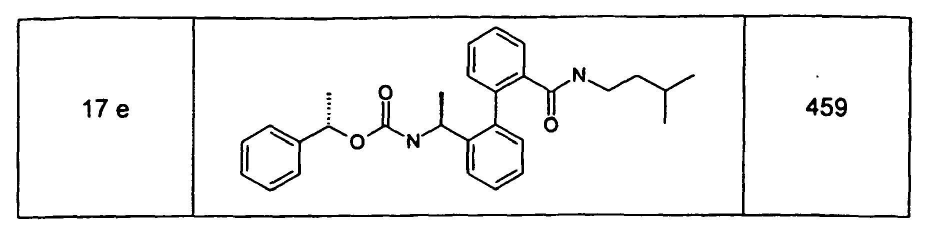

Analog den in den Beispielen 1 bis 17 beschriebenen Verfahrensweisen wurden folgende Verbindungen hergestellt:

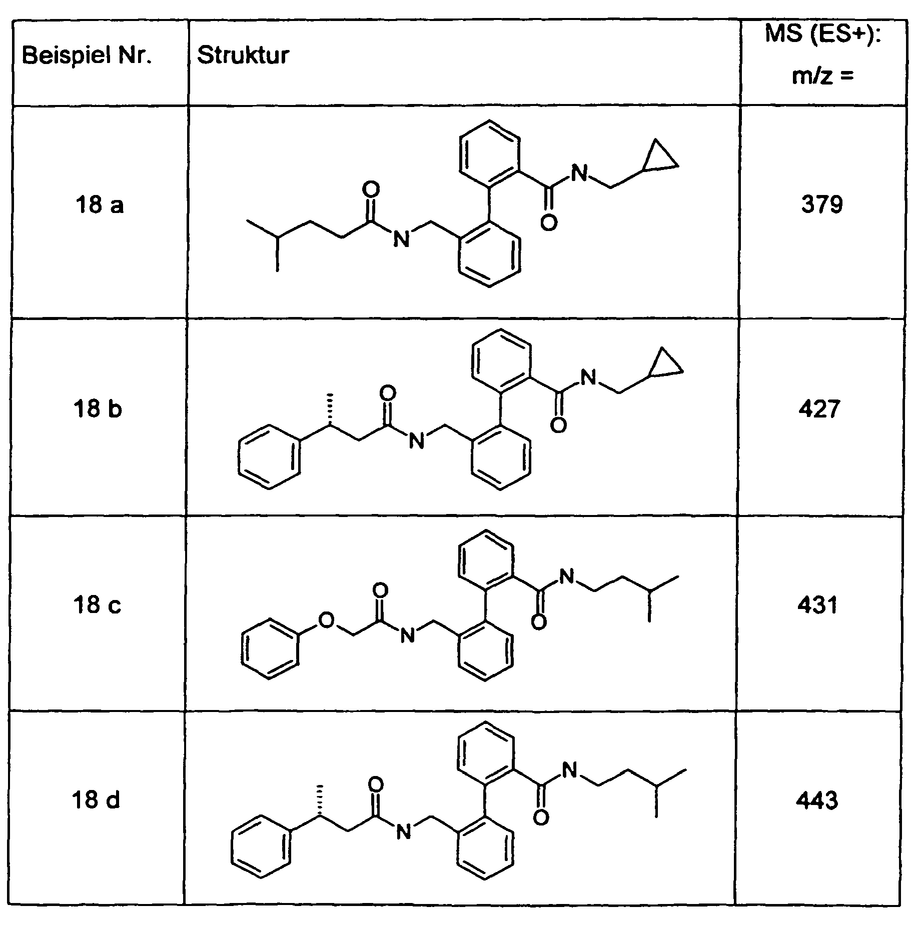

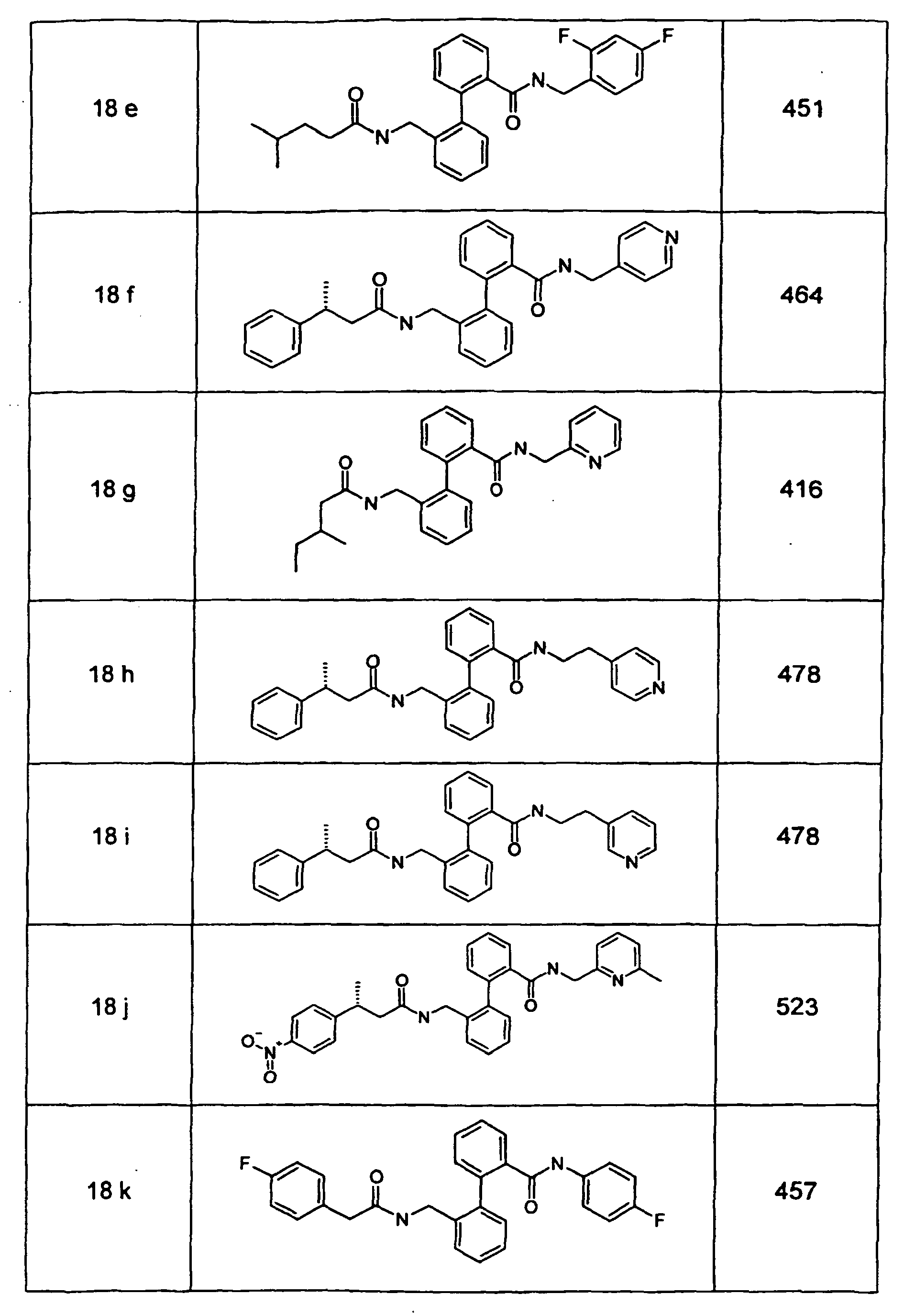

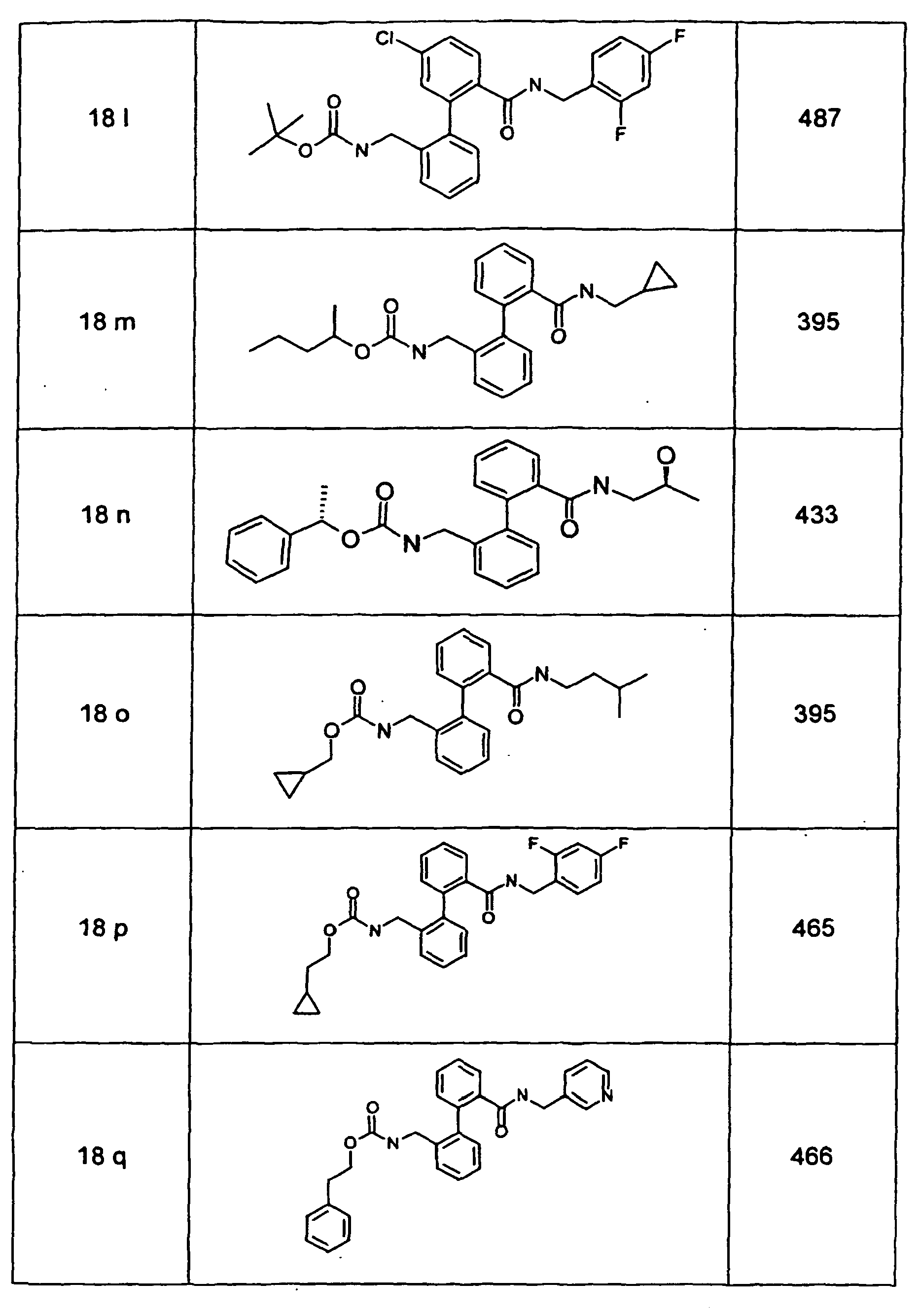

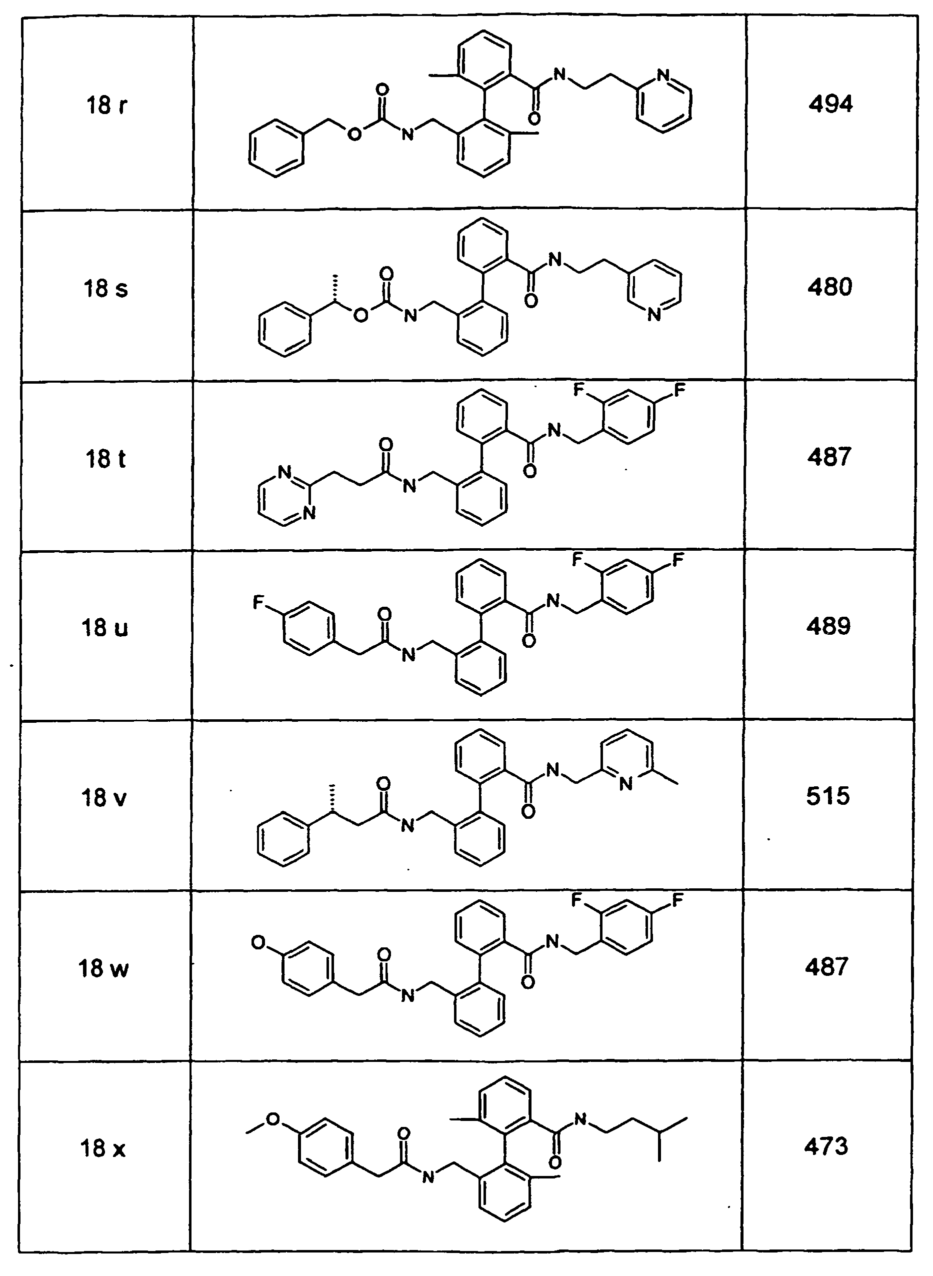

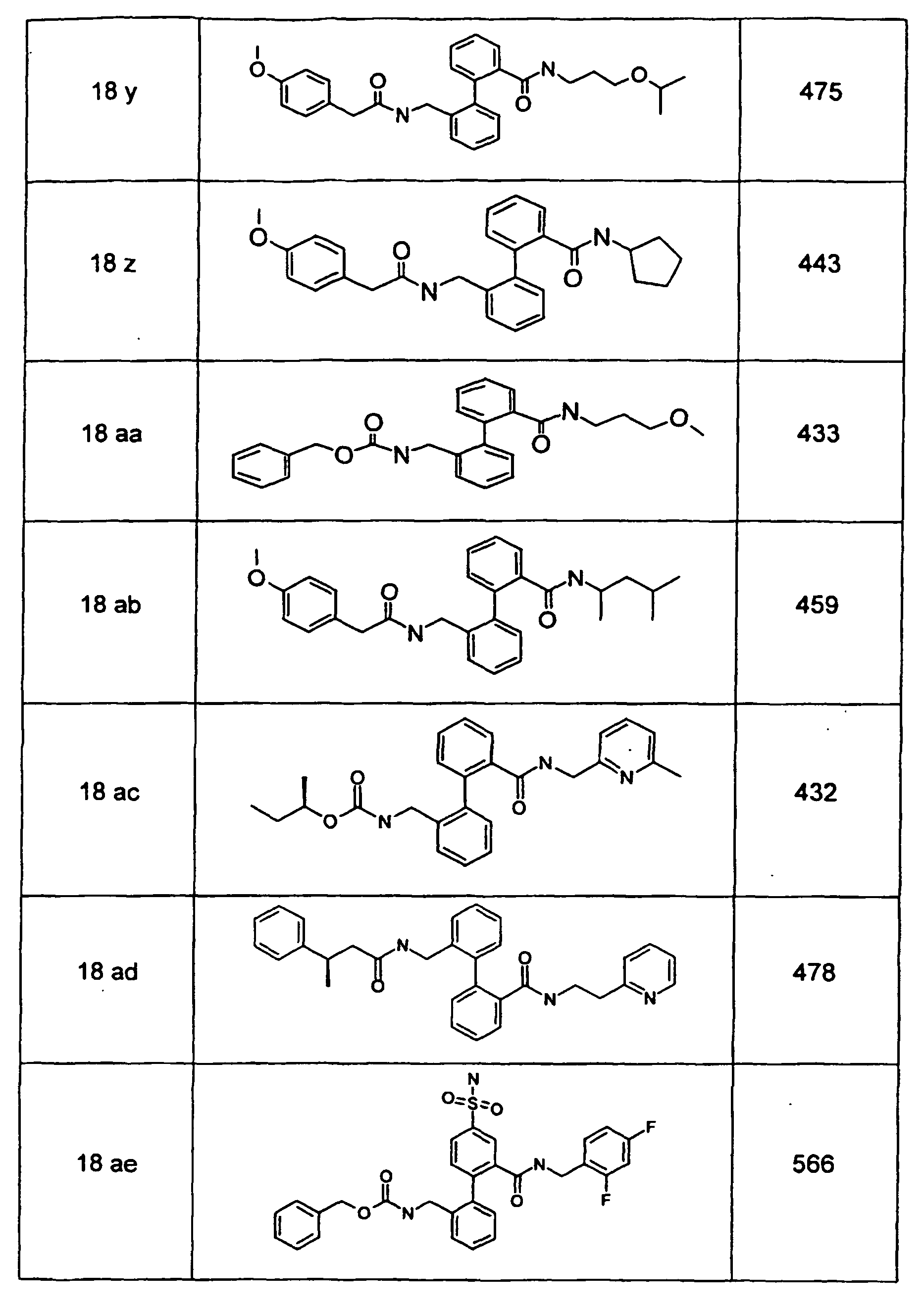

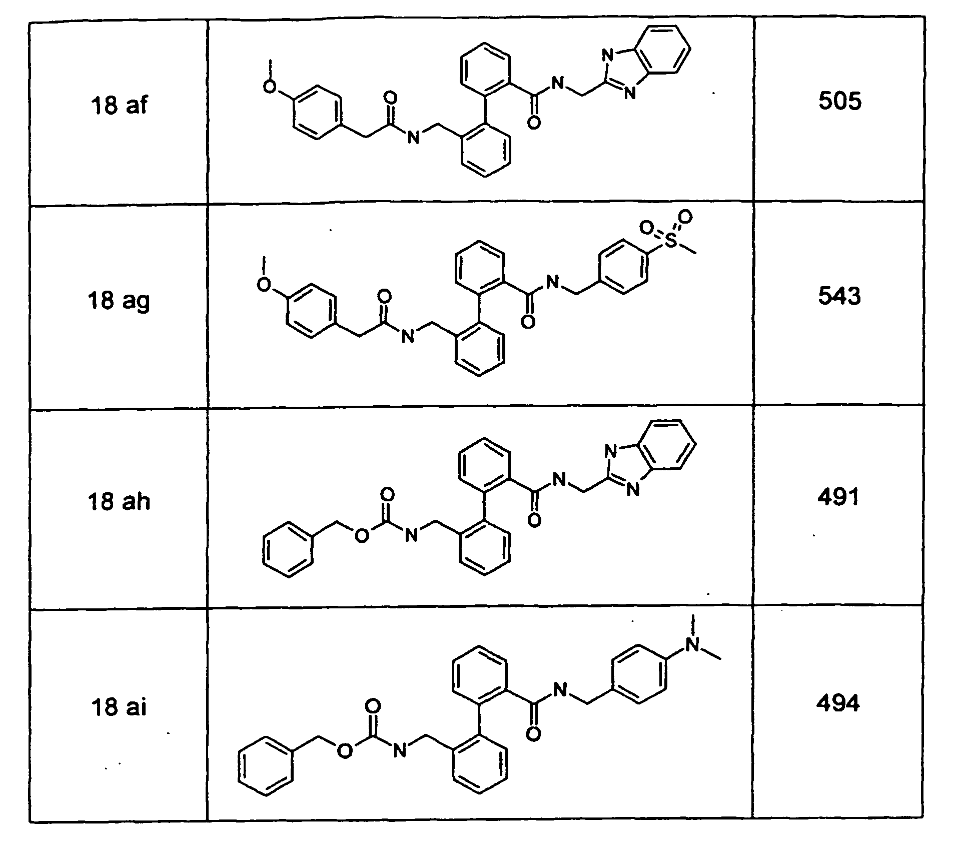

### Pharmakologische Untersuchungen

Kv1.5-Kanäle aus dem Menschen wurden in Xenopus Oozyten expremiert. Hierfür wurden zuerst Oozyten aus Xenopus laevis isoliert und defollikuliert. Anschließend wurde in diese Oozyten in vitro synthetisierte Kv1.5 kodierende RNA injiziert. Nach 1 - 7 Tagen Kv1.5-Proteinexpression wurden an den Oozyten mit der Zwei-Mikroelektroden Voltage-Clamp Technik Kv1.5-Ströme gemessen. Die Kv1.5-Kanäle wurden hierbei in der Regel mit 500 ms dauernden Spannungssprüngen auf 0 mV und 40 mV aktiviert. Das Bad wurde mit einer Lösung der nachfolgenden Zusammensetzung durchspült: NaCl 96 mM, KCl 2 mM, CaCl2 1,8 mM, MgCl2 1 mM, HEPES 5 mM (titriert mit NaOH auf pH 7,4). Diese Experimente wurden bei Raumtemperatur durchgeführt. Zur Datenerhebung und Analyse wurden eingesetzt: Geneclamp Verstärker (Axon Instruments, Foster City, USA) und MacLab D/A-Umwandler und Software (ADInstruments, Castle Hill, Australia). Die erfindungsgemäßen Substanzen wurden getestet, indem sie in unterschiedlichen Konzentrationen der Badlösung zugefügt wurden. Die Effekte der Substanzen wurden als prozentuale Inhibition des Kv1.5-Kontrollstromes berechnet, der erhalten wurde, wenn der Lösung keine Substanz zugesetzt wurde. Die Daten wurden anschließend mit der Hill-Gleichung extrapoliert, um die Hemmkonzentrationen IC50 für die jeweiligen Substanzen zu bestimmen.

Auf diese Weise wurden für die nachfolgend aufgeführten Verbindungen folgende IC₅₀-Werte bestimmt:

| Beispiel | IC₅₀ | Beispiel | IC₅₀ | Beispiel | IC₅₀ | Beispiel | IC₅₀ |
|---|---|---|---|---|---|---|---|
| Nr. | [µM] | Nr. | [µM] | Nr. | [µM] | Nr. | [µM] |
| 1 a | 6,1 | 2 a | 2,6 | 4 a | 4,1 | 6 h | 3,0 |
| 1 b | 3,3 | 2 b | 0,8 | 4 c | 1,4 | 7 a | ~6,0 |
| 1 d | 1,0 | 2 c | 0,7 | 4 d | 1,8 | 8 a | 0,3 |
| 1 e | 0,5 | 2 d | 1,7 | 4 g | 3,4 | 8 b | 0,9 |
| 1 f | 0,4 | 2 e | 3,4 | 4 h | 1,8 | 8 d | 6,4 |
| 1 g | 0,4 | 2 f | 7,1 | 4 i | 4,7 | 8 j | 4,5 |
| 1 h | 4,3 | 2 g | 3,3 | 4 j | 7,1 | 8 k | 3,1 |
| 1 i | 1,7 | 2 h | 2,5 | 4 k | 2,2 | 8 l | 3,5 |
| 1 j | 0,2 | 2 i | 3,3 | 4 l | 0,8 | 8 m | 5,2 |
| 1 k | 2,4 | 2 j | 2,5 | 5 a | 4,5 | 8 n | 3,7 |
| 1 l | 1,4 | 2 k | 3,8 | 5 c | 7,8 | 8 o | 8,4 |
| 1 m | 0,7 | 2 m | 2,6 | 5 d | 1,9 | 8 p | 1,4 |
| 1 n | 1,4 | 3 d | 1,7 | 5 e | 7,2 | 8 q | 7,3 |
| 1 o | 4,4 | 3 k | 2,4 | 6 a | 4,4 | 8 r | 1,0 |
| 1 r | 0,8 | 3 l | 2,6 | 6 b | 1,8 | 8 s | 1,0 |
| 1 s | 1,7 | 3 p | 1,9 | 6 c | 2,5 | 8 x | 3,3 |
| 1t | 1,3 | 3 r | 1,5 | 6 d | 3,1 | 8 y | 2,8 |
| 1 u | 0,8 | 3 | 3,0 | 6 e | 3,6 | 8 z | 1,6 |
| 8 aa | 0,8 | 8 ab | 1,2 | 8 ac | 1,1 | 9 b | 3,0 |
| 9 c | 2,0 | 9 f | 2,2 | 9 g | 2,2 | 11 a | 2,3 |
| 11 b | 7,3 | 11 d | 3,3 | 11 g | 7,8 | 11 h | 5,8 |
| 11 l | 2,7 | 11 m | 3,3 | 11 n | 5,9 | 11 o | 4,4 |
| 11 p | 7,3 | 12 c | 11,2 | 12 f | 11,3 | 12 g | 9,1 |
| 12 h | 4,8 | 12 1 | 10,3 | 12 m | 7,7 | 13 b | ~3,0 |
| 13 c | 1,4 | 13 d | 0,5 | 13 e | 2,8 | 13 f | 3,4 |
| 13 g | 1,1 | 13 h | 1,4 | 13 i | 1,2 | 14 a | 3,6 |
| 14 b | 2,7 | 14 d | 2,0 | 14 e | 0,8 | 14 f | 2,5 |
| 15 b | 3,1 | 16 b | 5,2 | 18 a | 7,2 | 18 b | 0,4 |
| 18 c | 4,2 | 18 d | 0,4 | 18 e | 1,7 | 18 f | 1,3 |
| 18 g | 3,9 | 18 h | 0,8 | 18 i | 0,4 | 18 j | 0,7 |
| 18 k | 3,0 | 18 m | 2,1 | 18 n | 0,4 | 18 o | 3,6 |
| 18 p | 4,7 | 18 q | 3,2 | 18 r | 0,7 | 18 s | 0,9 |
| 18 u | 1,1 | 18 v | 0,4 | 18 w | 5,4 | 18 x | 4,6 |
| 17 d | 1,3 | 17 e | 1,8 | 17 c | 2,1 | 18y | 1,9 |
| 18 z | 1,2 | 18 aa | 0,4 | 18 ab | 1,1 | 18 ac | 10 |
| 18 ad | 0,3 | 18 af | 5,8 | 18 ah | 2,1 | 18 ai | 6,6 |

## Patentansprüche

1. Verbindungen der Formel I, 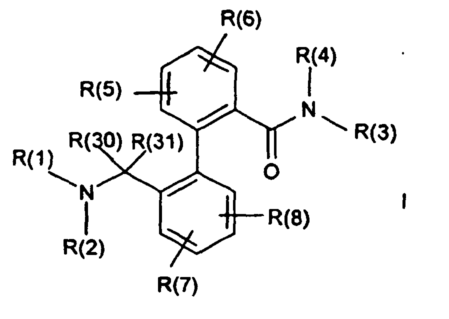 worin bedeuten:
R(1) C(O)OR(9), SO₂R(10), COR(11), C(O)NR(12)R(13) oder C(S)NR(12)R(13);
R(9) CₓH₂ₓ-R(14);
x 0, 1, 2, 3 oder 4,
wobei x nicht 0 sein kann, wenn R(14) OR(15) oder SO₂Me bedeutet;
R(14) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(15), SO₂Me, Phenyl, Naphthyl, Biphenylyl, Furyl, Thienyl oder ein N-haltiger Heteroaromat mit 1, 2, 3, 4, 5, 6, 7, 8 oder 9 C-Atomen,
wobei Phenyl, Naphthyl, Biphenylyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Alkyl mit 1, 2, 3, 4 oder 5 C-Atomen, Cycloalkyl mi 3, 4, 5 oder 6 C-Atomen, CF₃ oder Phenyl,
das unsubstituiert ist oder substituiert mit 2 oder 3 Substituenten ausgewählt aus de Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(10), R(11) und R(12) unabhängig von einander wie R(9) definiert;
R(13) Wasserstoff, Alkyl mit 1, 2, 3 oder 4 C-Atomen oder CF₃;
R(2) Wasserstoff, Alkyl mit 1, 2, 3 oder 4 C-Atomen oder CF₃;
R(3) C_{y}H_{2y}-R(16);
y 0, 1, 2, 3 oder 4,
wobei y nicht 0 sein kann, wenn R(16) OR(17) oder SO₂M bedeutet;
R(16) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(17), SO₂Me Phenyl, Naphthyl, Furyl, Thienyl oder ein N-haltiger Heteroaromat mit 1, 2, 3, 4, 5, 6, 7, 8 oder 9 C-Atomen,
wobei Phenyl, Naphthyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(17) Wasserstoff, Alkyl mit 1, 2, 3, 4 oder 5 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
oder
R(3) CHR(18)R(19);
R(18) Wasserstoff oder C_{z}H_{2z}-R(16), wobei R(16) wie oben angegeben definiert ist;
z 0, 1, 2 oder 3;
R(19) COOH, CONH₂, CONR(20)R(21), COOR(22), CH₂OH;
R(20) Wasserstoff, Alkyl mit 1, 2, 3, 4 oder 5 C-Atomen, CᵥH₂ᵥ₋CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
v 0, 1, 2 oder 3;
w 0, 1, 2 oder 3;
R(21) Wasserstoff oder Alkyl mit 1, 2, 3, 4 oder 5 C-Atomen;
R(22) Alkyl mit 1, 2, 3, 4 oder 5 C-Atomen;
R(4) Wasserstoff, Alkyl mit 1, 2, 3, 4, 5 oder 6 C-Atomen oder CF₃;
oder
R(3) und R(4) gemeinsam eine Kette von 4 oder 5 Methylengruppen, von denen eine Methylengruppe durch -O-, -S-, -NH-, -N(Methyl)- oder -N(Benzyl)- ersetzt sein kann;
R(5), R(6), R(7) und R(8) unabhängig voneinander Wasserstoff, F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino;
R(30) und R(31) unabhängig voneinander Wasserstoff oder Alkyl mit 1, 2 oder 3 C-Atomen;
oder
R(30) und R(31) gemeinsam eine Kette von 2 Methylengruppen;
sowie ihre pharmazeutisch akzeptablen Salze.

2. Verbindungen der Formel I nach Anspruch 1, in der bedeuten:
R(1) C(O)OR(9), SO₂R(10), COR(11) oder C(O)NR(12)R(13);
R(9) CₓH₂ₓ-R(14);
x 0, 1, 2, 3 oder 4,
wobei x nicht 0 sein kann, wenn R(14) OR(15) bedeutet;
R(14) Alkyl mit 1, 2, 3 oder 4 C-Atomen, Cycloalkyl mit 3, 4, 5, 6, 7, 8 oder 9 C-Atomen, CF₃, C₂F₅, OR(15), Phenyl, Furyl, Thienyl oder ein N-haltiger Heteroaromat mit 1, 2, 3, 4, 5, 6, 7, 8 oder 9 C-Atomen,
wobei Phenyl, Furyl, Thienyl und der N-haltige Heteroaromat unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, NH₂, OH, Alkyl mit 1,
2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Alkyl mit 1, 2, 3, 4 oder 5 C-Atomen, Cycloalkyl mit 3, 4, 5 oder 6 C-Atomen, CF₃ oder Phenyl, das unsubstituiert ist oder substituiert mit 1, 2 oder 3 Substituenten ausgewählt aus der Gruppe bestehend aus F, Cl, Br, CF₃, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(10), R(11) und R(12) unabhängig von einander wie R(9) definiert;
R(13) Wasserstoff, Alkyl mit 1, 2, 3 oder 4 C-Atomen oder CF₃;
R(2) Wasserstoff, Alkyl mit 1, 2, 3 oder 4 C-Atomen oder CF₃;
R(3) C_{y}H_{2y}-R(16);
y 0, 1, 2, 3 oder 4,
wobei y nicht 0 sein kann, wenn R(16) OR(17) bedeutet;
R(16) Alkyl mit 1, 2, 3 oder 4 C-Atomen, Cycloalkyl mit 3, 4, 5, 6, 7, 8 oder 9 C-Atomen, CF₃, C₂F₅, OR(17), Phenyl, Furyl, Thienyl oder ein N-haltiger Heteroaromat mit 1, 2, 3, 4, 5, 6, 7, 8 oder 9 C-Atomen,
wobei Phenyl, Furyl, Thienyl und der N-haltige Heteroaromat unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(17) Alkyl mit 1, 2, 3, 4 oder 5 C-Atomen, Cycloalkyl mit 3, 4, 5 oder 6 C-Atomen, CF₃, Phenyl oder 2-, 3-, oder 4- Pyridyl,
wobei Phenyl oder 2-, 3-, oder 4- Pyridyl unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
oder
R(3) CHR(18)R(19);
R(18) Wasserstoff oder C_{z}H_{2z}R(16), wobei R(16) wie in Anspruch 1 oben angegeben definiert ist;
z 0, 1, 2 oder 3;
R(19) CONH₂, CONR(20)R(21), COOR(22), CH₂OH;
R(20) Wasserstoff, Alkyl mit 1, 2, 3, 4 oder 5 C-Atomen, CᵥH₂ᵥ-CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
v 0, 1, 2 oder 3;
w 0, 1, 2 oder 3:
R(21) Wasserstoff oder Alkyl mit 1, 2, 3, 4 oder 5 C-Atomen;
R(22) Alkyl mit 1, 2, 3, 4 oder 5 C-Atomen;
R(4) Wasserstoff oder Alkyl mit 1, 2, 3, 4, 5 oder 6 C-Atomen, CF₃;
R(5), R(6), R(7) und R(8) unabhängig voneinander Wasserstoff, F, Cl, Br, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino;
R(30) und R(31) unabhängig voneinander Wasserstoff oder Alkyl mit 1, 2 oder 3 C-Atomen;
oder
R(30) und R(31) gemeinsam eine Kette von 2 Methylengruppen;
sowie ihre pharmazeutisch akzeptablen Salze.

3. Verbindungen der Formel I nach Ansprüchen 1 oder 2, in der bedeuten:
R(1) C(O)OR(9), SO₂R(10), COR(11) oder C(O)NR(12)R(13);
R(9) CₓH_{2X}-R(14);
x 0, 1, 2, 3 oder 4,
wobei x nicht 0 sein kann, wenn R(14) OR(15) bedeutet;
R(14) Cycloalkyl mit 3, 4, 5, 6, 7, 8 oder 9 C-Atomen, CF₃, OR(15), Phenyl, Furyl, Thienyl oder ein N-haltiger Heteroaromat mit 3, 4 oder 5 C-Atomen,
wobei Phenyl, Furyl, Thienyl und der N-haltige Heteroaromat unsubstituiert sind oder substituiert mit 1 oder 2 Substituenten ausgewählt aus der Gruppe bestehend aus F, Cl, Br, CF₃, OCF₃, CN, COOMe, CONH₂, COMe, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(15) Alkyl mit 1 oder 2 C-Atomen, Cycloalkyl mit 3, 4, 5 oder 6 C-Atomen, CF₃ oder Phenyl, das unsubstituiert ist oder substituiert mit 1 oder 2 Substituenten ausgewählt aus der Gruppe bestehend aus F, Cl, Br, CF₃, CN, COOMe, CONH₂, COMe, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(10), R(11) und R(12) unabhängig von einander wie R(9) definiert;
R(13) Wasserstoff;
R(2) Wasserstoff oder Alkyl mit 1, 2 oder 3 C-Atomen;
R(3) CHR(18)R(19);
R(18) Wasserstoff oder C_{z}H_{2z}-R(16);
z 0, 1, 2 oder 3;
R(19) CONH₂, CONR(20)R(21), COOR(22), CH₂OH;
R(20) Wasserstoff, Alkyl mit 1, 2, 3, 4 oder 5 C-Atomen, CᵥH₂ᵥ-CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, CF₃, OCF₃, CN, COOMe, CONH₂, COMe, OH, Alkyl mit 1, 2, oder 3 C-Atomen, Alkoxy mit 1 oder 2 C-Atomen, Dimethylamino, Sulfamoyl, Methylsulfonyl und Methylsulfonylamino;
v 0, 1, 2 oder 3;
w 0, 1, 2 oder 3;
R(21) Wasserstoff oder Alkyl mit 1, 2, 3,4 oder 5 C-Atomen;
R(22) Alkyl mit 1, 2, 3, 4 oder 5 C-Atomen;
R(16) Alkyl mit 1, 2 oder 3 C-Atomen, Cycloalkyl mit 3, 4, 5, 6, 7, 8 oder 9 C-Atomen, CF₃, OR(17), Phenyl, Furyl, Thienyl oder ein N-haltiger Heteroaromat mit 3, 4 oder 5 C-Atomen,
wobei Phenyl, Furyl, Thienyl und der N-haltige Heteroaromat unsubstituiert sind oder substituiert mit 1 oder 2 Substituenten ausgewählt aus der Gruppe bestehend aus F, Cl, Br, CF₃, OCF₃, CN, COOMe, CONH₂, COMe, NH₂, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(17) Alkyl mit 1, 2, 3 oder 4 C-Atomen, Cycloalkyl mit 3, 4, 5 oder 6 C-Atomen, CF₃, Phenyl oder 2-, 3- oder 4- Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, CF₃, OCF₃, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4) Wasserstoff oder Alkyl mit 1 oder 2 C-Atomen;
R(5), R(6), R(7) und R(8) unabhängig voneinander Wasserstoff, F, Cl, Br, CF₃, CN, COOMe, CONH₂, COMe, NH₂, OH, Alkyl mit 1, 2 oder 3 C-Atomen. Alkoxy mit 1 oder 2 C-Atomen, Dimethylamino, Suffamoyl, Methylsulfonyl oder Methylsulfonylamino;
R(30) und R(31) unabhängig voneinander Wasserstoff oder Methyl;
oder
R(30) und R(31) gemeinsam eine Kette von 2 Methylengruppen;
sowie ihre pharmazeutisch akzeptablen Salze.

4. Verbindungen der Formel I nach Ansprüchen 1 oder 2, in der bedeuten:
R(1) C(O)OR(9), SO₂R(10), COR(11) oder C(O)NR(12)R(13);
R(9) CₓH₂ₓ-R(14);
x 0, 1, 2, 3 oder 4,
wobei x nicht 0 sein kann, wenn R(14) OR(15) bedeutet;
R(14) Alkyl mit 1, 2, 3 oder 4 C-Atomen, Cycloalkyl mit 3, 4, 5, 6, 7, 8 oder 9 C-Atomen, CF₃, OR(15), Phenyl, Furyl, Thienyl oder ein N-haltiger Heteroaromat mit 3, 4 oder 5 C-Atomen,
wobei Phenyl, Furyl, Thienyl und der N-haltige Heteroaromat unsubstituiert sind oder substituiert mit 1 oder 2 Substituenten ausgewählt aus der Gruppe bestehend aus F, Cl, Br, CF₃, OCF₃, CN, COOMe, CONH₂, COMe, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(15) Alkyl mit 1 oder 2 C-Atomen, Cycloalkyl mit 3, 4, 5 oder 6 C-Atomen, CF₃ oder Phenyl,
das unsubstituiert ist oder substituiert mit 1 oder 2 Substituenten ausgewählt aus der Gruppe bestehend aus F, Cl, Br, CF₃, CN, COOMe, CONH₂, COMe, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(10), R(11) und R(12) unabhängig von einander wie R(9) definiert;
R(13) Wasserstoff;
R(2) Wasserstoff, Alkyl mit 1, 2 oder 3 C-Atomen;
R(3) C_{y}H_{2y}-R(16);
y 0, 1, 2, 3 oder 4,
wobei y nicht 0 sein kann, wenn R(16) OR(17) bedeutet;
R(16) Alkyl mit 1, 2 oder 3 C-Atomen, Cycloalkyl mit 3, 4, 5, 6, 7, 8 oder 9 C-Atomen, CF₃, OR(17), Phenyl, Furyl, Thienyl oder ein N-haltiger Heteroaromat mit 3, 4 oder 5 C-Atomen,
wobei Phenyl, Furyl, Thienyl und der N-haltige Heteroaromat unsubstituiert sind oder substituiert mit 1 oder 2 Substituenten ausgewählt aus der Gruppe bestehend aus F, Cl, Br, CF₃, OCF₃, CN, COOMe, CONH₂, COMe, NH₂, OH, Alkyl mit 1, 2 oder 3 C-Atomen, Alkoxy mit 1 oder 2 C-Atomen, Dimethylamino, Sulfamoyl, Methylsulfonyl und Methylsulfonylamino;
R(17) Alkyl mit 1, 2, 3, 4 oder 5 C-Atomen, Cycloalkyl mit 3, 4, 5 oder 6 C-Atomen, CF₃, Phenyl oder 2-, 3- oder 4- Pyridyl,
wobei Phenyl oder 2-, 3- oder 4-Pyridyl unsubstituiert sind oder substituiert mit 1, 2 oder 3 Substituenten ausgewählt aus der Gruppe bestehend aus F, Cl, Br, CF₃, OCF₃, NO₂, CN, COOMe, CONH₂, COMe, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4) Wasserstoff oder Alkyl mit 1 oder 2 C-Atomen;
R(5), R(6), R(7) und R(8) unabhängig voneinander Wasserstoff, F, Cl, Br, CF₃, CN, COOMe, CONH₂, COMe, NH₂, OH, Alkyl mit 1, 2 oder 3 C-Atomen, Alkoxy mit 1 oder 2 C-Atomen, Dimethylamino, Sulfamoyl, Methylsulfonyl oder Methylsulfonylamino;
R(30) und R(31) unabhängig voneinander Wasserstoff oder Methyl;
oder
R(30) und R(31) gemeinsam eine Kette von 2 Methylengruppen;
sowie ihre pharmazeutisch akzeptablen Salze.

5. Verbindungen der Formel I nach einem oder mehreren der Ansprüche 1, 2 oder 4, in der bedeuten:
R(1) C(O)OR(9), SO₂R(10), COR(11) oder C(O)NR(12)R(13);
R(9) CₓH₂ₓ-R(14);
x 0, 1, 2 oder 3;
R(14) Alkyl mit 1, 2, 3 oder 4 C-Atomen, Cycloalkyl mit 3, 4, 5, 6, 7, 8 oder 9 C-Atomen, CF₃, Phenyl oder Pyridyl,
wobei Phenyl und Pyridyl unsubstituiert sind oder substituiert mit 1 oder 2 Substituenten ausgewählt aus der Gruppe bestehend aus F, Cl, CF₃, OCF₃, OH, Alkyl mit 1, 2 oder 3 C-Atomen und Alkoxy mit 1 oder 2 C-Atomen;
R(10), R(11) und R(12) unabhängig von einander wie R(9) definiert;
R(13) Wasserstoff;
R(2) Wasserstoff
R(3) C_{y}H_{2y}-R(16);
y 0, 1 oder 2;
R(16) Alkyl mit 1, 2 oder 3 C-Atomen, Cycloalkyl mit 5 oder 6 C-Atomen, CF₃, Phenyl oder Pyridyl
wobei Phenyl und Pyridyl unsubstituiert sind oder substituiert mit 1 oder 2 Substituenten ausgewählt aus der Gruppe bestehend aus F, Cl, CF₃, OCF₃, OH, Alkyl mit 1, 2 oder 3 C-Atomen und Alkoxy mit 1 oder 2 C-Atomen;
R(4) Wasserstoff;
R(5), R(6), R(7) und R(8) unabhängig voneinander Wasserstoff, F, CF₃, CN, COOMe, CONH₂, NH₂, OH, Alkyl mit 1, 2 oder 3 C-Atomen oder Alkoxy mit 1 oder 2 C-Atomen;
R(30) und R(31) unabhängig voneinander Wasserstoff oder Methyl;
oder
R(30) und R(31) gemeinsam eine Kette von 2 Methylengruppen;
sowie ihre pharmazeutisch akzeptablen Salze.

6. Verbindungen der Formel I nach einem oder mehreren der Ansprüche 1, 2, 4 oder 5, in der bedeuten:
R(1) C(O)OR(9) oder COR(11)
R(9) CₓH₂ₓ-R(14);
x 0, 1, 2 oder 3;
R(14) Cycloalkyl mit 5 oder 6 C-Atomen oder Phenyl,
wobei Phenyl unsubstituiert ist oder substituiert mit 1 oder 2 Substituenten ausgewählt aus der Gruppe bestehend aus F, Cl, CF₃, OCF₃, Alkyl mit 1, 2 oder 3 C-Atomen und Alkoxy mit 1 oder 2 C-Atomen;
R(11) wie R(9) definiert;
R(2) Wasserstoff:
R(3) C_{y}H_{2y}-R(16);
y 0, 1 oder 2;
R(16) Alkyl mit 1, 2 oder 3 C-Atomen, Cycloalkyl mit 5 oder 6 C-Atomen, CF₃, Phenyl oder Pyridyl
wobei Phenyl und Pyridyl unsubstituiert sind oder substituiert mit 1 oder 2 Substituenten ausgewählt aus der Gruppe bestehend aus F, Cl, CF₃, OCF₃, Alkyl mit 1, 2 oder 3 C-Atomen und Alkoxy mit 1 oder 2 C-Atomen;
R(4) Wasserstoff;
R(5), R(6), R(7) und R(8) unabhängig voneinander Wasserstoff, F, CF₃, Alkyl mit 1, 2 oder 3 C-Atomen oder Alkoxy mit 1 oder 2 C-Atomen;
R(30) und R(31) Wasserstoff;
sowie ihre pharmazeutisch akzeptablen Salze.

7. Verbindung der Formel I nach einem oder mehreren der Ansprüche 1, 2, 4, 5 oder 6, die ist: 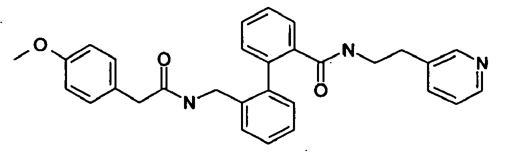 sowie ihre pharmazeutisch akzeptablen Salze.

8. Verbindungen der Formel I nach einem oder mehreren der Ansprüche 1 bis 7 und ihre pharmazeutisch akzeptablen Salze zur Anwendung als Arzneimittel.

9. Pharmazeutische Zubereitung, enthaltend eine wirksame Menge mindestens einer Verbindung der Formel I nach einem oder mehreren der Ansprüche 1 bis 7 und/oder eines pharmazeutisch akzeptablen Salzes davon als Wirkstoff, zusammen mit pharmazeutisch annehmbaren Träger- und Zusatzstoffen und gegebenenfalls noch einem oder mehreren anderen pharmakologischen Wirkstoffen.

10. Verwendung einer Verbindung der Formel I nach einem oder mehreren der Ansprüche 1 bis 7 und/oder eines pharmazeutisch akzeptablen Salzes davon zur Herstellung eines Medikaments mit K⁺-Kanal-blockierender Wirkung zur Therapie und Prophylaxe von K⁺-Kanal mediierten Krankheiten.

11. Verwendung einer Verbindung der Formel I nach einem oder mehreren der Ansprüche 1 bis 7 und/oder eines pharmazeutisch akzeptablen Salzes davon zur Herstellung eines Medikaments zur Therapie oder Prophylaxe von Herzrhythmusstörungen, die durch Aktionspotential-Verlängerung behoben werden können.

12. Verwendung einer Verbindung der Formel I nach einem oder mehreren der Ansprüche 1 bis 7 und/oder eines pharmazeutisch akzeptablen Salzes davon zur Herstellung, eines Medikaments zur Therapie oder Prophylaxe von Reentry-Arrhythmien.

13. Verwendung einer Verbindung der Formel I nach einem oder mehreren der Ansprüche 1 bis 7 und/oder eines pharmazeutisch akzeptablen Salzes davon zur Herstellung eines Medikaments zur Therapie oder Prophylaxe von supraventrikulären Arrhythmien.

14. Verwendung einer Verbindung der Formel I nach einem oder mehreren der Ansprüche 1 bis 7 und/oder eines pharmazeutisch akzeptablen Salzes davon zur Herstellung eines Medikaments zur Therapie oder Prophylaxe von atrialer Fibrillation oder atrialem Flattern.

15. Verwendung einer Verbindung der Formel I nach einem oder mehreren der Ansprüche 1 bis 7 und/oder eines pharmazeutisch akzeptablen Salzes davon zur Herstellung eines Medikamentes zur Terminierung von atrialer Fibrillation oder atrialem Flattern (Kardioversion).

16. Pharmazeutische Zubereitung, enthaltend eine wirksame Menge mindestens einer Verbindung der Formel I nach einem oder mehreren der Ansprüche 1 bis 7 und/oder eines pharmazeutisch akzeptablen Salzes davon sowie eines IKr-Kanalblockers als Wirkstoffe, zusammen mit pharmazeutisch annehmbaren Träger- und Zusatzstoffen.

17. Pharmazeutische Zubereitung, enthaltend eine wirksame Menge mindestens einer Verbindung der Formel I nach einem oder mehreren der Ansprüche 1 bis 7 und/oder eines pharmazeutisch akzeptablen Salzes davon sowie eines IKs-Kanalblockers als Wirkstoffe, zusammen mit pharmazeutisch annehmbaren Träger- und Zusatzstoffen.

18. Pharmazeutische Zubereitung, enthaltend eine wirksame Menge mindestens einer Verbindung der Formel I nach einem oder mehreren der Ansprüche 1 bis 7 und/oder eines pharmazeutisch akzeptablen Salzes davon sowie eines Betablockers als Wirkstoffe, zusammen mit pharmazeutisch annehmbaren Träger- und Zusatzstoffen.

## Claims

1. A compound of the formula I, 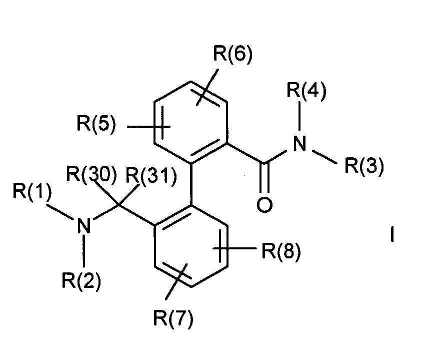 in which:
R(1) is C(O)OR(9), SO₂R(10), COR(11), C(O)NR(12)R(13) or C(S)NR(12)R(13);
R(9) is CₓH₂ₓ-R(14);
x is 0, 1, 2, 3 or 4,
where x cannot be 0 if R(14) is OR(15) or SO₂Me;
R(14) is alkyl having 1, 2, 3, 4, 5 or 6 carbon atoms, cycloalkyl having 3, 4, 5, 6, 7, 8, 9, 10 or 11 carbon atoms, CF₃, C₂F₅, C₃F₇, CH₂F, CHF₂, OR(15), SO₂Me, phenyl, naphthyl, biphenylyl, furyl, thienyl or an N-containing heteroaromatic having 1, 2, 3, 4, 5, 6, 7, 8 or 9 carbon atoms,
where phenyl, naphthyl, biphenylyl, furyl, thienyl and the N-containing hetero-aromatic are unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(15) is alkyl having 1, 2, 3, 4 or 5 carbon atoms, cycloalkyl having 3, 4, 5 or 6 carbon atoms, CF₃ or phenyl
which is unsubstituted or substituted by 1, 2 or 3 substituents selected from the group consisting of F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(10), R(11) and R(12) independently of one another are defined as R(9);
R(13) is hydrogen, alkyl having 1, 2, 3 or 4 carbon atoms or CF₃;
R(2) is hydrogen, alkyl having 1, 2, 3 or 4 carbon atoms or CF₃;
R(3) is CyH₂y-R(16);
y is 0, 1, 2, 3 or 4,
where y cannot be 0 if R(16) is OR(17) or SO₂Me;
R(16) is alkyl having 1, 2, 3, 4, 5 or 6 carbon atoms, cycloalkyl having 3, 4, 5, 6, 7, 8, 9, 10 or 11 carbon atoms, CF₃, C₂F₅, C₃F₇, CH₂F, CHF₂, OR(17), SO₂Me, phenyl, naphthyl, furyl, thienyl or an N-containing heteroaromatic having 1, 2, 3, 4, 5, 6, 7, 8 or 9 carbon atoms,
where phenyl, naphthyl, furyl, thienyl and the N-containing heteroaromatic are unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(17) is hydrogen, alkyl having 1, 2, 3, 4 or 5 carbon atoms, cycloalkyl having 3, 4, 5 or 6 carbon atoms, CF₃, phenyl or 2-, 3- or 4-pyridyl,
where phenyl or 2-, 3- or 4-pyridyl are unsubstituted or substituted by 1, 2 or 3 substituents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
or
R(3) is CHR(18)R(19);
R(18) is hydrogen or C_{z}H_{2z}-R(16), where R(16) is defined as indicated above;
z is 0, 1, 2 or 3;
R(19) is COOH, CONH₂, CONR(20)R(21), COOR(22), CH₂OH;
R(20) is hydrogen, alkyl having 1, 2, 3, 4 or 5 carbon atoms, CᵥH₂ᵥ-CF₃ or C_{w}H_{2w}-phenyl,
where the phenyl ring is unsubstituted or substituted by 1, 2 or 3 substitutents selected from the group consisting of F, Cl, Br, I, CF₃, OCF₃, NO₂, CN, COOMe, CONH₂, COMe, NH₂, OH, alkyl having 1, 2,
3 or 4 carbon atoms, alkoxy having 1, 2, 3 or 4 carbon atoms, dimethylamino, sulfamoyl, methylsulfonyl and methylsulfonylamino;
v is 0, 1, 2 or 3;
w is 0, 1, 2 or 3;
R(21) is hydrogen or alkyl having 1, 2, 3, 4 or 5 carbon atoms;
R(22) is alkyl having 1, 2, 3, 4 or 5 carbon atoms;
R(4) is hydrogen, alkyl having 1, 2, 3, 4, 5 or 6 carbon atoms or CF₃;
or
R(3) and R(4) together are a chain of 4 or 5 methylene groups, of which one methylene group can be replaced by -O-, -S-, -NH-, -N(methyl)- or -N(benzyl)-;
R(5), R(6), R(7) and R(8) independently of one another are hydrogen, F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl or methylsulfonylamino;
R(30) and R(31) independently of one another are hydrogen or alkyl having 1, 2 or 3 carbon atoms;
or
R(30) and R(31) together are a chain of 2 methylene groups;
or its pharmaceutically acceptable salts.

2. A compound of the formula I as claimed in claim 1, in which
R(1) is C(O)OR(9), SO₂R(10), COR(11) or C(O)NR(12)R(13);
R(9) is CₓH₂ₓ-R(14);
x is 0, 1, 2, 3 or 4,
where x cannot be 0 if R(14) is OR(15);
R(14) is alkyl having 1, 2, 3 or 4 carbon atoms, cycloalkyl having 3, 4, 5, 6, 7, 8 or 9 carbon atoms, CF₃, C₂F₅, OR(15), phenyl, furyl, thienyl or an N-containing heteroaromatic having 1, 2, 3, 4, 5, 6, 7, 8 or 9 carbon atoms,
where phenyl, furyl, thienyl and the N-containing heteroaromatic are unsubstituted or substituted by 1, 2 or 3 substituents selected from the group consisting of F, Cl, Br, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl, and methylsulfonylamino;
R(15) is alkyl having 1, 2, 3, 4 or 5 carbon atoms, cycloalkyl having 3, 4, 5 or 6 carbon atoms, CF₃ or phenyl,
which is unsubstituted or substituted by 1, 2 or 3 substituents selected from the group consisting of F, Cl, Br, CF₃, NO₂, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(10), R(11) and R(12) independently of one another are defined as R(9);
R(13) is hydrogen, alkyl having 1, 2, 3 or 4 carbon atoms or CF₃;
R(2) is hydrogen, alkyl having 1, 2, 3 or 4 carbon atoms or CF₃;
R(3) is C_{y}H_{2y}-R(16);
y is 0, 1, 2, 3 or 4,
where y cannot be 0 if R(16) is OR(17);
R(16) is alkyl having 1, 2, 3 or 4 carbon atoms, cycloalkyl having 3, 4, 5, 6, 7, 8 or 9 carbon atoms, CF₃, C₂F₅, OR(17), phenyl, furyl, thienyl or an N-containing heteroaromatic having 1, 2, 3, 4, 5, 6, 7, 8 or 9 carbon atoms,
where phenyl, furyl, thienyl and the N-containing heteroaromatic are unsubstituted or substituted by 1, 2 or 3 substituents selected from the group consisting of F, Cl, Br, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(17) is alkyl having 1, 2, 3, 4 or 5 carbon atoms, cycloalkyl having 3, 4, 5 or 6 carbon atoms, CF₃, phenyl or 2-, 3-, or 4-pyridyl,
where phenyl or 2-, 3- or 4-pyridyl are unsubstituted or substituted by 1, 2 or 3 substituents selected from the group consisting of F, Cl, Br, CF₃, OCF₃, NO₂, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
or
R(3) is CHR(18)R(19);
R(18) is hydrogen or C_{z}H_{2z}-R(16), where R(16) is defined as indicated in claim 1 above;
z is 0, 1, 2 or 3;
R(19) is CONH₂, CONR(20)R(21), COOR(22), CH₂OH;
R(20) is hydrogen, alkyl having 1, 2, 3, 4 or 5 carbon atoms, CᵥH₂ᵥ-CF₃ or C_{w}H_{2w}-phenyl,
where the phenyl ring is unsubstituted or substituted by 1, 2 or 3 substituents selected from the group consisting of F, Cl, Br, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
v is 0, 1, 2 or 3;
w is 0, 1, 2 or 3;
R(21) is hydrogen or alkyl having 1, 2, 3, 4 or 5 carbon atoms;
R(22) is alkyl having 1, 2, 3, 4 or 5 carbon atoms;
R(4) is hydrogen or alkyl having 1, 2, 3, 4, 5 or 6 carbon atoms or CF₃;
R(5), R(6), R(7) and R(8) independently of one another are hydrogen, F, Cl, Br, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl or methylsulfonylamino;
R(30) and R(31) independently of one another are hydrogen or alkyl having 1, 2 or 3 carbon atoms;
or
R(30) and R(31) together are a chain of 2 methylene groups;
or its pharmaceutically acceptable salts.

3. A compound of the formula I as claimed in claims 1 or 2, in which:
R(1) is C(O)OR(9), SO₂R(10), COR(11) or C(O)NR(12)R(13);
R(9) is CₓH₂ₓ-R(14);
x is 0, 1, 2, 3 or 4,
where x cannot be 0 if R(14) is OR(15);
R(14) is cycloalkyl having 3, 4, 5, 6, 7, 8 or 9 carbon atoms, CF₃, OR(15), phenyl, furyl, thienyl or an N-containing heteroaromatic having 3, 4 or 5 carbon atoms,
where phenyl, furyl, thienyl and the N-containing heteroaromatic are unsubstituted or substituted by 1 or 2 substituents selected from the group consisting of F, Cl, Br, CF₃, OCF₃, CN, COOMe, CONH₂, COMe, OH, alkyl having 1, 2 or 3 carbon atoms, alkoxy having 1 or 2 carbon atoms, dimethylamino, sulfamoyl, methylsulfonyl and methylsulfonylamino;
R(15) is alkyl having 1 or 2 carbon atoms, cycloalkyl having 3, 4, 5 or 6 carbon atoms, CF₃ or phenyl,
which is unsubstituted or substituted by 1 or 2 substituents selected from the group consisting of F, Cl, Br, CF₃, CN, COOMe, CONH₂, COMe, OH, alkyl having 1, 2 or 3 carbon atoms, alkoxy having 1 or 2 carbon atoms, dimethylamino, sulfamoyl, methylsulfonyl and methylsulfonylamino;
R(10), R(11) and R(12) independently of one another are defined as R(9);
R(13) is hydrogen;
R(2) is hydrogen or alkyl having 1, 2 or 3 carbon atoms;
R(3) is CHR(18)R(19);
R(18) is hydrogen or C_{z}H_{2z}-R(16);
z is 0, 1, 2 or 3;
R(19) is CONH₂, CONR(20)R(21), COOR(22) or CH₂OH;
R(20) is hydrogen, alkyl having 1, 2, 3, 4 or 5 carbon atoms, CᵥH₂ᵥ-CF₃ or CH₂H_{2w}-phenyl,
where the phenyl ring is unsubstituted or substituted by 1, 2 or 3 substituents selected from the group consisting of F, Cl, Br, CF₃, OCF₃, CN, COOMe, CONH₂, COMe, OH, alkyl having 1, 2 or 3 carbon atoms, alkoxy having 1 or 2 carbon atoms, dimethylamino, sulfamoyl, methylsulfonyl and methylsulfonylamino;
v is 0, 1, 2 or 3;
w is 0, 1, 2 or 3;
R(21) is hydrogen or alkyl having 1, 2, 3, 4 or 5 carbon atoms;
R(22) is alkyl having 1, 2, 3, 4 or 5 carbon atoms;
R(16) is alkyl having 1, 2 or 3 carbon atoms, cycloalkyl having 3, 4, 5, 6, 7, 8 or 9 carbon atoms, CF₃, OR(17), phenyl, furyl, thienyl or an N-containing heteroaromatic having 3, 4 or 5 carbon atoms,
where phenyl, furyl, thienyl and the N-containing heteroaromatic are unsubstituted or substituted by 1 or 2 substituents selected from the group consisting of F, Cl, Br, CF₃, OCF₃, CN, COOMe, CONH₂, COMe, NH₂, OH, alkyl having 1, 2 or 3 carbon atoms, alkoxy having 1 or 2 carbon atoms, dimethylamino, sulfamoyl, methylsulfonyl and methylsulfonylamino;
R(17) is alkyl having 1, 2, 3 or 4 carbon atoms, cycloalkyl having 3, 4, 5 or 6 carbon atoms, CF₃, phenyl or 2-, 3- or 4-pyridyl,
where phenyl or 2-, 3- or 4-pyridyl are unsubstituted or substituted by 1, 2 or 3 substituents selected from the group consisting of F, Cl, Br, CF₃, OCF₃, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4) is hydrogen or alkyl having 1 or 2 carbon atoms;
R(5), R(6), R(7) and R(8) independently of one another are hydrogen, F, Cl, Br, CF₃, CN, COOMe, CONH₂, COMe, NH₂, OH, alkyl having 1, 2 or 3 carbon atoms, alkoxy having 1 or 2 carbon atoms, dimethylamino, sulfamoyl, methylsulfonyl or methylsulfonylamino;
R(30) and R(31) independently of one another are hydrogen or methyl:
or
R(30) and R(31) together are a chain of 2 methylene groups;
or its pharmaceutically acceptable salts.

4. A compound of the formula I as claimed in claims 1 or 2, in which:
R(1) is C(O)OR(9), SO₂R(10), COR(11) or C(O)NR(12)R(13);
R(9) is CₓH₂ₓ-R(14);
x is 0, 1, 2, 3 or 4,
where x cannot be 0 if R(14) is OR(15);
R(14) is alkyl having 1, 2, 3 or 4 carbon atoms, cycloalkyl having 3, 4, 5, 6, 7, 8 or 9 carbon atoms, CF₃, OR(15), phenyl, furyl, thienyl or an N-containing heteroaromatic having 3, 4 or 5 carbon atoms,
where phenyl, furyl, thienyl and the N-containing heteroaromatic are unsubstituted or substituted by 1 or 2 substituents selected from the group consisting of F, Cl, Br, CF₃, OCF₃, CN, COOMe, CONH₂, COMe, OH, alkyl having 1, 2 or 3 carbon atoms, alkoxy having 1 or 2 carbon atoms, dimethylamino, sulfamoyl, methylsulfonyl and methylsulfonylamino;
R(15) is alkyl having 1 or 2 carbon atoms, cycloalkyl having 3, 4, 5 or 6 carbon atoms, CF₃ or phenyl,
which is unsubstituted or substituted by 1 or 2 substituents selected from the group consisting of F, Cl, Br, CF₃, CN, COOMe, CONH₂, COMe, OH, alkyl having 1, 2 or 3 carbon atoms, alkoxy having 1 or 2 carbon atoms, dimethylamino, sulfamoyl, methylsulfonyl and methylsulfonylamino;
R(10), R(11) and R(12) independently of one another are defined as R(9);
R(13) is hydrogen;
R(2) is hydrogen or alkyl having 1, 2 or 3 carbon atoms;
R(3) is C_{y}H_{2y}-R(16);
y is 0, 1, 2, 3 or 4,
where y cannot be 0 if R(16) is OR(17);
R(16) is alkyl having 1, 2 or 3 carbon atoms, cycloalkyl having 3, 4, 5, 6, 7, 8 or 9 carbon atoms, CF₃, OR(17), phenyl, furyl, thienyl or an N-containing heteroaromatic having 3, 4 or 5 carbon atoms,
where phenyl, furyl, thienyl and the N-containing heteroaromatic are unsubstituted or substituted by 1 or 2 substituents selected from the group consisting of F, Cl, Br, CF₃, OCF₃, CN, COOMe, CONH₂, COMe, NH₂, OH, alkyl having 1, 2 or 3 carbon atoms, alkoxy having 1 or 2 carbon atoms, dimethylamino, sulfamoyl, methylsulfonyl and methylsulfonylamino;
R(17) is alkyl having 1, 2, 3, 4 or 5 carbon atoms, cycloalkyl having 3, 4, 5 or 6 carbon atoms, CF₃, phenyl or 2-, 3- or 4-pyridyl,
where phenyl or 2-, 3- or 4-pyridyl are unsubstituted or substituted by 1, 2 or 3 substituents selected from the group consisting of F, Cl, Br, CF₃, OCF₃, NO₂, CN, COOMe, CONH₂, COMe, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4) is hydrogen or alkyl having 1 or 2 carbon atoms;
R(5), R(6), R(7) and R(8) independently of one another are hydrogen, F, Cl, Br, CF₃, CN, COOMe, CONH₂, COMe, NH₂, OH, alkyl having 1, 2 or 3 carbon atoms, alkoxy having 1 or 2 carbon atoms, dimethylamino, sulfamoyl, methylsulfonyl or methylsulfonylamino;
R(30) and R(31) independently of one another are hydrogen or methyl;
or
R(30) and R(31) together are a chain of 2 methylene groups;
or its pharmaceutically acceptable salts.

5. A compound of the formula I as claimed in one or more of claims 1, 2 or 4, in which:
R(1) is C(O)OR(9), SO₂R(10) COR(11) or C(O)NR(12)R(13);
R(9) is CₓH₂ₓ-R(14);
x is 0, 1, 2 or 3;
R(14) is alkyl having 1, 2, 3 or 4 carbon atoms, cycloalkyl having 3, 4, 5, 6, 7, 8 or 9 carbon atoms, CF₃, phenyl or pyridyl,
where phenyl and pyridyl are unsubstituted or substituted by 1 or 2 substituents selected from the group consisting of F, Cl, CF₃, OCF₃, OH, alkyl having 1, 2 or 3 carbon atoms and alkoxy having 1 or 2 carbon atoms;
R(10), R(11) and R(12) independently of one another are defined as R(9);
R(13) is hydrogen;
R(2) is hydrogen;
R(3) is CyH₂y-R(16);
y is 0, 1 or 2;
R(16) is alkyl having 1, 2 or 3 carbon atoms, cycloalkyl having 5 or 6 carbon atoms, CF₃, phenyl or pyridyl,
where phenyl and pyridyl are unsubstituted or substituted by 1 or 2 substituents selected from the group consisting of F, Cl, CF₃, OCF₃, OH, alkyl having 1, 2 or 3 carbon atoms and alkoxy having 1 or 2 carbon atoms;
R(4) is hydrogen;
R(5), R(6), R(7) and R(8) independently of one another are hydrogen, F, CF₃, CN, COOMe, CONH₂, NH₂, OH, alkyl having 1, 2 or 3 carbon atoms or alkoxy having 1 or 2 carbon atoms;
R(30) and R(31) independently of one another are hydrogen or methyl;
or
R(30) and R(31) together are a chain of 2 methylene groups;
or its pharmaceutically acceptable salts.

6. A compound of the formula I as claimed in one or more of claims 1, 2, 4 or 5, in which:
R(1) is C(O)OR(9) or COR(11);
R(9) is CₓH₂ₓ-R(14);
x is 0, 1, 2 or 3;
R(14) is cycloalkyl having 5 or 6 carbon atoms or phenyl,
where phenyl is unsubstituted or substituted by 1 or 2 substituents selected from the group consisting of F, Cl, CF₃, OCF₃, alkyl having 1, 2 or 3 carbon atoms and alkoxy having 1 or 2 carbon atoms;
R(11) is defined as R(9);
R(2) is hydrogen;
R(3) is C_{y}H_{2y}-R(16);
y is 0, 1 or 2;
R(16) is alkyl having 1, 2 or 3 carbon atoms, cycloalkyl having 5 or 6 carbon atoms, CF₃, phenyl or pyridyl
where phenyl and pyridyl are unsubstituted or substituted by 1 or 2 substituents selected from the group consisting of F, Cl, CF₃, OCF₃, alkyl having 1, 2 or 3 carbon atoms and alkoxy having 1 or 2 carbon atoms;
R(4) is hydrogen;
R(5), R(6), R(7) and R(8) independently of one another are hydrogen, F, CF₃, alkyl having 1, 2 or 3 carbon atoms or alkoxy having 1 or 2 carbon atoms;
R(30) and R(31) are hydrogen;
or its pharmaceutically acceptable salts.

7. A compound of the formula I as claimed in one or more of claims 1, 2, 4 5 or 6, which is: 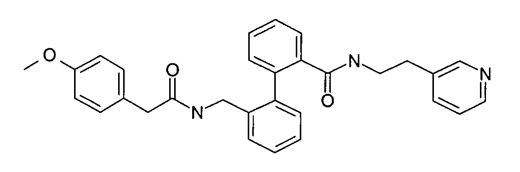 or its pharmaceutically acceptable salts.

8. A compound of the formula I as claimed in one or more of claims 1 to 7, and its pharmaceutically acceptable salts for use as a pharmaceutical.

9. A pharmaceutical preparation, comprising an efficacious amount of at least one compound of the formula I as claimed in one or more of claims 1 to 7 and/or a pharmaceutically acceptable salt thereof as active compound, together with pharmaceutically acceptable vehicles and additives and, if appropriate, additionally one or more other pharmacological active compounds.

10. The use of a compound of the formula I as claimed in one or more of claims 1 to 7 and/or of a pharmaceutically acceptable salt thereof for the production of a medicament having K⁺ channel-blocking action for the therapy and prophylaxis of K⁺ channel-mediated illnesses.

11. The use of a compound of the formula I as claimed in one or more of claims 1 to 7 and/or of a pharmaceutically acceptable salt thereof for the production of a medicament for the therapy or prophylaxis of cardiac arrythmias which can be eliminated by action potential prolongation.

12. The use of a compound of the formula I as claimed in one or more of claims 1 to 7 and/or of a pharmaceutically acceptable salt thereof for the production of a medicament for the therapy or prophylaxis of re-entry arrythmias.

13. The use of a compound of the formula I as claimed in one or more of claims 1 to 7 and/or of a pharmaceutically acceptable salt thereof for the production of a medicament for the therapy or prophylaxis of supraventricular arrythmias.

14. The use of a compound of the formula I as claimed in one or more of claims 1 to 7 and/or of a pharmaceutically acceptable salt thereof for the production of a medicament for the therapy or prophylaxis of atrial fibrillation or atrial flutter.

15. The use of a compound of the formula I as claimed in one or more of claims 1 to 7 and/or of a pharmaceutically acceptable salt thereof for the production of a medicament for the termination of atrial fibrillation or atrial flutters (cardioversion).

16. A pharmaceutical preparation comprising an efficacious amount of at least one compound of the formula I as claimed in one or more of claims 1 to 7 and/or of a pharmaceutically acceptable salt thereof and of an IKr channel blocker as active compounds, together with pharmaceutically acceptable vehicles and additives.

17. A pharmaceutical preparation comprising an efficacious amount of at least one compound of the formula I as claimed in one or more of claims 1 to 7 and/or of a pharmaceutically acceptable salt thereof and of an IKs channel blocker as active compounds, together with pharmaceutically acceptable vehicles and additives.

18. A pharmaceutical preparation comprising an efficacious amount of at least one compound of the formula I as claimed in one or more of claims 1 to 7 and/or of a pharmaceutically acceptable salt thereof and of a beta-blocker as active compounds, together with pharmaceutically acceptable vehicles and additives.

## Revendications

1. Composés de formule I, 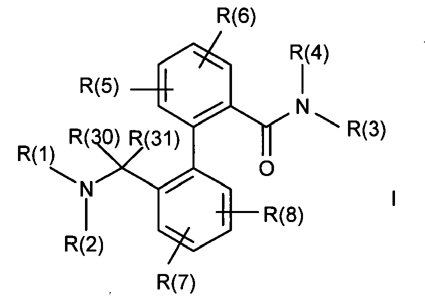 dans laquelle :
R(1) représente un C(O)OR(9), un SO₂R(10), un COR(11), un C(O)NR(12)R(13) ou un C(S)NR(12)R(13) ;
R(9) représente un CₓH₂ₓ-R(14) ;
x vaut 0, 1, 2, 3 ou 4,
où x ne peut pas valoir 0 si R(14) représente un OR(15) ou un SO₂Me ;
R(14) représente un alkyle renfermant 1, 2, 3, 4, 5 ou 6 atomes de carbone, un cycloalkyle renfermant 3, 4, 5, 6, 7, 8, 9, 10 ou 11 atomes de carbone, un CF₃, un C₂F₅, un C₃F₇, un CH₂F, un CHF₂, un OR(15), un SO₂Me, un phényle, un naphtyle, un biphénylyle, un furyle, un thiényle ou un groupe hétéroaromatique azoté renfermant 1, 2, 3, 4, 5, 6, 7, 8 ou 9 atomes de carbone,
où le phényle, le naphtyle, le biphénylyle, le furyle, le thiényle et le groupe hétéroaromatique azoté sont non substitués ou substitués par 1, 2 ou 3 substituants choisis parmi le groupe constitué d'un F, d'un Cl, d'un Br, d'un I, d'un CF₃, d'un OCF₃, d'un NO₂, d'un CN, d'un COOMe, d'un CONH₂, d'un COMe, d'un NH₂, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
R(15) représente un alkyle renfermant 1, 2, 3, 4 ou 5 atomes de carbone, un cycloalkyle renfermant 3, 4, 5 ou 6 atomes de carbone, un CF₃ ou un phényle,
qui est non substitué ou substitué par 1, 2
ou 3 substituants choisis parmi le groupe constitué d'un F, d'un Cl, d'un Br, d'un I, d'un CF₃, d'un NO₂, d'un CN, d'un COOMe, d'un CONH₂, d'un COMe, d'un NH₂, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
R(10), R(11) et R(12), indépendamment les uns des autres, sont tels que définis pour R(9);
R(13) représente un hydrogène, un alkyle renfermant 1, 2, 3 ou 4 atomes de carbone ou un CF₃;
R(2) représente un hydrogène, un alkyle renfermant 1, 2, 3 ou 4 atomes de carbone ou un CF₃;
R(3) représente un C_{y}H_{2y}-R(16) ;
y vaut 0, 1, 2, 3 ou 4,
où y ne peut pas valoir 0 si R(16) représente un OR(17) ou un SO₂Me;
R(16) représente un alkyle renfermant 1, 2, 3, 4, 5 ou 6 atomes de carbone, un cycloalkyle renfermant 3, 4, 5, 6, 7, 8, 9, 10 ou 11 atomes de carbone, un CF₃, un C₂F₅, un C₃F₇, un CH₂F, un CHF₂, un OR(17), un SO₂Me, un phényle, un naphtyle, un furyle, un thiényle ou un groupe hétéroaromatique azoté renfermant 1, 2, 3, 4, 5, 6, 7, 8 ou 9 atomes de carbone,
où le phényle, le naphtyle, le furyle, le thiényle et le groupe hétéroaromatique azoté sont non substitués ou substitués par 1, 2 ou 3 substituants choisis parmi le groupe constitué d'un F, d'un Cl, d'un Br, d'un I, d'un CF₃, d'un OCF₃, d'un NO₂, d'un CN, d'un COOMe, d'un CONH₂, d'un COMe, d'un NH₂, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
R(17) représente un hydrogène, un alkyle renfermant 1, 2, 3, 4 ou 5 atomes de carbone, un cycloalkyle renfermant 3, 4, 5 ou 6 atomes de carbone, un CF₃, un phényle ou un 2-, un 3- ou 4-pyridyle,
où le phényle ou le 2-, le 3- ou le 4-pyridyle sont non substitués ou substitués par 1, 2 ou 3 substituants choisis parmi le groupe constitué d'un F, d'un Cl, d'un Br, d'un I, d'un CF₃, d'un OCF₃, d'un NO₂, d'un CN, d'un COOMe, d'un CONH₂, d'un COMe, d'un NH₂, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
ou
R(3) représente un CHR(18)R(19) ;
R(18) représente un hydrogène ou un C_{z}H_{2z}-R(16), dans lequel R(16) est défini comme indiqué ci-dessus ;
z vaut 0, 1, 2 ou 3 ;
R(19) représente un COOH, un CONH₂, un CONR(20)R(21), un COOR(22), un CH₂OH ;
R(20) représente un hydrogène, un alkyle renfermant 1, 2, 3, 4 ou 5 atomes de carbone, un CᵥH₂ᵥ-CF₃ ou un C_{w}H_{2w}-phényle,
dans lequel le noyau phényle est non substitué ou substitué par 1, 2 ou 3 substituants choisis parmi le groupe constitué d'un F, d'un Cl, d'un Br, d'un I, d'un CF₃, d'un OCF₃, d'un NO₂, d'un CN, d'un COOMe, d'un CONH₂, d'un COMe, d'un NH₂, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
v vaut 0, 1, 2 ou 3 ;
w vaut 0, 1, 2 ou 3 ;
R(21) représente un hydrogène ou un alkyle renfermant 1, 2, 3, 4 ou 5 atomes de carbone ;
R(22) représente un alkyle renfermant 1, 2, 3, 4 ou 5 atomes de carbone ;
R(4) représente un hydrogène, un alkyle renfermant 1, 2, 3, 4, 5 ou 6 atomes de carbone ou un CF₃ ;
ou
R(3) et R(4) représentent conjointement une chaîne de 4 ou 5 groupes méthylène, parmi lesquels un groupe méthylène peut être remplacé par un -O-, un -S-, un -NH-, un -N(méthyle)- ou un -N(benzyle)-;
R(5), R(6), R(7) et R(8) représentent, indépendamment les uns des autres, un hydrogène, un F, un Cl, un Br, un I, un CF₃, un NO₂, un CN, un COOMe, un CONH₂, un COMe, un NH₂, un OH, un alkyle renfermant 1, 2, 3 ou 4 atomes de carbone, un alcoxy renfermant 1, 2, 3 ou 4 atomes de carbone, un diméthylamino, un sulfamoyle, un méthylsulfonyle ou un méthylsulfonylamino ;
R(30) et R(31) représentent, indépendamment l'un de l'autre, un hydrogène ou un alkyle renfermant 1, 2 ou 3 atomes de carbone ;
ou
R(30) et R(31) représentent conjointement une chaîne de 2 groupes méthylène ;
ainsi que leurs sels pharmaceutiquement acceptables.

2. Composés de formule I selon la revendication 1, dans laquelle :
R(1) représente un C(O)OR(9), un SO₂R(10), un COR(11) ou un C(O)NR(12)R(13) ;
R(9) représente un CₓH₂ₓ-R(14) ;
x vaut 0, 1, 2, 3 ou 4,
où x ne peut pas valoir 0 si R(14) représente un OR(15) ;
R(14) représente un alkyle renfermant 1, 2, 3 ou 4 atomes de carbone, un cycloalkyle renfermant 3, 4, 5, 6, 7, 8 ou 9 atomes de carbone, un CF₃, un C₂F₅, un OR(15), un phényle, un furyle, un thiényle ou un groupe hétéroaromatique azoté renfermant 1, 2, 3, 4, 5, 6, 7, 8 ou 9 atomes de carbone,
où le phényle, le furyle, le thiényle et le groupe hétéroaromatique azoté sont non substitués ou substitués par 1, 2 ou 3 substituants choisis parmi le groupe constitué d'un F, d'un Cl, d'un Br, d'un CF₃, d'un OCF₃, d'un NO₂, d'un CN, d'un COOMe, d'un CONH₂, d'un COMe, d'un NH₂, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
R(15) représente un alkyle renfermant 1, 2, 3, 4 ou 5 atomes de carbone, un cycloalkyle renfermant 3, 4, 5 ou 6 atomes de carbone, un CF₃ ou un phényle,
qui est non substitué ou substitué par 1, 2
ou 3 substituants choisis parmi le groupe constitué d'un F, d'un Cl, d'un Br, d'un CF₃, d'un NO₂, d'un CN, d'un COOMe, d'un CONH₂, d'un COMe, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
R(10), R(11) et R(12) indépendamment les uns des autres, sont tels que définis pour R(9) ;
R(13) représente un hydrogène, un alkyle renfermant 1, 2, 3 ou 4 atomes de carbone ou un CF₃ ;
R(2) représente un hydrogène, un alkyle renfermant 1, 2, 3 ou 4 atomes de carbone ou un CF₃ ;
R(3) représente un C_{y}H_{2y}-R(16) ;
y vaut 0, 1, 2, 3 ou 4,
où y ne peut pas valoir 0 si R(16) représente un OR(17) ;
R(16) représente un alkyle renfermant 1, 2, 3 ou 4 atomes de carbone, un cycloalkyle renfermant 3, 4, 5, 6, 7, 8 ou 9 atomes de carbone, un CF₃, un C₂F₅, un OR(17), un phényle, un furyle, un thiényle ou un groupe hétéroaromatique azoté renfermant 1, 2, 3, 4, 5, 6, 7, 8 ou 9 atomes de carbone,
où le phényle, le furyle, le thiényle et le groupe hétéroaromatique azoté 1, 2 ou 3 sont non substitués ou substitués par 1, 2 ou 3 substituants choisis parmi le groupe constitué d'un F, d'un Cl, d'un Br, d'un CF₃, d'un OCF₃, d'un NO₂, d'un CN, d'un COOMe, d'un CONH₂, d'un COMe, d'un NH₂, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
R(17) représente un alkyle renfermant 1, 2, 3, 4 ou 5 atomes de carbone, un cycloalkyle renfermant 3, 4, 5 ou 6 atomes de carbone, un CF₃, un phényle ou un 2-, un 3- ou 4-pyridyle,
où le phényle ou le 2-, le 3- ou le 4-pyridyle sont non substitués ou substitués par 1, 2 ou 3 substituants choisis parmi le groupe constitué d'un F, d'un Cl, d'un Br, d'un CF₃, d'un OCF₃, d'un NO₂, d'un CN, d'un COOMe, d'un CONH₂, d'un COMe, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
ou
R(3) représente un CHR(18)R(19) ;
R(18) représente un hydrogène ou un C_{z}H_{2z}-R(16), dans lequel R(16) est défini comme indiqué ci-dessus dans la revendication 1 ;
z vaut 0, 1, 2 ou 3 ;
R(19) représente un CONH₂, un CONR(20)R(21), un COOR(22), un CH₂OH ;
R(20) représente un hydrogène, un alkyle renfermant 1, 2, 3, 4 ou 5 atomes de carbone, un CᵥH₂ᵥ-CF₃ ou un C_{w}H_{2w}-phényle,
dans lequel le noyau phényle est non substitué ou substitué par 1, 2 ou 3 substituants choisis parmi le groupe constitué d'un F, d'un Cl, d'un Br, d'un CF₃, d'un OCF₃, d'un NO₂, d'un CN, d'un COOMe, d'un CONH₂, d'un COMe, d'un NH₂, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
v vaut 0, 1, 2 ou 3 ;
w vaut 0, 1, 2 ou 3 ;
R(21) représente un hydrogène ou un alkyle renfermant 1, 2, 3, 4 ou 5 atomes de carbone ;
R(22) représente un alkyle renfermant 1, 2, 3, 4 ou 5 atomes de carbone ;
R(4) représente un hydrogène, un alkyle renfermant 1, 2, 3, 4, 5 ou 6 atomes de carbone ou un CF₃ ;
R(5), R(6), R(7) et R(8) représentent, indépendamment les uns des autres, un hydrogène, un F, un Cl, un Br, un CF₃, un NO₂, un CN, un COOMe, un CONH₂, un COMe, un NH₂, un OH, un alkyle renfermant 1, 2, 3 ou 4 atomes de carbone, un alcoxy renfermant 1, 2, 3 ou 4 atomes de carbone, un diméthylamino, un sulfamoyle, un méthylsulfonyle ou un méthylsulfonylamino ;
R(30) et R(31) représentent, indépendamment l'un de l'autre, un hydrogène ou un alkyle renfermant 1, 2 ou 3 atomes de carbone ;
ou
R(30) et R(31) représentent conjointement une chaîne de 2 groupes méthylène ;
ainsi que leurs sels pharmaceutiquement acceptables.

3. Composés de formule I selon les revendications 1 ou 2, dans laquelle :
R(1) représente un C(O)OR(9), un SO₂R(10), un COR(11) ou un C(O)NR(12)R(13) ;
R(9) représente un CₓH₂ₓ-R(14) ;
x vaut 0, 1, 2, 3 ou 4,
où x ne peut pas valoir 0 si R(14) représente un OR(15);
R(14) représente un cycloalkyle renfermant 3, 4, 5, 6, 7, 8 ou 9 atomes de carbone, un CF₃, un OR(15), un phényle, un furyle, un thiényle ou un un groupe hétéroaromatique renfermant 3, 4 ou 5 atomes de carbone,
où le phényle, le furyle, le thiényle et le groupe hétéroaromatique azoté sont non substitués ou substitués par 1 ou 2 substituants choisis parmi le groupe constitué d'un F, d'un Cl, d'un Br, d'un CF₃, d'un OCF₃, d'un CN, d'un COOMe, d'un CONH₂, d'un COMe, d'un OH, d'un alkyle renfermant 1, 2 ou 3 atomes de carbone, d'un alcoxy renfermant 1 ou 2 atomes de carbone, d'un diméthylamino, d'un sulfamoyle, d'un méthylsulfonyle et d'un méthylsulfonylamino ;
R(15) représente un alkyle renfermant 1 ou 2 atomes de carbone, un cycloalkyle renfermant 3, 4, 5 ou 6 atomes de carbone, un CF₃ ou un phényle,
qui est non substitué ou substitué par 1 ou 2 substituants choisis parmi le groupe constitué d'un F, d'un Cl, d'un Br, d'un CF₃, d'un CN, d'un COOMe, d'un CONH₂, d'un COMe, d'un OH, d'un alkyle renfermant 1, 2 ou 3 atomes de carbone, d'un alcoxy renfermant 1 ou 2 atomes de carbone, d'un diméthylamino, d'un sulfamoyle, d'un méthylsulfonyle et d'un méthylsulfonylamino ;
R(10), R(11) et R(12) indépendamment les uns des autres, sont tels que définis pour R(9) ;
R(13) représente un hydrogène ;
R(2) représente un hydrogène ou un alkyle renfermant 1, 2 ou 3 atomes de carbone ;
R(3) représente un CHR(18)R(19) ;
R(18) représente un hydrogène ou un C_{z}H_{2z}-R(16) ;
z vaut 0, 1, 2 ou 3 ;
R(19) représente un CONH₂, un CONR(20)R(21), un COOR(22) ou un CH₂OH ;
R(20) représente un hydrogène, un alkyle renfermant 1, 2, 3, 4 ou 5 atomes de carbone, un CᵥH₂ᵥ-CF₃ ou un C_{w}H_{2w}-phényle,
dans lequel le noyau phényle est non substitué ou substitué par 1, 2 ou 3 substituants choisis parmi le groupe constitué d'un F, d'un Cl, d'un Br, d'un CF₃, d'un OCF₃, d'un CN, d'un COOMe, d'un CONH₂, d'un COMe, d'un OH, d'un alkyle renfermant 1, 2 ou 3 atomes de carbone, d'un alcoxy renfermant 1 ou 2 atomes de carbone, d'un diméthylamino, d'un sulfamoyle, d'un méthylsulfonyle et d'un méthylsulfonylamino ;
v vaut 0, 1, 2 ou 3 ;
w vaut 0, 1, 2 ou 3 ;
R(21) représente un hydrogène ou un alkyle renfermant 1, 2, 3, 4 ou 5 atomes de carbone ;
R(22) représente un alkyle renfermant 1, 2, 3, 4 ou 5 atomes de carbone ;
R(16) représente un alkyle renfermant 1, 2 ou 3 atomes de carbone, un cycloalkyle renfermant 3, 4, 5, 6, 7, 8 ou 9 atomes de carbone, un CF₃, un OR(17), un phényle, un furyle, un thiényle ou un groupe hétéroaromatique azoté renfermant 3, 4 ou 5 atomes de carbone, où le phényle, le furyle, le thiényle et le groupe hétéroatomatique azoté sont non substitués ou substitués par 1 ou 2 substituants choisis parmi le groupe constitué d'un F, d'un Cl, d'un Br, d'un CF₃, d'un OCF₃, d'un CN, d'un COOMe, d'un CONH₂, d'un COMe, d'un NH₂, d'un OH, d'un alkyle renfermant 1, 2 ou 3 atomes de carbone, d'un alcoxy renfermant 1 ou 2 atomes de carbone, d'un diméthylamino, d'un sulfamoyle, d'un méthylsulfonyle et d'un méthylsulfonylamino ;
R(17) représente un alkyle renfermant 1, 2, 3 ou 4 atomes de carbone, un cycloalkyle renfermant 3, 4, 5 ou 6 atomes de carbone, un CF₃, un phényle ou un 2-, un 3- ou un 4-pyridyle,
où le phényle ou le 2-, le 3- ou le 4-pyridyle sont non substitués ou substitués par 1, 2 ou 3 substituants choisis parmi le groupe constitué d'un F, d'un Cl, d'un Br, d'un CF₃, d'un OCF₃, d'un CN, d'un COOMe, d'un CONH₂, d'un COMe, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
R(4) représente un hydrogène ou un alkyle renfermant 1 ou 2 atomes de carbone ;
R(5), R(6), R(7) et R(8) représentent, indépendamment les uns des autres, un hydrogène, un F, un Cl, un Br, un CF₃, un CN, un COOMe, un CONH₂, un COMe, un NH₂, un OH, un alkyle renfermant 1, 2 ou 3 atomes de carbone, un alcoxy renfermant 1 ou 2 atomes de carbone, un diméthylamino, un sulfamoyle, un méthylsulfonyle ou un méthylsulfonylamino ;
R(30) et R(31) représentent, indépendamment l'un de l'autre, un hydrogène ou un méthyle ;
ou
R(30) et R(31) représentent conjointement une chaîne de 2 groupes méthylène ;
ainsi que leurs sels pharmaceutiquement acceptables.

4. Composés de formule I selon les revendications 1 ou 2, dans laquelle :
R(1) représente un C(O)OR(9), un SO₂R(10), un COR(11) ou un C(O)NR(12)R(13) ;
R(9) représente un CₓH₂ₓ-R(14) ;
x vaut 0, 1, 2, 3 ou 4,
où x ne peut pas valoir 0 si R(14) représente un OR(15) ;
R(14) représente un alkyle renfermant 1, 2, 3 ou 4 atomes de carbone, un cycloalkyle renfermant 3, 4, 5, 6, 7, 8 ou 9 atomes de carbone, un CF₃, un OR(15), un phényle, un furyle, un thiényle ou un groupe hétéroaromatique azoté renfermant 3, 4 ou 5 atomes de carbone,
où le phényle, le furyle, le thiényle et le groupe hétéroaromatique azoté sont non substitués ou substitués par 1 ou 2 substituants choisis parmi le groupe constitué d'un F, d'un Cl, d'un Br, d'un CF₃, d'un OCF₃, d'un CN, d'un COOMe, d'un CONH₂, d'un COMe, d'un OH, d'un alkyle renfermant 1, 2 ou 3 atomes de carbone, d'un alcoxy renfermant 1 ou 2 atomes de carbone, d'un diméthylamino, d'un sulfamoyle, d'un méthylsulfonyle et d'un méthylsulfonylamino ;
R(15) représente un alkyle renfermant 1 ou 2 atomes de carbone, un cycloalkyle renfermant 3, 4, 5 ou 6 atomes de carbone, un CF₃ ou un phényle,
qui est non substitué ou substitué par 1 ou 2 substituants choisis parmi le groupe constitué d'un F, d'un Cl, d'un Br, d'un CF₃, d'un CN, d'un COOMe, d'un CONH₂, d'un COMe, d'un OH, d'un alkyle renfermant 1, 2 ou 3 atomes de carbone, d'un alcoxy renfermant 1 ou 2 atomes de carbone, d'un diméthylamino, d'un sulfamoyle, d'un méthylsulfonyle et d'un méthylsulfonylamino ;
R(10), R(11) et R(12), indépendamment les uns des autres, sont tels que définis pour R(9) ;
R(13) représente un hydrogène ;
R(2) représente un hydrogène ou un alkyle renfermant 1, 2 ou 3 atomes de carbone ;
R(3) représente un CyH₂y-R(16) ;
y vaut 0,1, 2, 3 ou 4,
où y ne peut pas valoir 0 si R(16) représente un OR(17) ;
R(16) représente un alkyle renfermant 1, 2 ou 3 atomes de carbone, un cycloalkyle renfermant 3, 4, 5, 6, 7, 8 ou 9 atomes de carbone, un CF₃, un OR(17), un phényle, un furyle, un thiényle ou un groupe hétéroaromatique azoté renfermant 3, 4 ou 5 atomes de carbone,
où le phényle, le furyle, le thiényle et le groupe hétéroaromatique azoté sont non substitués ou substitués par 1 ou 2 substituants choisis parmi le groupe constitué d'un F, d'un Cl, d'un Br, d'un CF₃, d'un OCF₃, d'un CN, d'un COOMe, d'un CONH₂, d'un COMe, d'un NH₂, d'un OH, d'un alkyle renfermant 1, 2 ou 3 atomes de carbone, d'un alcoxy renfermant 1 ou 2 atomes de carbone, d'un diméthylamino, d'un sulfamoyle, d'un méthylsulfonyle et d'un méthylsulfonylamino ;
R(17) représente un alkyle renfermant 1, 2, 3, 4 ou 5 atomes de carbone, un cycloalkyle renfermant 3, 4, 5 ou 6 atomes de carbone, un CF₃, un phényle ou un 2-, un 3- ou un 4-pyridyle, où le phényle ou le 2-, le 3- ou le 4-pyridyle sont non substitués ou substitué par 1, 2
ou 3 substituants choisis parmi le groupe constitué d'un F, d'un Cl, d'un Br, d'un CF₃, d'un OCF₃, d'un NO₂, d'un CN, d'un COOMe, d'un CONH₂, d'un COMe, d'un OH, d'un alkyle renfermant 1, 2, 3 ou 4 atomes de carbone, d'un alcoxy renfermant 1, 2, 3 ou 4 atomes de carbone, d'un diméthylamino, d'un sulfamoyle, d'un méthylsulfonyle et d'un méthylsulfonylamino ;
R(4) représente un hydrogène ou un alkyle renfermant 1 ou 2 atomes de carbone ;
R(5), R(6), R(7) et R(8) représentent, indépendamment les uns des autres, un hydrogène, un F, un Cl, un Br, un CF₃, un CN, un COOMe, un CONH₂, un COMe, un NH₂, un OH, un alkyle renfermant 1, 2 ou 3 atomes de carbone, un alcoxy renfermant 1 ou 2 atomes de carbone, un diméthylamino, un sulfamoyle, un méthylsulfonyle ou un méthylsulfonylamino ;
R(30) et R(31) représentent, indépendamment l'un de l'autre, un hydrogène ou un méthyle ;
ou
R(30) et R(31) représentent conjointement une chaîne de 2 groupes méthylène ;
ainsi que leurs sels pharmaceutiquement acceptables.

5. Composés de formule I selon l'une ou plusieurs des revendications 1, 2 ou 4, dans laquelle :
R(1) représente un C(O)OR(9), un SO₂R(10), un COR(11) ou un C(O)NR(12)R(13) ;
R(9) représente un CₓH₂ₓ-R(14) ;
x vaut 0, 1, 2 ou 3 ;
R(14) représente un alkyle renfermant 1, 2, 3 ou 4 atomes de carbone, un cycloalkyle renfermant 3, 4, 5, 6, 7, 8 ou 9 atomes de carbone, un CF₃, un phényle ou un pyridyle,
où le phényle et le pyridyle sont non substitués ou substitués par 1 ou 2 substituants choisis parmi le groupe constitué d'un F, d'un Cl, d'un CF₃, d'un OCF₃, d'un OH, d'un alkyle renfermant 1, 2 ou 3 atomes de carbone et d'un alcoxy renfermant 1 ou 2 atomes de carbone ;
R(10), R(11) et R(12), indépendamment les uns des autres, sont tels que définis pour R(9) ;
R(13) représente un hydrogène ;
R(2) représente un hydrogène ;
R(3) représente un C_{y}H_{2y}-R(16) ;
y vaut 0, 1 ou 2 ;
R(16) représente un alkyle renfermant 1, 2 ou 3 atomes de carbone, un cycloalkyle renfermant 5 ou 6 atomes de carbone, un CF₃, un phényle ou un pyridyle,
où le phényle et le pyridyle sont non substitués ou substitués par 1 ou 2 substituants choisis parmi le groupe constitué d'un F, d'un Cl, d'un CF₃, d'un OCF₃, d'un OH, d'un alkyle renfermant 1, 2 ou 3 atomes de carbone et d'un alcoxy renfermant 1 ou 2 atomes de carbone ;
R(4) représente un hydrogène ;
R(5), R(6), R(7) et R(8) représentent, indépendamment les uns des autres, un hydrogène, un F, un CF₃, un CN, un COOMe, un CONH₂, un NH₂, un OH, un alkyle renfermant 1, 2 ou 3 atomes de carbone ou un alcoxy renfermant 1 ou 2 atomes de carbone ;
R(30) et R(31) représentent, indépendamment l'un de l'autre, un hydrogène ou un méthyle ;
ou
R(30) et R(31) représentent conjointement une chaîne de 2 groupes méthylène ;
ainsi que leurs sels pharmaceutiquement acceptables.

6. Composés de formule I selon l'une ou plusieurs des revendications 1, 2, 4 ou 5, dans laquelle :
R(1) représente un C(O)OR(9) ou un COR(11) ;
R(9) représente un CₓH₂ₓ-R(14) ;
x vaut 0, 1, 2 ou 3 ;
R(14) représente un cycloalkyle renfermant 5 ou 6 atomes de carbone ou un phényle,
où le phényle est non substitué ou substitué par 1 ou 2 substituants choisis parmi le groupe constitué d'un F, d'un Cl, d'un CF₃, d'un OCF₃, d'un alkyle renfermant 1, 2 ou 3 atomes de carbone et d'un alcoxy renfermant 1 ou 2 atomes de carbone ;
R(11) est tel que défini pour R(9) ;
R(2) représente un hydrogène ;
R(3) représente un C_{y}H_{2y}-R(16) ;
y vaut 0, 1 ou 2 ;
R(16) représente un alkyle renfermant 1, 2 ou 3 atomes de carbone, un cycloalkyle renfermant 5 ou 6 atomes de carbone, un CF₃, un phényle ou un pyridyle,
où le phényle et le pyridyle sont non substitués ou substitués par 1 ou 2 substituants choisis parmi le groupe constitué d'un F, d'un Cl, d'un CF₃, d'un OCF₃, d'un alkyle renfermant 1, 2 ou 3 atomes de carbone et d'un alcoxy renfermant 1 ou 2 atomes de carbone ;
R(4) représente un hydrogène ;
R(5), R(6), R(7) et R(8) représentent, indépendamment les uns des autres, un hydrogène, un F, un CF₃, un alkyle renfermant 1, 2 ou 3 atomes de carbone ou un alcoxy renfermant 1 ou 2 atomes de carbone ;
R(30) et R(31) représentent un hydrogène ;
ainsi que leurs sels pharmaceutiquement acceptables.

7. Composé de formule I selon l'une ou plusieurs des revendications 1, 2, 4, 5 ou 6, qui est: 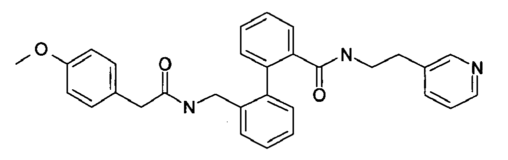 ainsi que leurs sels pharmaceutiquement acceptables.

8. Composés de formule I selon l'une ou plusieurs des revendications 1 à 7 et leurs sels pharmaceutiquement acceptables destinés à être utilisés en tant qu'agents pharmaceutiques.

9. Préparation pharmaceutique comprenant une quantité efficace d'au moins un composé de formule I selon l'une ou plusieurs des revendications 1 à 7 et/ou d'un sel pharmaceutiquement acceptable de celui-ci en tant qu'ingrédient actif, conjointement avec des supports et des additifs pharmaceutiquement acceptables et éventuellement en outre un ou plusieurs autres ingrédients actifs pharmacologiques.

10. Utilisation d'un composé de formule I selon l'une ou plusieurs des revendications 1 à 7 et/ou d'un sel pharmaceutiquement acceptable de celui-ci pour la production d'un médicament présentant une action de blocage des canaux K⁺ pour la thérapie et la prophylaxie de maladies à médiation par les canaux K⁺.

11. Utilisation d'un composé de formule I selon l'une ou plusieurs des revendications 1 à 7 et/ou d'un sel pharmaceutiquement acceptable de celui-ci pour la production d'un médicament pour la thérapie ou la prophylaxie de troubles du rythme cardiaque, qui peuvent être éliminés par prolongement du potentiel d'action.

12. Utilisation d'un composé de formule I selon l'une ou plusieurs des revendications 1 à 7 et/ou d'un sel pharmaceutiquement acceptable de celui-ci pour la production d'un médicament pour la thérapie ou la prophylaxie d'arythmies à réentrée.

13. Utilisation d'un composé de formule I selon l'une ou plusieurs des revendications 1 à 7 et/ou d'un sel pharmaceutiquement acceptable de celui-ci pour la production d'un médicament pour la thérapie ou la prophylaxie d'arythmies supraventriculaire.

14. Utilisation d'un composé de formule I selon l'une ou plusieurs des revendications 1 à 7 et/ou d'un sel pharmaceutiquement acceptable de celui-ci pour la production d'un médicament pour la thérapie ou la prophylaxie d'une fibrillation auriculaire ou d'un flutter auriculaire.

15. Utilisation d'un composé de formule I selon l'une ou plusieurs des revendications 1 à 7 et/ou d'un sel pharmaceutiquement acceptable de celui-ci pour la production d'un médicament pour stopper une fibrillation auriculaire ou un flutter auriculaire (cardioversion).

16. Préparation pharmaceutique comprenant une quantité efficace d'au moins un composé de formule I selon l'une ou plusieurs des revendications 1 à 7 et/ou d'un sel pharmaceutiquement acceptable de celui-ci ainsi qu'un agent bloquant les canaux IKr en tant qu'ingrédients actifs, conjointement avec des supports et des additifs pharmaceutiquement acceptables.

17. Préparation pharmaceutique comprenant une quantité efficace d'au moins un composé de formule I selon l'une ou plusieurs des revendications 1 à 7 et/ou d'un sel pharmaceutiquement acceptable de celui-ci ainsi qu'un agent bloquant les canaux IKs en tant qu'ingrédients actifs, conjointement avec des supports et des additifs pharmaceutiquement acceptables.

18. Préparation pharmaceutique comprenant une quantité efficace d'au moins un composé de formule I selon l'une ou plusieurs des revendications 1 à 7 et/ou un sel pharmaceutiquement acceptable de celui-ci ainsi qu'un agent bêta-bloquant en tant qu'ingrédients actifs, conjointement avec des supports et des additifs pharmaceutiquement acceptables.
